# EUROPEAN PATENT SPECIFICATION

(11) **EP 3 433 370 B1**
(45) Date of publication and mention of the grant of the patent: **29.04.2020**
(21) Application number: 17717632.8
(22) Date of filing: 22.03.2017
(51) Int. Cl.: C12P 5/02

(54) **ENZYMATIC METHOD FOR PRODUCING ISOBUTENE FROM 3-METHYLBUTYRIC ACID**
ENZYMATISCHES VERFAHREN ZUR HERSTELLUNG VON ISOBUTEN AUS 3-METHYLBUTTERSÄURE
PROCÉDÉ ENZYMATIQUE POUR LA PRODUCTION D'ISOBUTÈNE À PARTIR D'ACIDE 3-MÉTHYL-BUTYRIQUE

(30) Priority: 22.03.2016 EP 16161683
(43) Date of publication of application: 30.01.2019
(73) Proprietor: Global Bioenergies, 91000 Evry (FR); Scientist of Fortune S.A., 1940 Luxembourg (LU)
(72) Inventor: ALLARD, Mathieu, 91770 Saint-Vrain (FR); MARLIERE, Philippe, 7500 Tournai (BE)
(74) Representative: Vossius & Partner Patentanwälte Rechtsanwälte mbB
(86) International application number: PCT/EP2017/056822
(87) International publication number: WO 2017/162738

(56) References cited:
- WO-A1-2014/085612
- WO-A1-2016/042011
- ROSSONI ET AL: "The putative mevalonate diphosphate decarboxylase from Picrophilus torridus is in reality a mevalonate-3-kinase with high potential for bioproduction of isobutene", APPLIED AND ENVIRONMENTAL MICROBIOLOGY, vol. 81, 2015, pages 2625-2634, XP002770168,
- DE LA CRUZ ET AL: "Integrated synthesis of biodiesel, bioethanol, isobutene, and glycerol ethers from algae", INDUSTRIAL & ENGINEERING CHEMISTRY RESEARCH, vol. 53, 2014, pages 14397-14407, XP002770169,
- VAN LEEUWEN ET AL: "Fermentative production of isobutene", APPLIED MICROBIOLOGY AND BIOTECHNOLOGY, vol. 93, 2012, pages 1377-1387, XP035013024, cited in the application
- GOGERTY ET AL: "Formation of isobutene from 3-hydroxy-3-methylbutyrate by diphosphomevalonate decarboxylate", APPLIED AND ENVIRONMENTAL MICROBIOLOGY, vol. 76, 2010, pages 8004-8010, XP002680645, cited in the application
- BENOIT ET AL: "An artificial pathway for isobutene production by direct fermentation: combining metabolic engineering and protein engineering", PROTEIN SCIENCE, vol. 24(Suppl 1), 2015, page 70, XP002770191,

### SEQUENCE LISTING

<110> Global Bioenergies
   Scientist of Fortune S.A.
<120> Method for producing isobutene from 3-methylcrotonyl-CoA
<130> Z1483 PCT S3
<150> EP 16 16 1683.4
   <151> 2016-03-22
<160> 15
<170> BiSSAP 1.2
<210> 1
   <211> 422
   <212> PRT
   <213> Jeotgalicoccus sp. ATCC 8456
<400> 1
   Met Ala Thr Leu Lys Arg Asp Lys Gly Leu Asp Asn Thr Leu Lys Val
   1               5                   10                  15
   Leu Lys Gln Gly Tyr Leu Tyr Thr Thr Asn Gln Arg Asn Arg Leu Asn
               20                  25                  30
   Thr Ser Val Phe Gln Thr Lys Ala Leu Gly Gly Lys Pro Phe Val Val
           35                  40                  45
   Val Thr Gly Lys Glu Gly Ala Glu Met Phe Tyr Asn Asn Asp Val Val
       50                  55                  60
   Gln Arg Glu Gly Met Leu Pro Lys Arg Ile Val Asn Thr Leu Phe Gly
   65                  70                  75                  80
   Lys Gly Ala Ile His Thr Val Asp Gly Lys Lys His Val Asp Arg Lys
                   85                  90                  95
   Ala Leu Phe Met Ser Leu Met Thr Glu Gly Asn Leu Asn Tyr Val Arg
               100                 105                 110
   Glu Leu Thr Arg Thr Leu Trp His Ala Asn Thr Gln Arg Met Glu Ser
           115                 120                 125
   Met Asp Glu Val Asn Ile Tyr Arg Glu Ser Ile Val Leu Leu Thr Lys
       130                 135                 140
   Val Gly Thr Arg Trp Ala Gly Val Gln Ala Pro Pro Glu Asp Ile Glu
   145                 150                 155                 160
   Arg Ile Ala Thr Asp Met Asp Ile Met Ile Asp Ser Phe Arg Ala Leu
                   165                 170                 175
   Gly Gly Ala Phe Lys Gly Tyr Lys Ala Ser Lys Glu Ala Arg Arg Arg
               180                 185                 190
   Val Glu Asp Trp Leu Glu Glu Gln Ile Ile Glu Thr Arg Lys Gly Asn
           195                 200                 205
   Ile His Pro Pro Glu Gly Thr Ala Leu Tyr Glu Phe Ala His Trp Glu
       210                 215                 220
   Asp Tyr Leu Gly Asn Pro Met Asp Ser Arg Thr Cys Ala Ile Asp Leu
   225                 230                 235                 240
   Met Asn Thr Phe Arg Pro Leu Ile Ala Ile Asn Arg Phe Val Ser Phe
                   245                 250                 255
   Gly Leu His Ala Met Asn Glu Asn Pro Ile Thr Arg Glu Lys Ile Lys
               260                 265                 270
   Ser Glu Pro Asp Tyr Ala Tyr Lys Phe Ala Gln Glu Val Arg Arg Tyr
           275                 280                 285
   Tyr Pro Phe Val Pro Phe Leu Pro Gly Lys Ala Lys Val Asp Ile Asp
       290                 295                 300
   Phe Gln Gly Val Thr Ile Pro Ala Gly Val Gly Leu Ala Leu Asp Val
   305                 310                 315                 320
   Tyr Gly Thr Thr His Asp Glu Ser Leu Trp Asp Asp Pro Asn Glu Phe
                   325                 330                 335
   Arg Pro Glu Arg Phe Glu Thr Trp Asp Gly Ser Pro Phe Asp Leu Ile
               340                 345                 350
   Pro Gln Gly Gly Gly Asp Tyr Trp Thr Asn His Arg Cys Ala Gly Glu
           355                 360                 365
   Trp Ile Thr Val Ile Ile Met Glu Glu Thr Met Lys Tyr Phe Ala Glu
       370                 375                 380
   Lys Ile Thr Tyr Asp Val Pro Glu Gln Asp Leu Glu Val Asp Leu Asn
   385                 390                 395                 400
   Ser Ile Pro Gly Tyr Val Lys Ser Gly Phe Val Ile Lys Asn Val Arg
                   405                 410                 415
   Glu Val Val Asp Arg Thr
               420
<210> 2
   <211> 441
   <212> PRT
   <213> Macrococcus caseolyticus (strain JCSC5402)
<400> 2
   Met Arg Val Glu Phe Thr Ile Asn Tyr Ile Asn Val Glu Gly Ile Ser
   1               5                   10                  15
   Met Ser Lys Arg Val Pro Lys Asp Arg Gly Ile Asp Asn Ser Leu Lys
               20                  25                  30
   Ile Met Lys Glu Gly Tyr Glu Tyr Val Pro Ala Arg Met Lys Lys Phe
           35                  40                  45
   Asn Thr Asn Ile Phe Glu Thr Arg Val Leu Gly Gly Lys Thr Ala Val
       50                  55                  60
   Val Ile Ser Gly Lys Asp Ala Ala Glu Leu Phe Tyr Asp Asn Asp Lys
   65                  70                  75                  80
   Thr Glu Arg Lys Gly Thr Leu Pro Lys Arg Val Val Lys Thr Leu Phe
                   85                  90                  95
   Gly Lys Gly Ala Ile His Thr Thr Thr Gly Lys Lys His Ile Asp Arg
               100                 105                 110
   Lys Ala Leu Phe Met Ser Leu Met Thr Asp Glu Asn Leu Ala Tyr Leu
           115                 120                 125
   Arg Lys Leu Thr Arg Ile Tyr Trp Phe Gln Asn Ile Glu His Met Gln
       130                 135                 140
   Tyr Lys Gln Lys Val Asn Val Tyr Glu Glu Ala Thr Glu Leu Leu Thr
   145                 150                 155                 160
   Lys Val Gly Leu Arg Trp Ala Gly Ile Val Asp His Pro Glu Asn Ile
                   165                 170                 175
   Gln Lys Met Ala Asp Asp Met Asn Lys Met Ile Asp Ser Phe Ser Ala
               180                 185                 190
   Ile Gly Ser Leu Tyr Gly Gly Tyr Arg Glu Ala Lys Lys Ala Arg Ala
           195                 200                 205
   Arg Val Glu Gln Phe Leu Glu Asp Gln Ile Thr Ala Val Arg Lys Gly
       210                 215                 220
   Lys Ile His Pro Glu Lys Gly Thr Ala Leu Tyr Glu Phe Ser His Trp
   225                 230                 235                 240
   Glu Asp Met Asn Gly Lys Pro Met Asp Ala Arg Leu Cys Ala Val Asp
                   245                 250                 255
   Leu Met Asn Val Ile Arg Pro Leu Val Ala Ile Asn Lys Phe Val Ser
               260                 265                 270
   Phe Gly Val Leu Ala Leu His Glu Phe Pro Gly Glu Arg Val Arg Val
           275                 280                 285
   Ala Leu Asn Glu Gly Asp Tyr Ala Tyr Lys Phe Val Gln Glu Val Arg
       290                 295                 300
   Arg Tyr Tyr Pro Phe Val Pro Phe Leu Pro Gly Lys Ala Lys Glu Asn
   305                 310                 315                 320
   Ile Thr Phe Asp Gly Tyr Lys Ile Gln Lys Asp Thr Met Met Leu Leu
                   325                 330                 335
   Asp Ile Tyr Gly Thr Leu His Arg Asp Asp Leu Phe Ser Glu Pro Glu
               340                 345                 350
   Arg Phe Asn Pro Tyr Arg Phe Asp Asn Trp Asp Gly Ser Pro Phe Asp
           355                 360                 365
   Leu Ile Pro Gln Gly Gly Gly Asp Tyr Tyr Thr Asn His Arg Cys Ala
       370                 375                 380
   Gly Glu Trp Met Thr Ile Ile Ile Met Glu Glu Thr Met Lys Phe Phe
   385                 390                 395                 400
   Ala Asn Glu Ile Ser Tyr Asp Val Pro Pro Gln Asp Phe Thr Val Asp
                   405                 410                 415
   Thr Thr Lys Phe Pro Gly Lys Val Ala Ser Gly Met Asp Ile Glu Asn
               420                 425                 430
   Ile Arg Val Asn Ile Asp Arg Thr Lys
           435                 440
<210> 3
   <211> 345
   <212> PRT
   <213> Myxococcus xanthus (strain DK 1622)
<400> 3
   Met Lys Ala Val Val Leu Arg Ser Phe Gly Glu Ala Gly Asn Leu Lys
   1               5                   10                  15
   Met Glu Thr Met Pro Met Pro Arg Pro Gly Arg Gly Glu Val Leu Leu
               20                  25                  30
   Arg Val His Ala Cys Gly Val Cys Tyr His Asp Val Ile Asn Arg Arg
           35                  40                  45
   Gly Asn Leu Pro Arg Thr Ser Val Pro Ala Ile Leu Gly His Glu Ala
       50                  55                  60
   Ala Gly Glu Val Ile Glu Val Gly Pro Asp Thr Pro Gly Trp Lys Thr
   65                  70                  75                  80
   Gly Asp Arg Ala Ala Thr Leu Gln Arg Met Ser Cys Gly Asp Cys Ala
                   85                  90                  95
   Leu Cys Arg Ser Gly Arg Asn Ser Leu Cys Lys Thr Asp Asn Arg Phe
               100                 105                 110
   Phe Gly Glu Glu Leu Pro Gly Gly Tyr Ala Gln Phe Met Val Ala Pro
           115                 120                 125
   Val Gly Gly Leu Gly Arg Val Pro Ala Ser Leu Pro Trp Asn Glu Ala
       130                 135                 140
   Ala Thr Val Cys Cys Thr Thr Gly Thr Ala Val His Thr Val Arg Thr
   145                 150                 155                 160
   Arg Gly Lys Val Arg Ala Gly Glu Thr Val Leu Ile Thr Gly Ala Ser
                   165                 170                 175
   Gly Gly Val Gly Leu Ser Ser Val Gln Leu Ala Arg Leu Asp Gly Ala
               180                 185                 190
   Arg Val Ile Ala Val Thr Ser Ser Glu Ala Lys Val Gln Ala Leu Lys
           195                 200                 205
   Glu Ala Gly Ala Asp Glu Val Ile Val Ser Arg Gly Leu Asp Phe Ala
       210                 215                 220
   Ser Asp Val Arg Lys Arg Thr Gln Gly Ala Gly Val Asp Val Ala Val
   225                 230                 235                 240
   Glu Ile Val Gly Ser Ala Thr Phe Asp Gln Thr Leu Lys Ser Met Ala
                   245                 250                 255
   Pro Gly Gly Arg Val Val Val Val Gly Asn Leu Glu Ser Gly Met Val
               260                 265                 270
   Gln Leu Asn Pro Gly Leu Val Ile Val Lys Glu Leu Glu Ile Leu Gly
           275                 280                 285
   Ala Tyr Ala Thr Thr Gln Ala Glu Leu Asp Glu Ala Leu Arg Leu Thr
       290                 295                 300
   Ala Thr Gly Gly Val Arg Gln Phe Val Thr Asp Ala Val Pro Leu Ala
   305                 310                 315                 320
   Glu Ala Ala Lys Ala His Phe Arg Leu Glu Asn Arg Glu Val Ala Gly
                   325                 330                 335
   Arg Leu Val Leu Val Pro Pro Glu Ala
               340                 345
<210> 4
   <211> 527
   <212> PRT
   <213> Rhodotorula minuta
<400> 4
   Met Gly Ile Val Gln Glu Ala Ala Ala Phe Val Gly Ser Leu Thr Ala
   1               5                   10                  15
   Val Gln Leu Ala Gly Ala Phe Ala Ala Leu Val Leu Ala Phe His Val
               20                  25                  30
   Val Pro Tyr Ile Leu Asp Pro His Gly Leu Arg Lys Tyr Pro Ala Ala
           35                  40                  45
   Gly Pro Phe Gly Ile Ala Ala Phe Thr Asp Ala Trp Val Val Trp Gln
       50                  55                  60
   Ala Arg Arg Gly Lys Arg Phe Glu Ala Leu Asp Lys Ala His Arg Lys
   65                  70                  75                  80
   Tyr Gly Lys Tyr Val Arg Ile Ser Pro Arg Gln Val Ser Ile Ala Asp
                   85                  90                  95
   Pro Lys Ala Leu Asp Thr Ile Tyr Gly His Gly Thr Gly Thr Met Lys
               100                 105                 110
   Pro Ala Phe Tyr Asp Ala Phe Val Gly Leu Lys Asn Val Arg Gly Leu
           115                 120                 125
   Phe Asn Thr Arg Asp Arg Ala Glu His Thr Arg Lys Arg Lys Ile Val
       130                 135                 140
   Ser Ala Thr Phe Ala Gln Arg Asn Val Leu Glu Phe Glu Pro Tyr Ile
   145                 150                 155                 160
   Ala Ser Val Met Arg Gln Leu Leu Asn Lys Trp Asp Ala Met Cys Asp
                   165                 170                 175
   Lys Ala Val Lys Asp Gly Lys Gly Trp Val Thr Leu Asp Thr Leu Thr
               180                 185                 190
   Trp Leu Asn Phe Leu Ala Phe Asp Ile Ile Gly Asp Leu Ala Phe Gly
           195                 200                 205
   Ala Pro Phe Gly Met Val Glu Arg Glu Ala Asp Ile Cys Glu Ile Glu
       210                 215                 220
   Gln Glu Asp Gly Ser Ile Lys His Leu Pro Ala Val Thr Ile Leu Asn
   225                 230                 235                 240
   Glu Arg Gly Glu Tyr Ser Asn Cys Leu Gly Val Met Pro Pro His Trp
                   245                 250                 255
   Arg Pro Phe Ser Lys Tyr Val Asp Pro Trp Phe Ser Arg Gly His Ala
               260                 265                 270
   Ser Val Ile Asn Leu Ala Gly Met Ala Arg Ala Arg Val Asn Ala Arg
           275                 280                 285
   Leu Lys Ser Gly Ala Gly Asp Arg Lys Asp Leu Leu Ala Arg Leu Gln
       290                 295                 300
   Glu Ala Arg Asp Glu Ser Gly Asn Pro Met Asp Ile Asp Glu Leu Thr
   305                 310                 315                 320
   Ala Glu Ala Leu Thr Gln Leu Ile Ala Gly Ser Asp Thr Thr Ser Asn
                   325                 330                 335
   Ser Ser Cys Ala Ile Ile Phe Tyr Leu Ala Arg Asn Gln Asp Ala Gln
               340                 345                 350
   Lys Lys Leu Gln Ala Glu Leu Asp Lys Thr Phe Lys Asp Arg Gly Ile
           355                 360                 365
   Ser Gly Val Met Glu Tyr Glu Asp Ile Lys Thr Leu Pro Tyr Leu Glu
       370                 375                 380
   Ala Cys Ile Asn Glu Ala Leu Arg Met His Ser Thr Ser Ser Met Gly
   385                 390                 395                 400
   Leu Pro Arg Val Met Pro Ala Gln Gly Ala Asp Phe Gln Gly Glu His
                   405                 410                 415
   Phe Lys Glu Gly Thr Glu Val Ser Val Pro Ala Tyr Thr Ile His His
               420                 425                 430
   Leu Glu Ser Ile Trp Gly Asp Pro Phe Asn Tyr Arg Pro Glu Arg Trp
           435                 440                 445
   Leu Gly Ser Asp Ala Lys Gln Leu Glu Lys Ser Phe Ile Pro Phe Ser
       450                 455                 460
   Ile Gly Pro Arg Ser Cys Val Gly Arg Asn Leu Ala Thr Met Glu Leu
   465                 470                 475                 480
   Leu Val Phe Met Ser Thr Leu Phe Tyr Arg Tyr Asp Phe Lys Leu Ala
                   485                 490                 495
   Asp Glu Asn Gln Thr Lys Leu Glu Thr Ser Glu Gly Phe Leu Arg Lys
               500                 505                 510
   Pro Leu Glu Ser Trp Ile Lys Met Arg Arg Arg Ser Ile Asp Ala
           515                 520                 525
<210> 5
   <211> 260
   <212> PRT
   <213> Pseudomonas aeruginosa (strain UCBPP-PA14)
<400> 5
   Met Ser Glu Phe Phe Asp Arg Thr Gly Pro Leu Gln Glu Ala Gly Ser
   1               5                   10                  15
   Tyr Pro Gln Trp Ala Gln Gln Leu Ile Val Asp Cys Gln Ala Ser Lys
               20                  25                  30
   Asp Arg Val Ser Gly His Glu Leu Tyr Arg Arg Met Arg Asp Ala Glu
           35                  40                  45
   Leu Ser Pro Ala Leu Met Arg Leu Tyr Leu Ile Gly Gly Trp Pro Val
       50                  55                  60
   Val Glu Gln Phe Pro Leu Tyr Met Ser Gln Asn Leu Leu Lys Thr Arg
   65                  70                  75                  80
   Phe Ala Arg His Pro Gly Glu Asp Met Ala Arg Arg Trp Leu Met Arg
                   85                  90                  95
   Asn Ile Arg Val Glu Leu Asn His Ala Asp Tyr Trp Leu His Trp Ala
               100                 105                 110
   Glu Ala His Gly Val Ser Leu Ala Glu Ile Gln Ala Gln Asp Val Pro
           115                 120                 125
   Ala Glu Leu His Ala Leu Ser His Trp Cys Trp His Thr Cys Ala Ser
       130                 135                 140
   Asp Ser Leu Pro Val Ala Met Ala Ala Thr Asn Tyr Ala Ile Glu Gly
   145                 150                 155                 160
   Val Thr Gly Glu Trp Ser Ala Leu Val Cys Ser Asn Gly Val Tyr Glu
                   165                 170                 175
   Asn Ala Phe Pro Lys Glu Gly Arg Lys Arg Ala Met Lys Trp Leu Lys
               180                 185                 190
   Leu His Ala Gln Tyr Asp Asp Ala His Pro Trp Glu Ala Leu Glu Ile
           195                 200                 205
   Ile Cys Thr Leu Ala Gly Thr Asn Pro Ser Ala Glu Leu Arg Arg Gln
       210                 215                 220
   Leu Arg Asp Ala Ile Cys Lys Ser Tyr Asp Tyr Met Tyr Leu Phe Leu
   225                 230                 235                 240
   Glu Arg Cys Met Gln Leu Glu Glu Ala Arg Ser Ala Arg Lys Leu Ala
                   245                 250                 255
   Val Gly Ala Glu
               260
<210> 6
   <211> 261
   <212> PRT
   <213> Pseudomonas syringae pv. tomato (strain DC3000)
<400> 6
   Met Ile Asp Thr Phe Glu Arg Thr Gly Pro Leu Met Glu Ala Ser Ser
   1               5                   10                  15
   Tyr Pro Ala Trp Ala Gln Gln Leu Ile Asn Asp Cys Ser Pro Ala Lys
               20                  25                  30
   Ala Arg Val Val Glu His Glu Leu Tyr Gln Gln Met Arg Asp Ala Lys
           35                  40                  45
   Leu Ser Pro Gln Ile Met Arg Gln Tyr Leu Ile Gly Gly Trp Pro Val
       50                  55                  60
   Val Glu Gln Phe Ala Val Tyr Met Ala Lys Asn Leu Thr Lys Thr Arg
   65                  70                  75                  80
   Phe Gly Arg His Pro Gly Glu Asp Met Ala Arg Arg Trp Leu Met Arg
                   85                  90                  95
   Asn Ile Arg Val Glu Leu Asn His Ala Asp Tyr Trp Val Asn Trp Cys
               100                 105                 110
   Ala Ala His Asp Val Thr Leu Glu Asp Leu His Asp Gln Arg Val Ala
           115                 120                 125
   Pro Glu Leu His Ala Leu Ser His Trp Cys Trp Gln Thr Ser Ser Ser
       130                 135                 140
   Asp Ser Leu Ala Val Ala Met Ala Ala Thr Asn Tyr Ala Ile Glu Gly
   145                 150                 155                 160
   Ala Thr Gly Glu Trp Ser Ala Val Val Cys Ser Thr Gly Val Tyr Ala
                   165                 170                 175
   Glu Ala Phe Ala Glu Glu Thr Arg Lys Lys Ser Met Lys Trp Leu Lys
               180                 185                 190
   Met His Ala Gln Tyr Asp Asp Ala His Pro Trp Glu Ala Leu Glu Ile
           195                 200                 205
   Ile Cys Thr Leu Val Gly Asn Lys Pro Ser Leu Gln Leu Gln Ala Glu
       210                 215                 220
   Leu Arg Gln Ala Val Thr Lys Ser Tyr Asp Tyr Met Tyr Leu Phe Leu
   225                 230                 235                 240
   Glu Arg Cys Ile Gln Leu Asp Lys Val Lys Ser Pro Arg Gly Arg Val
                   245                 250                 255
   Ala Ala Leu Glu Met
               260
<210> 7
   <211> 270
   <212> PRT
   <213> Pseudomonas putida (strain F1 / ATCC 700007)
<400> 7
   Met Glu Ile Thr Arg Ile Lys Glu Leu Lys Val Ile Asp Ala Phe Val
   1               5                   10                  15
   Arg Ile Gly Pro Leu Met Asp Pro Ala Ser Tyr Pro Gln Trp Ala Gln
               20                  25                  30
   Gln Leu Ile Glu Asp Cys Arg Glu Ser Lys Arg Arg Val Val Glu His
           35                  40                  45
   Glu Phe Tyr Ala Arg Leu Arg Asp Gly Gln Leu Lys Gln Ser Thr Ile
       50                  55                  60
   Arg Gln Tyr Leu Ile Gly Gly Trp Pro Val Val Glu Gln Phe Ser Leu
   65                  70                  75                  80
   Tyr Met Ala His Asn Leu Thr Lys Thr Arg Tyr Gly Arg His Gln Gly
                   85                  90                  95
   Glu Asp Met Ala Arg Arg Trp Leu Met Arg Asn Ile Arg Val Glu Leu
               100                 105                 110
   Asn His Ala Asp Tyr Trp Val Asn Trp Cys Gln Ala His Gly Val His
           115                 120                 125
   Leu His Glu Leu Gln Ala Gln Glu Val Pro Pro Glu Leu Asn Gly Leu
       130                 135                 140
   Asn Asp Trp Cys Trp Arg Val Cys Ala Thr Glu Asn Leu Ala Ile Ser
   145                 150                 155                 160
   Met Ala Ala Thr Asn Tyr Ala Ile Glu Gly Ala Thr Gly Glu Trp Ser
                   165                 170                 175
   Ala Val Val Cys Ser Thr Asp Thr Tyr Ala Gln Gly Phe Pro Glu Glu
               180                 185                 190
   Gly Arg Lys Arg Ala Met Lys Trp Leu Lys Met His Ala Gln Tyr Asp
           195                 200                 205
   Asp Ala His Pro Trp Glu Ala Leu Glu Ile Ile Cys Thr Leu Ala Gly
       210                 215                 220
   Glu Asn Pro Thr Leu Gly Leu Arg Thr Glu Leu Arg Arg Ala Ile Cys
   225                 230                 235                 240
   Lys Ser Tyr Asp Cys Met Phe Leu Phe Leu Glu Arg Cys Met Gln Leu
                   245                 250                 255
   Glu Gly Arg Gln Gln Gly Arg Met Arg Pro Ala Leu Ala Ala
               260                 265                 270
<210> 8
   <211> 299
   <212> PRT
   <213> Bacillus subtilis (strain 168)
<400> 8
   Met Lys Leu Lys Asp Leu Ile Gly Lys Ala Ser Ile His Lys Asn Lys
   1               5                   10                  15
   Thr Ile Ala Val Ala His Ala Glu Asp Glu Glu Val Ile Arg Ala Val
               20                  25                  30
   Lys Leu Ala Ala Glu His Leu Ser Ala Arg Phe Leu Leu Thr Gly Asp
           35                  40                  45
   Ser Lys Lys Leu Asn Glu Leu Thr Ser Ser Met Gln Gly His Gln Val
       50                  55                  60
   Glu Ile Val His Ala Asn Thr Pro Glu Glu Ser Ala Lys Leu Ala Val
   65                  70                  75                  80
   Arg Ala Val His His Lys Thr Ala Asp Val Leu Met Lys Gly Asn Val
                   85                  90                  95
   Pro Thr Ser Val Leu Leu Lys Ala Val Leu Asn Arg Gln Glu Gly Leu
               100                 105                 110
   Arg Ser Ala Ser Val Leu Ser His Val Ala Val Phe Asp Ile Pro Asp
           115                 120                 125
   Phe Asp Arg Leu Met Phe Val Thr Asp Ser Ala Met Asn Ile Ala Pro
       130                 135                 140
   Ser Leu Glu Glu Leu Arg Gln Ile Leu Gln Asn Ala Val His Val Ala
   145                 150                 155                 160
   His Ala Val Gly Asn Asn Met Pro Lys Ala Ala Ala Leu Ala Ala Val
                   165                 170                 175
   Glu Thr Val Asn Pro Lys Met Glu Ala Thr Val Asn Ala Ala Ala Leu
               180                 185                 190
   Ala Gln Met Tyr Lys Arg Gly Gln Ile Lys Gly Cys Ile Val Asp Gly
           195                 200                 205
   Pro Leu Ala Leu Asp Asn Ala Val Ser Gln Ile Ala Ala Ala Gln Lys
       210                 215                 220
   Lys Ile Ser Gly Asp Val Ala Gly Asn Ala Asp Ile Leu Leu Val Pro
   225                 230                 235                 240
   Thr Ile Glu Ala Gly Asn Ile Leu Tyr Lys Ser Leu Ile Tyr Phe Ala
                   245                 250                 255
   Lys Ala Ser Val Ala Ala Val Ile Thr Gly Ala Lys Ala Pro Ile Ala
               260                 265                 270
   Leu Thr Ser Arg Ala Asp Ser Ala Glu Asn Lys Leu Tyr Ser Ile Ala
           275                 280                 285
   Leu Ala Ile Cys Ala Ser Glu Glu Tyr Thr His
       290                 295
<210> 9
   <211> 273
   <212> PRT
   <213> Enterococcus faecalis MTUP9
<400> 9
   Met Ile Thr Val Ser Ile Ala Gly Gly Ser Gln Pro Glu Ile Leu Gln
   1               5                   10                  15
   Leu Val Lys Lys Ala Leu Lys Glu Ala Glu Gln Pro Leu Gln Phe Ile
               20                  25                  30
   Val Phe Asp Thr Asn Glu Asn Leu Asp Thr Glu Asn Leu Trp Lys Tyr
           35                  40                  45
   Val His Cys Ser Asp Glu Ala Thr Val Ala Gln Glu Ala Val Ser Leu
       50                  55                  60
   Val Ala Thr Gly Gln Ala Gln Ile Leu Leu Lys Gly Ile Ile Gln Thr
   65                  70                  75                  80
   His Thr Leu Leu Lys Glu Met Leu Lys Ser Glu His Gln Leu Lys Asn
                   85                  90                  95
   Lys Pro Ile Leu Ser His Val Ala Met Val Glu Leu Pro Ala Gly Lys
               100                 105                 110
   Thr Phe Leu Leu Thr Asp Cys Ala Met Asn Ile Ala Pro Thr Gln Ala
           115                 120                 125
   Thr Leu Ile Glu Ile Val Glu Asn Ala Lys Glu Val Ala Gln Lys Leu
       130                 135                 140
   Gly Leu His His Pro Lys Ile Ala Leu Leu Ser Ala Ala Glu Asn Phe
   145                 150                 155                 160
   Asn Pro Lys Met Pro Ser Ser Val Leu Ala Lys Glu Val Thr Ala His
                   165                 170                 175
   Phe Asn Gly Gln Gln Glu Ala Thr Val Phe Gly Pro Leu Ser Leu Asp
               180                 185                 190
   Leu Ala Thr Ser Glu Glu Ala Val Ala His Lys Arg Tyr Ser Gly Pro
           195                 200                 205
   Ile Met Gly Asp Ala Asp Ile Leu Val Val Pro Thr Ile Asp Val Gly
       210                 215                 220
   Asn Cys Leu Tyr Lys Ser Leu Thr Leu Phe Gly His Ala Lys Val Gly
   225                 230                 235                 240
   Gly Thr Ile Val Gly Thr Lys Val Pro Val Val Leu Thr Ser Arg Ser
                   245                 250                 255
   Asp Ser Thr Glu Ser Lys Phe His Ser Leu Arg Phe Ala Met Arg Gln
               260                 265                 270
   Val
<210> 10
   <211> 384
   <212> PRT
   <213> Lactobacillus casei W56
<400> 10
   Met Arg Asp Cys Thr Thr Glu Arg Arg Cys Leu Met Thr Met His Pro
   1               5                   10                  15
   Lys Arg Asp Val Val Ile Val Ile Asn Pro Gly Ser Thr Ser Ser Lys
               20                  25                  30
   Ile Ala Leu Phe Lys Ala Gly Lys Met Val Ala Glu Arg Thr Leu Asn
           35                  40                  45
   His Ser Leu Ala Glu Leu Ser Gln Phe Asp Ser Val Ile Ala Gln Lys
       50                  55                  60
   Asp Phe Arg Met Gln Ala Ile Gln Glu Phe Leu Ala Asp Gln Asp Phe
   65                  70                  75                  80
   Ser Ala Ser Glu Val Leu Ala Val Ala Gly Arg Gly Gly Leu Leu Lys
                   85                  90                  95
   Pro Ile Pro Gly Gly Thr Tyr Ala Val Asn Glu Ala Met Leu Asp Asp
                   100                 105                 110
   Leu Thr Ala Ala Lys Arg Asn Glu His Ala Ser Asn Leu Gly Ala Gly
               115                 120                 125
   Leu Ala Gln Gln Val Ala Asp Gln Tyr Gly Val Lys Ala Tyr Val Val
       130                 135                 140
   Asp Pro Pro Val Val Asp Glu Leu Gln Pro Leu Ala Arg Ile Ser Gly
   145                 150                 155                 160
   Leu Lys Gly Ile Glu Arg His Ser Ala Ala His Val Leu Asn Gln Lys
                   165                 170                 175
   Ala Met Ala Arg Gln Val Leu Ala Thr Met Gly Lys Thr Tyr Ala Thr
                   180                 185                 190
   Ser Arg Val Ile Val Ala His Ile Gly Gly Gly Leu Ser Ile His Ala
               195                 200                 205
   His Glu Asn Gly Arg Met Ile Asp Gly Asn Asn Gly Ile Asp Gly Glu
       210                 215                 220
   Gly Pro Tyr Ser Pro Glu Arg Ala Gly Ser Leu Pro Leu Val Asp Phe
   225                 230                 235                 240
   Val Ala Lys Val Leu Ala Glu Arg Leu Thr Leu Asp Gln Val Lys Lys
                   245                 250                 255
   Leu Leu Ala Ser Gln Ser Gly Leu Arg Ser Tyr Leu Asn Asp Ile Ser
                   260                 265                 270
   Ile Lys Asn Ile Val Thr Arg Ile Ala Glu Gly Asp Glu Thr Ala Lys
               275                 280                 285
   Phe Tyr Leu Asp Gly Met Ile Tyr Gln Ile Lys Lys Gln Ile Ala Glu
       290                 295                 300
   Met Ala Gly Val Leu Asn Gly Gln Val Asp Val Ile Ile Leu Thr Gly
   305                 310                 315                 320
   Gly Ala Ala Tyr Ala Thr Ala Val Thr Val Pro Leu Gln His Asp Leu
                   325                 330                 335
   Ala Trp Ile Ala Pro Val Val Val Arg Pro Gly Glu Met Glu Met Gln
                   340                 345                 350
   Ala Leu Tyr Glu Gly Val Met Arg Val Leu Asn His Glu Glu Pro Val
               355                 360                 365
   Arg Val Tyr Gln Ser Asp Ala Ser Thr Ile Lys Gly Gly Thr Gly Arg
       370                 375                 380
<210> 11
   <211> 370
   <212> PRT
   <213> Geobacillus sp. GHH01
<400> 11
   Met Glu Glu Gln Lys Phe Arg Ile Leu Thr Ile Asn Pro Gly Ser Thr
   1               5                   10                  15
   Ser Thr Lys Ile Gly Val Phe Glu Asn Glu Arg Pro Leu Leu Glu Lys
                   20                  25                  30
   Thr Ile Arg His Glu Ala Asp Val Leu Arg Gln Tyr Lys Thr Ile Ala
               35                  40                  45
   Asp Gln Tyr Glu Phe Arg Lys Gln Thr Ile Leu Gln Ala Leu Asp Glu
       50                  55                  60
   Glu Gly Ile Asn Leu Ser Lys Leu Ser Ala Val Cys Gly Arg Gly Gly
   65                  70                  75                  80
   Leu Leu Arg Pro Ile Glu Gly Gly Thr Tyr Arg Val Asn Glu Ala Met
                   85                  90                  95
   Leu Glu Asp Leu Arg Arg Gly Tyr Ser Gly Gln His Ala Ser Asn Leu
                   100                 105                 110
   Gly Gly Ile Leu Ala His Glu Ile Ala Ser Ala Leu Asn Ile Pro Ala
               115                 120                 125
   Phe Ile Val Asp Pro Val Val Val Asp Glu Leu Asp Pro Ile Ala Arg
       130                 135                 140
   Ile Ser Gly Phe Pro Leu Ile Glu Arg Arg Ser Ile Phe His Ala Leu
   145                 150                 155                 160
   Asn Gln Lys Ala Val Ala Arg Arg Val Ala Lys Gln Leu Gly Lys Arg
                   165                 170                 175
   Tyr Asp Glu Leu Asn Leu Ile Val Ala His Met Gly Gly Gly Ile Thr
               180                 185                 190
   Val Gly Ala His Lys Gln Gly Arg Val Val Asp Val Asn Asn Gly Leu
           195                 200                 205
   Asp Gly Glu Gly Pro Phe Ser Pro Glu Arg Ala Gly Thr Val Pro Ala
       210                 215                 220
   Gly Asp Leu Val Ala Leu Cys Phe Ser Gly Glu Tyr Tyr Arg Glu Glu
   225                 230                 235                 240
   Ile Met Asn Met Leu Val Gly Gly Gly Gly Leu Val Gly Tyr Leu Gly
                   245                 250                 255
   Thr Asn Asp Ala Val Lys Val Glu Asn Met Ile Glu Ala Gly Asp Glu
               260                 265                 270
   Lys Ala Lys Leu Val Tyr Glu Ala Met Ala Tyr Gln Val Ala Lys Glu
           275                 280                 285
   Ile Gly Ala Ala Ser Ala Val Leu Ser Gly Lys Val Asp Ala Ile Ile
       290                 295                 300
   Leu Thr Gly Gly Leu Ala Tyr Gly Lys Ser Phe Val Glu Gln Ile Thr
   305                 310                 315                 320
   Arg Arg Val Gln Trp Ile Ala Asp Val Ile Val His Pro Gly Glu Asn
                   325                 330                 335
   Glu Leu Gln Ala Leu Ala Glu Gly Ala Leu Arg Val Leu Arg Gly Glu
               340                 345                 350
   Glu Glu Glu Lys Val Tyr Pro Gly Glu Ala Val Ser Pro Ile Pro Ala
           355                 360                 365
   Arg Arg
       370
<210> 12
   <211> 286
   <212> PRT
   <213> Escherichia coli (strain K12)
<400> 12
   Met Ser Gln Ala Leu Lys Asn Leu Leu Thr Leu Leu Asn Leu Glu Lys
   1               5                   10                  15
   Ile Glu Glu Gly Leu Phe Arg Gly Gln Ser Glu Asp Leu Gly Leu Arg
               20                  25                  30
   Gln Val Phe Gly Gly Gln Val Val Gly Gln Ala Leu Tyr Ala Ala Lys
           35                  40                  45
   Glu Thr Val Pro Glu Glu Arg Leu Val His Ser Phe His Ser Tyr Phe
       50                  55                  60
   Leu Arg Pro Gly Asp Ser Lys Lys Pro Ile Ile Tyr Asp Val Glu Thr
   65                  70                  75                  80
   Leu Arg Asp Gly Asn Ser Phe Ser Ala Arg Arg Val Ala Ala Ile Gln
                   85                  90                  95
   Asn Gly Lys Pro Ile Phe Tyr Met Thr Ala Ser Phe Gln Ala Pro Glu
               100                 105                 110
   Ala Gly Phe Glu His Gln Lys Thr Met Pro Ser Ala Pro Ala Pro Asp
           115                 120                 125
   Gly Leu Pro Ser Glu Thr Gln Ile Ala Gln Ser Leu Ala His Leu Leu
       130                 135                 140
   Pro Pro Val Leu Lys Asp Lys Phe Ile Cys Asp Arg Pro Leu Glu Val
   145                 150                 155                 160
   Arg Pro Val Glu Phe His Asn Pro Leu Lys Gly His Val Ala Glu Pro
                   165                 170                 175
   His Arg Gln Val Trp Ile Arg Ala Asn Gly Ser Val Pro Asp Asp Leu
               180                 185                 190
   Arg Val His Gln Tyr Leu Leu Gly Tyr Ala Ser Asp Leu Asn Phe Leu
           195                 200                 205
   Pro Val Ala Leu Gln Pro His Gly Ile Gly Phe Leu Glu Pro Gly Ile
       210                 215                 220
   Gln Ile Ala Thr Ile Asp His Ser Met Trp Phe His Arg Pro Phe Asn
   225                 230                 235                 240
   Leu Asn Glu Trp Leu Leu Tyr Ser Val Glu Ser Thr Ser Ala Ser Ser
                   245                 250                 255
   Ala Arg Gly Phe Val Arg Gly Glu Phe Tyr Thr Gln Asp Gly Val Leu
               260                 265                 270
   Val Ala Ser Thr Val Gln Glu Gly Val Met Arg Asn His Asn
           275                 280                 285
<210> 13
   <211> 289
   <212> PRT
   <213> Pseudomonas putida
<400> 13
   Met Ser His Val Leu Asp Asp Leu Val Asp Leu Leu Ser Leu Glu Ser
   1               5                   10                  15
   Ile Glu Glu Asn Leu Phe Arg Gly Arg Ser Gln Asp Leu Gly Phe Arg
               20                  25                  30
   Gln Leu Tyr Gly Gly Gln Val Leu Gly Gln Ser Leu Ser Ala Ala Ser
           35                  40                  45
   Gln Thr Val Glu Asp Ala Arg His Val His Ser Leu His Gly Tyr Phe
       50                  55                  60
   Leu Arg Pro Gly Asp Ala Ser Leu Pro Val Val Tyr Ser Val Asp Arg
   65                  70                  75                  80
   Val Arg Asp Gly Gly Ser Phe Ser Thr Arg Arg Val Thr Ala Ile Gln
                   85                  90                  95
   Lys Gly Gln Thr Ile Phe Thr Cys Ser Ala Ser Phe Gln Tyr Asp Glu
               100                 105                 110
   Glu Gly Phe Glu His Gln Ala Gln Met Pro Asp Val Val Gly Pro Glu
           115                 120                 125
   Asn Leu Pro Thr Glu Val Glu Leu Ala His Ala Met Ala Asp Gln Leu
       130                 135                 140
   Pro Glu Arg Ile Arg Asp Lys Val Leu Cys Ala Lys Pro Ile Glu Ile
   145                 150                 155                 160
   Arg Pro Val Thr Glu Arg Asp Pro Phe Asn Pro Lys Pro Gly Asp Pro
                   165                 170                 175
   Val Lys Tyr Ala Trp Phe Arg Ala Asp Gly Asn Leu Pro Asp Val Pro
               180                 185                 190
   Ala Leu His Lys Tyr Met Leu Ala Tyr Ala Ser Asp Phe Gly Leu Leu
           195                 200                 205
   Thr Thr Ala Leu Leu Pro His Gly Lys Ser Val Trp Gln Arg Asp Met
       210                 215                 220
   Gln Ile Ala Ser Leu Asp His Ser Leu Trp Phe His Gly Asn Leu Arg
   225                 230                 235                 240
   Ala Asp Gln Trp Leu Leu Tyr Ala Thr Asp Ser Pro Trp Ala Gly Asn
                   245                 250                 255
   Ser Arg Gly Phe Cys Arg Gly Ser Ile Phe Asn Gln Ala Gly Gln Leu
               260                 265                 270
   Val Ala Ser Ser Ser Gln Glu Gly Leu Ile Arg His Arg Lys Asp Trp
           275                 280                 285
   Ala
<210> 14
   <211> 352
   <212> PRT
   <213> Pseudomonas mendocina (strain ymp)
<400> 14
   Met Ser Pro Ser Pro Ala Ser Leu Asn Asp Gln Gln Arg Ala Ala His
   1               5                   10                  15
   Ile Arg Glu Gln Val Met Ala His Gly Asn Ala Leu Arg Gln Arg Tyr
               20                  25                  30
   Pro Ile Leu Gln His Gln Asp Ala Leu Gly Ala Gly Ile Leu Ala Phe
           35                  40                  45
   Ala Leu Cys Gly Met Ile Gly Ser Ala Ala Leu Tyr Ile Gly Gly His
       50                  55                  60
   Leu Pro Trp Trp Ala Cys Leu Leu Leu Asn Ala Phe Phe Ala Ser Leu
   65                  70                  75                  80
   Thr His Glu Leu Glu His Asp Leu Ile His Ser Met Tyr Phe Arg Lys
                   85                  90                  95
   Gln Pro Leu Pro His Asn Leu Met Leu Ala Leu Val Trp Leu Ala Arg
               100                 105                 110
   Pro Ser Thr Ile Asn Pro Trp Val Arg Arg His Leu His Leu Asn His
           115                 120                 125
   His Lys Val Ser Gly Ser Glu Ala Asp Met Glu Glu Arg Ala Ile Thr
       130                 135                 140
   Asn Gly Glu Pro Trp Gly Ile Ala Arg Leu Leu Met Val Gly Asp Asn
   145                 150                 155                 160
   Met Met Ser Ser Phe Ile Arg Trp Leu Arg Ala Lys Asn Pro Glu His
                   165                 170                 175
   Arg Arg Leu Ile Leu Thr Arg Thr Leu Lys Val Tyr Ala Pro Leu Gly
               180                 185                 190
   Leu Leu Asn Trp Ala Thr Trp Tyr Leu Phe Leu Gly Phe His Leu Leu
           195                 200                 205
   Asp Trp Ala Ala Ala Ala Leu Gly Ala Pro Ile Ala Trp Ser Ala Ser
       210                 215                 220
   Thr Leu Ser Val Met Gln Val Val Asn Val Ala Val Val Val Leu Val
   225                 230                 235                 240
   Gly Pro Asn Val Leu Arg Thr Phe Cys Leu His Phe Val Ser Ser Asn
                   245                 250                 255
   Met His Tyr Tyr Gly Asp Val Glu Pro Gly Asn Val Ile Gln Gln Thr
               260                 265                 270
   Gln Val Leu Asn Pro Trp Trp Leu Trp Pro Leu Gln Ala Phe Cys Phe
           275                 280                 285
   Asn Phe Gly Ser Ser His Ala Ile His His Phe Val Val Lys Glu Pro
       290                 295                 300
   Phe Tyr Ile Arg Gln Leu Thr Val Pro Phe Ala His Arg Val Met Arg
   305                 310                 315                 320
   Glu Met Gly Val Arg Phe Asn Asp Phe Gly Thr Phe Ala Arg Ala Asn
                   325                 330                 335
   Arg Trp Thr Arg Arg Ala Arg Thr Gln Gln Glu Arg Ala Ser Thr Ala
               340                 345                 350
<210> 15
   <211> 351
   <212> PRT
   <213> Acinetobacter baumannii
<400> 15
   Met Thr Tyr Ile Tyr Lys Asn Pro Ala Gly Met Thr Asp Ser Glu Lys
   1               5                   10                  15
   Thr Glu His Ile Lys Lys Ile Val Thr Ala Glu Gly Val Ala Leu Arg
               20                  25                  30
   Lys Arg Tyr Pro Ile Leu Asn His Gln Asn Ala Ile Gly Ala Val Ile
           35                  40                  45
   Leu Phe Phe Ser Leu Ala Gly Met Ile Thr Thr Ala Val Leu Tyr Ile
       50                  55                  60
   Asn His Gln Leu Ser Ala Trp Phe Ala Ile Pro Ile Ile Ala Phe Phe
   65                  70                  75                  80
   Ala Ser Leu Thr His Glu Leu Glu His Asp Leu Ile His Trp Met Tyr
                   85                  90                  95
   Phe Arg Lys Lys Pro Trp Ala His His Leu Met Met Gly Leu Val Trp
               100                 105                 110
   Leu Ala Arg Pro Ser Thr Ile Asn Pro Trp Lys Arg Arg Glu Leu His
           115                 120                 125
   Phe Asn His His Lys Asn Ser Gly Thr Glu Val Asp Leu Glu Glu Arg
       130                 135                 140
   Ala Leu Thr Asn Gly Glu Gln Trp Ser Ile Arg Arg Leu Ile Ala Ile
   145                 150                 155                 160
   Gly Asp Asn Gly Leu Ala Val Leu Phe Arg Ile Ile Ser Ala Ser Asn
                   165                 170                 175
   Trp Thr Val Arg Lys Val Ile Phe Lys Arg Ala Phe Met Ala Tyr Phe
               180                 185                 190
   Pro Leu Gly Ile Ile His Trp Ser Leu Trp Tyr Ile Phe Leu Gly Phe
           195                 200                 205
   His Ala Val Asp Ala Val Leu Ser Trp Ala Asn Ala Pro Ile Ala Trp
       210                 215                 220
   Ser Ala Thr Thr Leu Asn Ile Met His Val Val Asn Ile Leu Thr Val
   225                 230                 235                 240
   Val Trp Val Ala Pro Asn Val Leu Arg Thr Phe Cys Leu His Phe Val
                   245                 250                 255
   Thr Ser Asn Met His Tyr Tyr Gly Asp Val Glu Leu Gly Asn Val Ile
               260                 265                 270
   Gln Gln Thr Gln Val Leu Lys Pro Trp Trp Met Met Pro Phe Gln Leu
           275                 280                 285
   Phe Cys Phe Asn Phe Gly Ser Thr His Ala Ile His His Phe Val Val
       290                 295                 300
   Lys Glu Pro Phe Tyr Ile Arg Gln Met Thr Ala Pro Val Ala His Lys
   305                 310                 315                 320
   Val Met Arg Asp Met Gly Val Arg Phe Asn Asp Val Gly Thr Phe Lys
                   325                 330                 335
   Arg Ala Asn Arg Trp Asn Ile Asn Asp Leu Ser Glu Ser Lys Ser
               340                 345                 350

## Description

The present invention relates to a method for the production of isobutene from 3-methylcrotonyl-CoA comprising the steps of:
(a) enzymatically converting 3-methylcrotonyl-CoA into 3-methylbutyric acid; and
(b) further enzymatically converting the thus produced 3-methylbutyric acid into isobutene, wherein the conversion of 3-methylbutyric acid into isobutene according to step (b) is achieved by a UndB fatty acid desaturase (trans-membrane non-heme iron oxygenase).

The conversion of 3-methylcrotonyl-CoA into 3-methylbutyric acid can be achieved by first enzymatically converting 3-methylcrotonyl-CoA into 3-methylbutyryl-CoA and further enzymatically converting the thus produced 3-methylbutyryl-CoA into 3-methylbutyric acid. Alternatively, the conversion of 3-methylcrotonyl-CoA into 3-methylbutyric acid can be achieved by first enzymatically converting 3-methylcrotonyl-CoA into 3-methylcrotonic acid and then further enzymatically converting the thus produced 3-methylcrotonic acid into 3-methylbutyric acid.

A large number of chemical compounds are currently derived from petrochemicals. Alkenes (such as ethylene, propylene, the different butenes, or else the pentenes, for example) are used in the plastics industry, for example for producing polypropylene or polyethylene, and in other areas of the chemical industry and that of fuels.

Butylene exists in four forms, one of which, isobutene (also referred to as isobutylene), enters into the composition of methyl-tert-butyl-ether (MTBE), an antiknock additive for automobile fuel. Isobutene can also be used to produce isooctene, which in turn can be reduced to isooctane (2,2,4-trimethylpentane); the very high octane rating of isooctane makes it the best fuel for so-called "gasoline" engines. Alkenes such as isobutene are currently produced by catalytic cracking of petroleum products (or by a derivative of the Fischer-Tropsch process in the case of hexene, from coal or gas). The production costs are therefore tightly linked to the price of oil. Moreover, catalytic cracking is sometimes associated with considerable technical difficulties which increase process complexity and production costs.

The production by a biological pathway of alkenes such as isobutene is called for in the context of a sustainable industrial operation in harmony with geochemical cycles. The first generation of biofuels consisted in the fermentative production of ethanol, as fermentation and distillation processes already existed in the food processing industry. The production of second generation biofuels is in an exploratory phase, encompassing in particular the production of long chain alcohols (butanol and pentanol), terpenes, linear alkanes and fatty acids. Two recent reviews provide a general overview of research in this field: Ladygina et al. (Process Biochemistry 41 (2006), 1001) and Wackett (Current Opinions in Chemical Biology 21 (2008), 187).

The conversion of isovalerate to isobutene by the yeast *Rhodotorula minuta* has been described (Fujii et al. (Appl. Environ. Microbiol. 54 (1988), 583)), but the efficiency of this reaction, less than 1 millionth per minute, or about 1 for 1000 per day, is far from permitting an industrial application. The reaction mechanism was elucidated by Fukuda et al. (BBRC 201 (1994), 516) and involves a cytochrome P450 enzyme which decarboxylates isovalerate by reduction of an oxoferryl group Fe^{V}=O. Large-scale biosynthesis of isobutene by this pathway seems highly unfavorable, since it would require the synthesis and degradation of one molecule of leucine to form one molecule of isobutene and the cytochrome P450 from Rhodotorula minuta is a membrane bound protein, poorly lending itself to a recombinant expression in bacteria in a soluble form. For all these reasons, it appears very unlikely that this pathway can serve as a basis for industrial exploitation. Other microorganisms have been described as being marginally capable of naturally producing isobutene from isovalerate; the yields obtained are even lower than those obtained with *Rhodotorula minuta* (Fukuda et al. (Agric. Biol. Chem. 48 (1984), 1679)).

Gogerty et al. (Appl. Environm. Microbiol. 76 (2010), 8004-8010) and van Leeuwen et al. (Appl. Microbiol. Biotechnol. 93 (2012), 1377-1387) describe the production of isobutene from acetoacetyl-CoA by enzymatic conversions wherein the last step of the proposed pathway is the conversion of 3-hydroxy-3-methylbutyric acid (also referred to as 3-hydroxyisovalerate (HIV)) by making use of a mevalonate diphosphate decarboxylase. This reaction for the production of isobutene from 3-hydroxy-3-methylbutyric acid is also described in WO2010/001078. In Gogerty et al. (loc. cit.) and in van Leeuwen et al. (loc. cit.) the production of 3-hydroxy-3-methylbutyric acid is proposed to be achieved by the conversion of 3-methylcrotonyl-CoA via 3-hydroxy-3-methylbutyryl-CoA. Van Leeuwen et al. (loc cit.) also describe alternative routes for producing isobutene from renewable resources, one of which comprises the conversion of isovalerate (3-methylbutyrate) into isobutene wherein the isovalerate is provided by a hydrolysis reaction from isovaleryl-CoA. The isovaleryl-CoA itself is proposed to be produced from 2-oxoisocaproate and CoA by making use of a 4-methyl-2-oxopentanoate:NAD⁺ oxidoreductase. In order to further improve the efficiency and variability of methods for producing isobutene from renewable resources, there is a need for alternative routes for the provision of precursors which can be used as a substrate for the enzymatic conversion into isobutene.

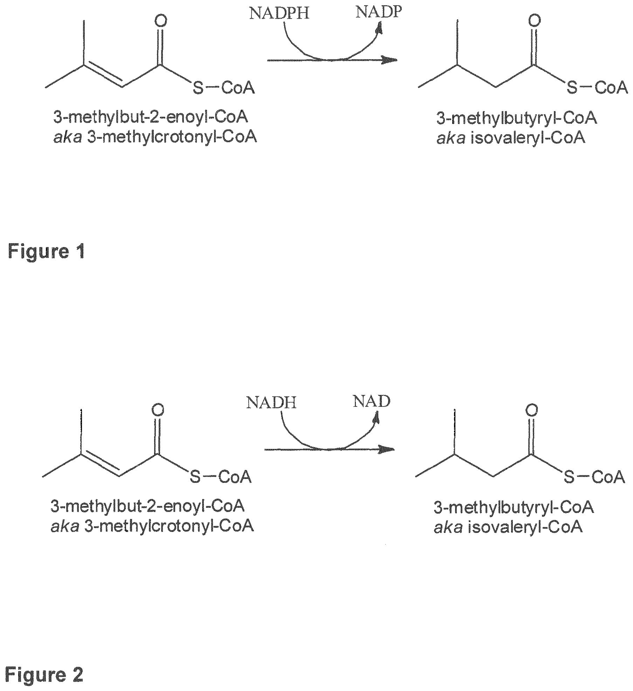

WO 2014/085612 (Invista North America) refers to methods for biosynthesis of isobutene that are illustrated in Figure 2 and wherein 3-methyl-3-hydroxybutanoate is converted to isobutene.

WO 2016/042011 (Global Bioenergies et al), published on 24 March 2016, is a related document with a similar teaching as the present document, except the use of UndB.

The present invention meets this demand by providing a method for the production of isobutene from 3-methylcrotonyl-CoA comprising the steps of:
(a) enzymatically converting 3-methylcrotonyl-CoA into 3-methylbutyric acid; and
(b) further enzymatically converting the thus produced 3-methylbutyric acid into isobutene, wherein the conversion of 3-methylbutyric acid into isobutene according to step (b) is achieved by a UndB fatty acid desaturase (trans-membrane non-heme iron oxygenase).

The conversion of 3-methylcrotonyl-CoA into 3-methylbutyric acid (isovalerate) according to step (a) provides an alternative route to that proposed in the prior art which converts 2-oxoisocaproate via 3-methylbutyryl-CoA (isovaleryl-CoA) into 3-methylbutyric acid (isovalerate).

According to the present invention the conversion of 3-methylcrotonyl-CoA into 3-methylbutyric and its subsequent conversion to isobutene can be achieved via different enzymatic routes, but all include the final conversion of 3-methylbutyric acid into isobutene by a UndB fatty acid desaturase (trans-membrane non-heme iron oxygenase). One possibility is to first convert 3-methylcrotonyl-CoA into 3-methylbutyryl-CoA and then to further convert 3-methylbutyryl-CoA into 3-methylbutyric acid. Another possibility is to first convert 3-methylcrotonyl-CoA into 3-methylcrotonic acid and then to further convert 3-methylcrotonic acid into 3-methylbutyric acid.

Thus, in one preferred embodiment the conversion of 3-methylcrotonyl-CoA into 3-methylbutyric acid is achieved by first converting 3-methylcrotonyl-CoA into 3-methylbutyryl-CoA and then to further convert 3-methylbutyryl-CoA into 3-methylbutyric acid. Accordingly, the present invention relates to a method for the production of isobutene from 3-methylcrotonyl-CoA comprising the steps of:
(a) enzymatically converting 3-methylcrotonyl-CoA into 3-methylbutyric acid; and
(b) further enzymatically converting the thus produced 3-methylbutyric acid into isobutene, wherein the conversion of 3-methylbutyric acid into isobutene according to step (b) is achieved by a UndB fatty acid desaturase (trans-membrane non-heme iron oxygenase). ,
wherein the enzymatic conversion of 3-methylcrotonyl-CoA into 3-methylbutyric acid according to step (a) is achieved by a method comprising the following steps:
(a1) enzymatically converting 3-methylcrotonyl-CoA into 3-methylbutyryl-CoA; and
(a2) further enzymatically converting the thus produced 3-methylbutyryl-CoA into 3-methylbutyric acid.

The enzymatic conversion of 3-methylcrotonyl-CoA into 3-methylbutyryl-CoA according to step (a1), i.e. the reduction of the double bond in 3-methylcrotonyl-CoA, can, for example, be achieved by employing an enzyme classified as EC 1.3._._. Enzymes classified as EC 1.3._._ are oxidoreductases acting on the CH-CH group of a donor molecule. This subclass contains enzymes that reversibly catalyze the conversion of a carbon-carbon single bond to a carbon-carbon double bond between two carbon atoms. Sub-classes of EC 1.3 are classified depending on the acceptor. In one preferred embodiment the enzyme is an enzyme which is classified as EC 1.3._._ and which uses NADH or NADPH as co-factor.

In one particularly preferred embodiment the enzyme is an enzyme which uses NADPH as a co-factor. The conversion using such an enzyme is schematically shown in Figure 1. In a preferred embodiment the enzyme is selected from the group consisting of:
- acyl-CoA dehydrogenase (NADP+) (EC 1.3.1.8);
- enoyl-[acyl-carrier-protein] reductase (NADPH, Si-specific) (EC 1.3.1.10);
- cis-2-enoyl-CoA reductase (NADPH) (EC 1.3.1.37);
- trans-2-enoyl-CoA reductase (NADPH) (EC 1.3.1.38);
- enoyl-[acyl-carrier-protein] reductase (NADPH, Re-specific) (EC 1.3.1.39); and
- crotonyl-CoA reductase (EC 1.3.1.86).

Thus, in one preferred embodiment of the claimed method, the conversion of 3-methylcrotonyl-CoA into 3-methylbutyryl-CoA is achieved by making use of an acyl-CoA dehydrogenase (NADP+) (EC 1.3.1.8). Acyl-CoA dehydrogenases are enzymes which catalyze the following reaction:

Acyl-CoA + NADP⁺ ⇄ 2,3-dehydroacyl-CoA + NADPH + H⁺

This enzyme occurs in a variety of organism, including eukaryotic and prokaryotic organisms, such as plants, animals, fungi and bacteria. The enzyme has, e.g., been described in Bos, taurus, Rattus novegicus, Mus musculus, Columba sp., Arabidopsis thaliana, Nicotiana benthamiana, Allium ampeloprasum, Euglena gracilis, Candida albicans, Streptococcus collinus, Rhodobacter sphaeroides and Mycobacterium smegmatis.

In a further preferred embodiment the conversion of 3-methylcrotonyl-CoA into 3-methylbutyryl-CoA is achieved by making use of an enoyl-[acyl-carrier-protein] reductase (NADPH, Si-specific) (EC 1.3.1.10). Enoyl-[acyl-carrier-protein] reductases (NADPH, Si-specific) are enzymes which catalyze the following reaction:

acyl-[acyl-carrier-protein] + NADP⁺ ⇄ trans-2,3-dehydroacyl-[acyl-carrier-protein] + NADPH + H⁺

This enzyme occurs in a variety of organism, including eukaryotic and prokaryotic organisms, such as plants, fungi and bacteria, The enzyme has, e.g., been described in Carthamus tinctorius, Candida tropicalis, Saccharomyces cerevisiae, Streptococcus collinus, Streptococcus pneumoniae, Staphylococcus aureus, Bacillus subtilis, Bacillus cereus, Porphyromonas gingivalis, Escherichia coli and Salmonella enterica.

In a further preferred embodiment the conversion of 3-methylcrotonyl-CoA into 3-methylbutyryl-CoA is achieved by making use of a cis-2-enoyl-CoA reductase (NADPH) (EC 1.3.1.37). Cis-2-enoyl-CoA reductases (NADPH) are enzymes which catalyze the following reaction:

Acyl-CoA + NADP⁺ ⇄ cis-2,3-dehydroacyl-CoA + NADPH + H⁺

This enzyme has been described to occur in Escherichia coli.

In a further preferred embodiment the conversion of 3-methylcrotonyl-CoA into 3-methylbutyryl-CoA is achieved by making use of a trans-2-enoyl-CoA reductase (NADPH) (EC 1.3.1.38). Trans-2-enoyl-CoA reductases (NADPH) are enzymes which catalyze the following reaction:

Acyl-CoA + NADP⁺ ⇄ trans-2,3-dehydroacyl-CoA + NADPH + H⁺

This enzyme occurs in a variety of organism, including eukaryotic and prokaryotic organisms, such as plants, animals and bacteria. The enzyme has, e.g., been described in Homo sapien, Rattus norvegicus, Mus musculus, Cavia porcellus, Caenorhabditis elegans, Phalaenopsis amabilis, Gossypium hirsutum, Mycobacterium tuberculosis, Streptococcus collinu and Escherichia coli.

In a further preferred embodiment the conversion of 3-methylcrotonyl-CoA into 3-methylbutyryl-CoA is achieved by making use of an enoyl-[acyl-carrier-protein] reductase (NADPH, Re-specific) (EC 1.3.1.39). Enoyl-[acyl-carrier-protein] reductases (NADPH, Re-specific) are enzymes which catalyze the following reaction:

acyl-[acyl-carrier-protein] + NADP⁺ ⇄ trans-2,3-dehydroacyl-[acyl-carrier-protein] + NADPH + H⁺

This enzyme occurs in a variety of organism, including eukaryotic and prokaryotic organisms, such as animals and bacteria. The enzyme has, e.g., been described in Gallus gallus, Pigeon, Rattus norvegicus, Cavia porcellus, Staphylococcus aureus, Bacillus subtilis and Porphyromonas gingivalis.

In a further preferred embodiment the conversion of 3-methylcrotonyl-CoA into 3-methylbutyryl-CoA is achieved by making use of a crotonyl-CoA reductase (EC 1.3.1.86). Crotonyl-CoA reductases are enzymes which catalyze the following reaction:

butanoyl-CoA + NADP⁺ ⇄ (E)-but-2-enoyl-CoA + NADPH + H⁺

This enzyme occurs in a variety of organism, including eukaryotic and prokaryotic organisms, such as animals, fungi and bacteria. The enzyme has, e.g., been described in Bos taurus, Salinospora tropica, Clostridium difficile, Streptomyces collinus, Streptomyces cinnamonensis and Streptomyces hygroscopicus.

In another particularly preferred embodiment the enzyme is an enzyme which uses NADH as a co-factor. The conversion using such an enzyme is schematically shown in Figure 2. In a preferred embodiment the enzyme is selected from the group consisting of:
- enoyl-[acyl-carrier-protein] reductase (NADH) (EC 1.3.1.9); and
- trans-2-enoyl-CoA reductase (NAD⁺) (EC 1.3.1.44).

Thus, in one preferred embodiment the conversion of 3-methylcrotonyl-CoA into 3-methylbutyryl-CoA is achieved by making use of an enoyl-[acyl-carrier-protein] reductase (NADH) (EC 1.3.1.9). Enoyl-[acyl-carrier-protein] reductases (NADH) are enzymes which catalyze the following reaction:

acyl-[acyl-carrier-protein] + NAD⁺ ⇄ trans-2,3-dehydroacyl-[acyl-carrier-protein] + NADH + H⁺

This enzyme occurs in a variety of organism, including eukaryotic and prokaryotic organisms, such as plants and bacteria. The enzyme has, e.g., been described in Arabidopsis thaliana, Plasmodium falciparum, Eimeria tenella, Toxoplasma gondii, Mycobacterium tuberculosis, Streptococcus pneumoniae, Escherichia coli, Staphylococcus aureus, Bacillus anthracis, Birkholderia mallei, Pseudomonas aeruginosa, Helicobacter pylori, Yersinia pestis and many others.

In a further preferred embodiment the conversion of 3-methylcrotonyl-CoA into 3-methylbutyryl-CoA is achieved by making use of a trans-2-enoyl-CoA reductase (NAD⁺) (EC 1.3.1.44). Trans-2-enoyl-CoA reductases (NAD⁺) are enzymes which catalyze the following reaction:

Acyl-CoA + NAD⁺ ⇄ trans-2,3-dehydroacyl-CoA + NADH + H⁺

This enzyme occurs in a variety of organism, including eukaryotic and prokaryotic organisms, such as plants, animals and bacteria. The enzyme has, e.g., been described in Ratus norvegicus, Euglena gracilis, Mycobacterium smegmatis, Pseudomonas fluorescens, Clostridium acetobutylicum, Butyrivibrio fibrisolvens, Pseudomonas aeruginosa, Mycobacterium tuberculosis and Treponema denticola.

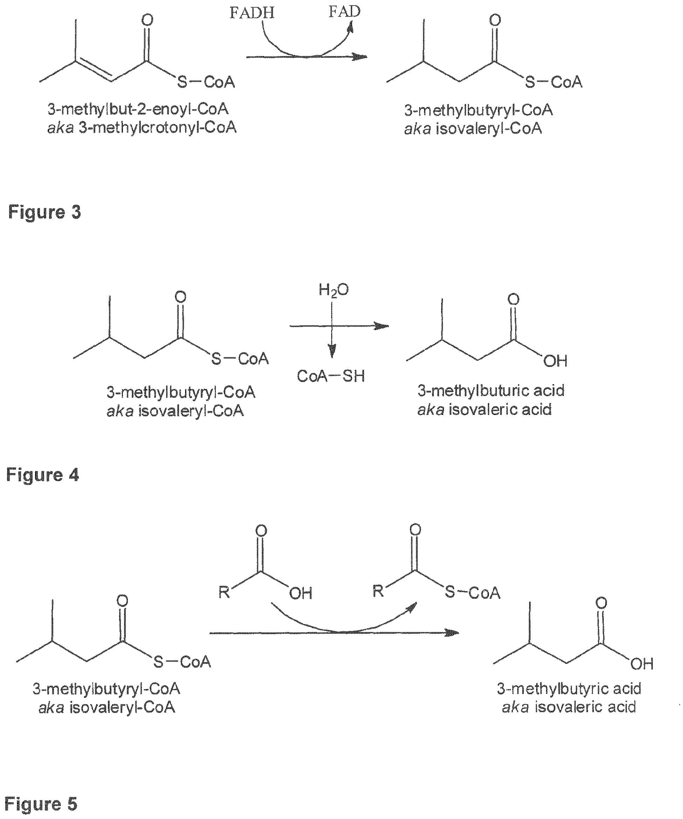

In another preferred embodiment the enzyme is an enzyme which is classified as EC 1.3.8 and which uses a flavin prosthetic group as acceptor. The conversion using such an enzyme is schematically shown in Figure 3. In a preferred embodiment the enzyme is an isovaleryl-CoA dehydrogenase (EC 1.3.8.4). Isovaleryl-CoA dehydrogenases are enzymes which catalyze the following reaction:

Isovaleryl-CoA + electron-transfer flavoprotein ⇄ 3-methylcrotonyl-CoA + reduced electron-transfer flavoprotein

This enzyme occurs in a variety of organism, including eukaryotic and prokaryotic organisms, such as plants, animals, fungi and bacteria. The enzyme has, e.g., been described in Homo sapiens, Bos taurus, Rattus norvegicus, Mus musculus, Cavia porcellus, Bombix mori, Caenorhabditis elegans, Solanum tuberosum, Arabidopsis thaliana, Pisum sativum, Aspergillus oryzae, Pseudomonas aeruginosa and Halobacterium salinarum.

In another embodiment the conversion of 3-methylcrotonyl-CoA into 3-methylbutyryl-CoA may, for example, be achieved by making use of the enzyme from Myxococcus sp. which is encoded by the liuA gene (Li et al., Angew. Chem. Int. Ed. 52 (2013), 1304-1308). This enzyme (AibC, LiuA) was annotated as an oxidoreductase and as belonging to the zinc-binding dehydrogenase family. The enzyme was shown to reduce 3-methylcrotonyl-CoA into 3-methylbutyryl-CoA using NADH as co-factor. The amino acid sequence of said protein is available under Uniprot Accession Number Q1D4I2.

In a preferred embodiment such an enzyme has an amino acid sequence as shown in SEQ ID NO: 3 or shows an amino acid sequence which is at least x% homologous to SEQ ID NO: 3 and has the activity of an oxidoreductase with x being an integer between 30 and 100, preferably 35, 40, 45, 50, 55, 60, 65, 70, 75, 80, 85, 90, 91, 92, 93, 94, 95, 96, 97, 98 or 99 wherein such an enzyme is capable of converting 3-methylcrotonyl-CoA into 3-methylbutyryl-CoA as set forth herein above.

Preferably, the degree of identity is determined by comparing the respective sequence with the amino acid sequence of any one of the above-mentioned SEQ ID NOs. When the sequences which are compared do not have the same length, the degree of identity preferably either refers to the percentage of amino acid residues in the shorter sequence which are identical to amino acid residues in the longer sequence or to the percentage of amino acid residues in the longer sequence which are identical to amino acid residues in the shorter sequence. The degree of sequence identity can be determined according to methods well known in the art using preferably suitable computer algorithms such as CLUSTAL.

When using the Clustal analysis method to determine whether a particular sequence is, for instance, 80% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.

Preferably, the degree of identity is calculated over the complete length of the sequence.

The conversion of 3-methylbutyryl-CoA into 3-methylbutyric acid according to step (a2) can be achieved by different enzymatic conversions. One possibility is the direct conversion via a hydrolysis reaction. Another possibility is the direct conversion via a reaction catalyzed by a CoA-transferase and a third possibility is a two-step conversion via 3-methylbutyryl phosphate. These three options will be further discussed in the following.

In one embodiment the conversion of 3-methylbutyryl-CoA into 3-methylbutyric acid according to step (a2) can be achieved by a method comprising the step of enzymatically converting 3-methylbutyryl-CoA into 3-methylbutyric acid in a hydrolysis reaction by making use of a thioester hydrolase (EC 3.1.2; in the following referred to as thioesterase).

Thioesterases (TEs; also referred to as thioester hydrolases) are enzymes which are classified as EC 3.1.2. Presently thioesterases are classified as EC 3.1.2.1 through EC 3.1.2.27 and EC 3.1.2.- for unclassified TEs. Cantu et al. (Protein Science 19 (2010), 1281-1295) describe that there are 23 families of thioesterases which are unrelated to each other as regards the primary structure. However, it is assumed that all members of the same family have essentially the same tertiary structure. Thioesterases hydrolyze the thioester bond between a carbonyl group and a sulfur atom.

The conversion of 3-methylbutyryl-CoA into 3-methylbutyric acid catalyzed by a thioesterase is schematically shown in Figure 4.

In a preferred embodiment, a thioesterase employed in a method according to the present invention for converting 3-methylbutyryl-CoA into 3-methylbutyric acid is selected from the group consisting of:
- acetyl-CoA hydrolase (EC 3.1.2.1);
- palmitoyl-CoA hydrolase (EC 3.1.2.2);
- 3-hydroxyisobutyryl-CoA hydrolase (EC 3.1.2.4);
- oleoyl-[acyl-carrier-protein] hydrolase (EC 3.1.2.14);
- ADP-dependent short-chain-acyl-CoA hydrolase (EC 3.1.2.18);
- ADP-dependent medium-chain-acyl-CoA hydrolase (EC 3.1.2.19); and
- acyl-CoA hydrolase (EC 3.1.2.20).

Thus, in one preferred embodiment the direct conversion of 3-methylbutyryl-CoA into 3-methylbutyric acid is achieved by making use of an acetyl-CoA hydrolase (EC 3.1.2.1). Acetyl-CoA hydrolases are enzymes which catalyze the following reaction:

Acetyl-CoA + H₂O → acetate + CoA

This enzyme occurs in a variety of organism, including eukaryotic and prokaryotic organisms, such as plants, animals, fungi and bacteria. The enzyme has, e.g., been described in Rattus norvegicus (Uniprot Accession number: Q99NB7), Mus musculus, Sus scrofa, Bos taurus, Gallus gallus, Platyrrhini, Ovis aries, Mesocricetus auratus, Ascaris suum, Homo sapiens (Uniprot Accession number: Q8WYK0), Pisum sativum, Cucumis sativus, Panicus sp., Ricinus communis, Solanum tuberosum, Spinacia oleracea, Zea mays, Glycine max, Saccharomyces cerevisiae, Neurospora crassa, Candida albicans, Trypanosoma brucei brucei, Trypanosoma cruzi, Trypanosoma dionisii, Trypanosoma vespertilionis, Crithidia fasciculate, Clostridium aminovalericum, Acidaminococcus fermaentans, Bradyrhizobium japonicum and Methanosarcina barkeri.

In another preferred embodiment the direct conversion of 3-methylbutyryl-CoA into 3-methylbutyric acid is achieved by making use of a palmitoyl-CoA hydrolase (EC 3.1.2.2). Palmitoyl-CoA hydrolases are enzymes which catalyze the following reaction:

Palmitoyl-CoA + H₂O → palmitate + CoA

This enzyme occurs in a variety of organism, including eukaryotic and prokaryotic organisms, such as plants, animals, fungi and bacteria. The enzyme has, e.g., been described in Arabidopsis thaliana (Uniprot Accession number: Q8GYW7), Pisum sativum, Spinacia oleracea, Bumilleriopsis filiformis, Eremosphaera viridis, Mougeotia scalaris, Euglena gracilis, Rhodotorula aurantiaca, Saccharaomyces cerevisiae, Candida rugosa, Caenorhabditis elegans, Mus musculus (Uniprot Accession number: P58137), Homo sapiens, Platyrrhini, Bos taurus, Canis lupus familiaris, Sus scrofa, Cavia procellus, Columba sp., Cricetulus griseus, Mesocricetus auratus, Drosophila melanogaster, Rattus norvegicus, Oryctolagus cuniculus, Gallus gallus, Anas platyrhynchos, Mycobacterium tuberculosis, Mycobacterium phlei, Mycobacterium smegmatis, Acinetobacter colcaceticus, Haemophilus influenza, Helicobacter pylori, Bacillus subtilis, Pseudomonas aeruginosa, Rhodobacter shpaeroides, Streptomyces coelicolor, Streptomyces venezuelae and E. coli.

In a further preferred embodiment the direct conversion of 3-methylbutyryl-CoA into 3-methylbutyric acid is achieved by making use of a 3-hydroxyisobutyryl-CoA hydrolase (EC 3.1.2.4). 3-hydroxyisobutyryl-CoA hydrolases are enzymes which catalyze the following reaction:

3-hydroxyisobutyryl-CoA + H₂O → 3-hydroxyisobutyrate + CoA

This enzyme occurs in a variety of organism, including eukaryotic and prokaryotic organisms, such as plants, animals, fungi and bacteria. The enzyme has, e.g., been described in Arabidopsis thaliana, Homo sapiens, Canus lupus familiaris, Rattus norvegicus, Bacillus cereus, Pseudomonas fluorescens and Pseudomonas aeruginosa.

In yet another preferred embodiment the direct conversion of 3-methylbutyryl-CoA into 3-methylbutyric acid is achieved by making use of an oleoyl-[acyl-carrier-protein] hydrolase (EC 3.1.2.14). Oleoyl-[acyl-carrier-protein] hydrolases are enzymes which catalyze the following reaction:

oleoyl-[acyl-carrier-protein] + H₂O → oleate + [acyl-carrier-protein]

This enzyme occurs in a variety of plants and bacteria. The enzyme has, e.g., been described in Arabidopsis thaliana, Allium ampeloprasum, Curcurbita moschata, Cuphea calophylla, Cuphea hookeriana, Cuphea lanceolata, Cuphea wrightii, Umbellularia californica, Coriandrum sativum, Spinacia oleracea, Elaeis sp., Elaeis guineensis, Glycine max, Persea americana, Pisum sativum, Sinapis alba, Ulmus americana, Zea mays, Brassica juncea, Brassica napus, Brassica rapa subsp. campestris, Jatropha curcas, Ricinus communis, Cinnamomum camphorum, Macadamia tetraphylla, Magnifera indica, Madhuca longifolia, Populus tomentosa, Chimonanthus praecox, Gossypium hirsutum, Diploknema butyracea, Helianthus annuus and Streptococcus pyogenes.

In yet another preferred embodiment the direct conversion of 3-methylbutyryl-CoA into 3-methylbutyric acid is achieved by making use of an ADP-dependent short-chain-acyl-CoA hydrolase (EC 3.1.2.18). ADP-dependent short-chain-acyl-CoA hydrolases are enzymes which catalyze the following reaction:

an acyl-CoA + H₂O → a carboxylate + CoA

This enzyme occurs in a variety of animals and has, e.g., been described in Mus musculus, Rattus norvegicus and Mesocricetus auratus.

In yet another preferred embodiment the direct conversion of 3-methylbutyryl-CoA into 3-methylbutyric acid is achieved by making use of an ADP-dependent medium-chain-acyl-CoA hydrolase (EC 3.1.2.19). ADP-dependent medium-chain-acyl-CoA hydrolases are enzymes which catalyze the following reaction:

an acyl-CoA + H₂O → a carboxylate + CoA

This enzyme occurs in a variety of animals and has, e.g., been described in Rattus norvegicus and Mesocricetus auratus.

In a further preferred embodiment the direct conversion of 3-methylbutyryl-CoA into 3-methylbutyric acid is achieved by making use of an acyl-CoA hydrolase (EC 3.1.2.20). Acyl-CoA hydrolases are enzymes which catalyze the following reaction:

an acyl-CoA + H₂O → a carboxylate + CoA

This enzyme occurs in a variety of organism, including eukaryotic and prokaryotic organisms, such as plants, animals, fungi and bacteria. The enzyme has, e.g., been described in Arabidopsis thaliana, Rhodotorula aurantiaca, Bumilleriopsis filiformis, Eremosphaera viridis, Euglena gracilis, Mus musculus, Rattus norvegicus, Homo sapiens, Sus, scrofa, Bos taurus, Cais lupus familiaris, Cavia porcellus, Cricetus griseus, Drosophila melanogaster, Anas platyrhynchos, Gallus gallus, Caenorhabditis elegans, Saccharomyces cerevisia, Candida rugosa, Escherichia coli, Haemophilus influenzae, Xanthomonas campestris, Streptomyces sp., Streptomyces coelicolor, Alcaligenes faecalis, Pseudomonas aeruginosa, Amycolatopsis mediterranei, Acinetobacter calcoaceticus, Helicobacter pylori, Rhodobacter spaeroides and Mycobacterium phlei.

In a preferred embodiment the acyl-CoA hydrolase is an enzyme from Escherichia coli or from Haemophilus influenza, more preferably the YciA enzyme from E. coli or its closely related homolog HI0827 from Haemophilus influenza (Zhuang et al., Biochemistry 47 (2008), 2789-2796). In another preferred embodiment the acetyl-CoA hydrolase is an enzyme from Homo sapiens (Cao et al., Biochemistry 48 (2009),1293-1304). The enzyme from Haemophilus influenza has been reported to be able to catalyze the conversion of 3-methylbutyryl-CoA into 3-methylbutyric acid (Zhuang et al., loc.cit.).

In another embodiment the conversion of 3-methylbutyryl-CoA into 3-methylbutyric acid according to step (a2) can be achieved by a method comprising the step of enzymatically converting 3-methylbutyryl-CoA into 3-methylbutyric acid in a transferase reaction by making use of a CoA-transferase (EC 2.8.3).

The conversion of 3-methylbutyryl-CoA into 3-methylbutyric acid catalyzed by a CoA-transferase is schematically shown in Figure 5.

CoA-transferases are found in organisms from all lines of descent. Most of the CoA-transferase belong to two well-known enzyme families (referred to in the following as families I and II) and there exists a third family which had been identified in anaerobic metabolic pathways of bacteria. A review describing the different families can be found in Heider (FEBS Letters 509 (2001), 345-349).

Family I contains, e.g., the following CoA-transferases:
For 3-oxo acids: enzymes classified in EC 2.8.3.5 or EC 2.8.3.6;
for short chain fatty acids: enzymes classified in EC 2.8.3.8 or EC 2.8.3.9;
for glutaconate: enzymes classified in EC 2.8.3.12;
for succinate: succinyl-CoA:acetate CoA-transferases, i.e. enzymes classified in EC 2.8.3.18 (see also Mullins et al., Biochemistry 51(2012), 8422-34; Mullins et al., J. Bacteriol. 190 (2006), 4933-4940).

Most enzymes of family I naturally use succinyl-CoA or acetyl-CoA as CoA donors. These enzymes contain two dissimilar subunits in different aggregation states. Two conserved amino acid sequence motives have been identified:
Prosites entry PS01273 (http://prosite.expasy.org/cgi-bin/prosite/prosite-search-ac?PDOC00980)
COA_TRANSF_1,http://prosite.expasy.org/PS01273"; Coenzyme A transferases signature 1 (PATTERN)

Consensus pattern:
[DN]-[GN]-x(2)-[LIVMFA](3)-G-G-F-x(3)-G-x-P
   and
Prosites entries PS01273 (http://prosite.expasy.org/cgi-bin/prosite/prosite-search-ac?PDOC00980)
COA_TRANSF_2, PS01274; Coenzyme A transferases signature 2 (PATTERN)

### Consensus pattern:

[LF]-[HQ]-S-E-N-G-[LIVF](2)-[GA]
E (glutamic acid) is an active site residue.

The family II of CoA-transferases consists of the homodimeric α-subunits of citrate lyase (EC 2.8.3.10) and citramalate lyase (EC 2.8.3.11). These enzymes catalyse the transfer of acyl carrier protein (ACP) which contains a covalently bound CoA-derivative. It was shown that such enzymes also accept free CoA-thioester in vitro, such as acetyl-CoA, propionyl-CoA, butyryl-CoA in the case of citrate lyase (Dimroth et al., Eur. J. Biochem. 80 (1977), 479-488) and acetyl-CoA and succinyl-CoA in the case of citramalate lyase (Dimroth et al., Eur. J. Biochem. 80 (1977), 469-477). According to Heider (loc. cit.) family III of CoA-transferases consists of formyl-CoA: oxalate CoA-transferase, succinyl-CoA:(*R*)-benzylsuccinate CoA-transferase, (E)-cinnamoyl-CoA:(*R*)-phenyllactate CoA-transferase and butyrobetainyl-CoA:(R)-carnitine CoA-transferase. A further member of family III is succinyl-CoA:L-malate CoA-transferase whose function in autrophic CO₂ fixation of Chloroflexus aurantiacus is to activate L-malate to its CoA thioester with succinyl-CoA as the CoA donor (Friedman S. et al. J. Bacteriol. 188 (2006), 2646-2655). The amino acid sequences of the CoA-tranferase of this family show only a low degree of sequence identity to those of families I and II. These CoA-transferases occur in prokaryotes and eukaryotes.

In a preferred embodiment the CoA-transferase employed in a method according to the present invention, as defined in the claims, is a CoA-transferase which belongs to family I or II as described herein-above.

Preferably, the CoA-transferase employed in a method according to the present invention for the direct conversion of 3-methylbutyryl-CoA into 3-methylbutyric acid is selected from the group consisting of:
- acetate CoA-transferase (EC 2.8.3.8); and
- butyrate-acetoacetate CoA-transferase (EC 2.8.3.9).

Thus, in one preferred embodiment the direct conversion of 3-methylbutyryl-CoA into 3-methylbutyric acid is achieved by making use of an acetate CoA-transferase (EC 2.8.3.8). Acetate CoA-transferases are enzymes which catalyze the following reaction:

Acyl-CoA + acetate ⇄ a fatty acid anion + acetyl-CoA

This enzyme occurs in a variety of bacteria and has, e.g., been described in Anaerostipes caccae, Eubacterium hallii, Faecalibacterium prausnitzii, Roseburia hominis, Roseburia intestinalis, Coprococcus sp. and Escherichia coli,

In another preferred embodiment the direct conversion of 3-methylbutyryl-CoA into 3-methylbutyric acid is achieved by making use of a butyrate-acetoacetate CoA-transferase (EC 2.8.3.9). Butyrate-acetoacetate CoA-transferase are enzymes which catalyze the following reaction:

Butanoyl-CoA + acetoacetate ⇄ butanoate + acetoacetyl-CoA

This enzyme occurs in a variety of organism, including eukaryotic and prokaryotic organisms, such as animals and bacteria. The enzyme has, e.g., been described in Bos taurus, Clostridium sp. and Escherichia coli.

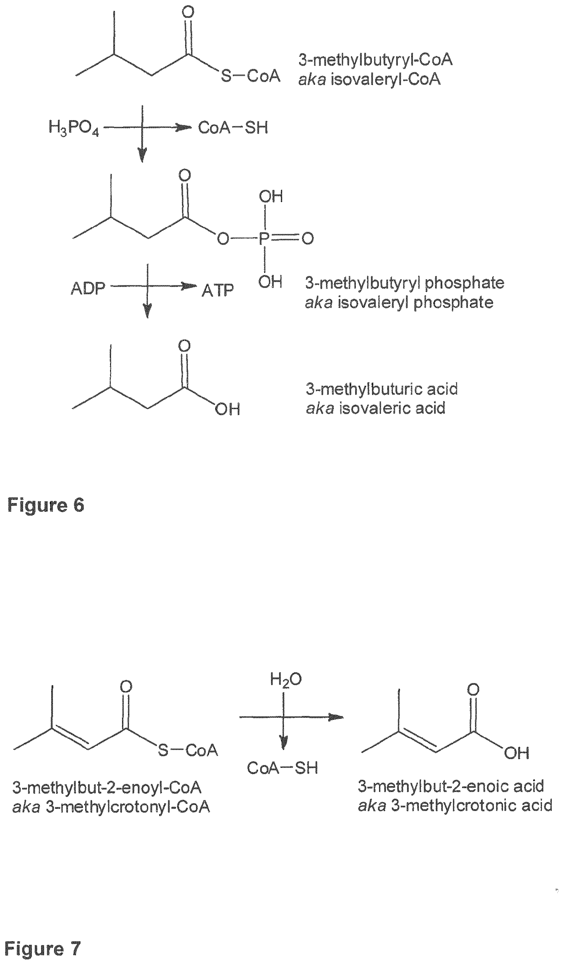

As mentioned above, the conversion of 3-methylbutyryl-CoA into 3-methylbutyric acid can in the alternative also be achieved by a conversion which first encompasses the conversion of 3-methylbutyryl-CoA into 3-methylbutyryl phosphate and the subsequent conversion of 3-methylbutyryl phosphate into 3-methylbutyric acid. The corresponding reaction is schematically shown in Figure 6. This pathway has the advantage of generating one molecule of ATP and is, thus, energetically more favourable for a cell if carried out in vivo.

The conversion of 3-methylbutyryl-CoA into 3-methylbutyryl phosphate can, e.g., be achieved by the use of a phosphate butyryltransferase (EC 2.3.1.19) or a phosphate acetyltransferase (EC 2.3.1.8).

Phosphate butyryltransferase (EC 2.3.1.19) naturally catalyzes the following reaction

Butyryl-CoA + H₃PO₄ ⇄ butyryl phosphate + CoA

It has been described by Wiesenborn et al. (Appl. Environ. Microbiol. 55 (1989), 317-322) and by Ward et al. (J. Bacteriol. 181 (1999), 5433-5442) that phosphate butyryltransferases (EC 2.3.1.19) can use a number of substrates in addition to butyryl coenzyme A (butyryl-CoA), in particular acetyl-CoA, propionyl-CoA, isobutyryl-CoA, valeryl-CoA and isovaleryl-CoA.

The enzyme has been described to occur in a number of organism, in particular in bacteria and in protozoae. In one embodiment the enzyme is from the protozoae Dasytricha ruminantium. In a preferred embodiment the phosphate butyryltransferase is a phosphate butyryltransferase from a bacterium, preferably from a bacterium of the genus Bacillus, Butyrivibrio, Enterococcus or Clostridium, more preferably Enterococcus or Clostridium, and even more preferably from Bacillus megaterium, Bacillus subtilis, Butyrivibrio fibrisolvens, Clostridium acetobutylicum, Clostridium beijerinckii, Clostridium butyricum, Clostridium kluyveri, Clostridium saccharoacetobutylicum, Clostridium sprorogenes or Enterococcus faecalis. Most preferably, the enzyme is from Bacillus subtilis (strain 168) (Uniprot Accession number P54530), Clostridium acetobutylicum, in particular the enzyme encoded by the ptb gene (Uniprot Accession number F0K6W0; Wiesenborn et al. (Appl. Environ. Microbiol. 55 (1989), 317-322)) or from Enterococcus faecalis, in particular Enterococcus faecalis MTUP9 (Uniprot Accession number K4YRE8 or Uniprot Accession number A0A038BNC2; Ward et al. (J. Bacteriol. 181 (1999), 5433-5442)). The sequences available for the phosphate butyryltransferase from Enterococcus faecalis under Uniprot Accession number K4YRE8 and Uniprot Accession number A0A038BNC2 have a sequence homology of 99.3%. The sequences available for the phosphate butyryltransferase from Enterococcus faecalis under Uniprot Accession number A0A038BNC2 is the more preferred one.

As mentioned, in a preferred embodiment, the enzyme is a phosphate butyryltransferase (EC 2.3.1.19) from Bacillus subtilis (strain 168) (Uniprot Accession number P54530). In a particularly preferred embodiment, the phosphate butyryltransferase (EC 2.3.1.19) employed in the method of the invention has an amino acid sequence as shown in SEQ ID NO: 8 or shows an amino acid sequence which is at least x% homologous to SEQ ID NO: 8 and has the activity of a phosphate butyryltransferase with x being an integer between 30 and 100, preferably 35, 40, 45, 50, 55, 60, 65, 70, 75, 80, 85, 90, 91, 92, 93, 94, 95, 96, 97, 98 or 99 wherein such an enzyme is capable of converting 3-methylcrotonyl-CoA into 3-methylcrotonyl-phosphate as set forth herein above.

In another preferred embodiment, as mentioned, the enzyme is a phosphate butyryltransferase (EC 2.3.1.19) from Enterococcus faecalis MTUP9 (Uniprot Accession number K4YRE8 or Uniprot Accession number A0A038BNC2). In a particularly preferred embodiment, the phosphate butyryltransferase (EC 2.3.1.19) employed in the method of the invention has an amino acid sequence as shown in SEQ ID NO: 9 or shows an amino acid sequence which is at least x% homologous to SEQ ID NO: 9 and has the activity of a phosphate butyryltransferase with x being an integer between 30 and 100, preferably 35, 40, 45, 50, 55, 60, 65, 70, 75, 80, 85, 90, 91, 92, 93, 94, 95, 96, 97, 98 or 99 wherein such an enzyme is capable of converting 3-methylcrotonyl-CoA into 3-methylcrotonyl-phosphate as set forth herein above.

As regards the determination of the degree of identity, the same applies as has been set forth herein above.

Phosphate acetyltransferase (EC 2.3.1.8) naturally catalyzes the following reaction

Acetyl-CoA + H₃PO₄ ⇄ acetyl phosphate + CoA

It has been described by Veit et al. (J. Biotechnol.140 (2009), 75-83) that phosphate acetyltransferase can also use as a substrate butyryl-CoA or propionyl-CoA.

The accession numbers for this enzyme family in InterPro database are IPR012147 and IPR002505, "http://www.ebi.ac.uk/interpro/entry/IPR002505" (http://www.ebi.ac.uk/interpro/entry/IPR012147 http://www.ebi.ac.uk/interpro/entry/IPR002505)

See also http://pfam.sanger.ac.uk/family/PF01515

The enzyme has been described in a variety of organisms, in particular bacteria and fungi. Thus, in one preferred embodiment the enzyme is an enzyme from a bacterium, preferably of the genus Escherichia, Chlorogonium, Clostridium, Veillonella, Methanosarcina, Corynebacterium, Ruegeria, Salmonella, Azotobacter, Bradorhizobium, Lactobacillus, Moorella, Rhodopseudomonas, Sinorhizobium, Streptococcus, Thermotoga or Bacillus, more preferably of the species Escherichia coli, Chlorogonium elongatum, Clostridium kluyveri, Clostridium acetobutylicum, Clostridium acidurici, Veillonella parvula, Methanosarcina thermophila, Corynebacterium glutamicum, Ruegeria pomeroyi, Salmonella enterica, Azotobacter vinelandii, Bradyrhizobium japonicum, Lactobacillus fermentum, Lactobacillus sanfranciscensis, Moorella thermoacetica, Rhodopseudomonas palustris, Sinorhizobium meliloti, Streptococcus pyogenes, Thermotoga maritima or Bacillus subtilis. In another preferred embodiment the enzyme is an enzyme from a fungus, preferably from the genus Saccharomyces, more preferably of the species Saccharomyces cerevisiae.

The conversion of 3-methylbutyryl phosphate into 3-methylbutyric acid can, e.g., be achieved by the use of a butyrate kinase (EC 2.7.2.7), a branched-chain-fatty-acid kinase (EC 2.7.2.14), a propionate kinase (EC 2.7.2.15) or an acetate kinase (EC 2.7.2.1).

Butyrate kinases (EC 2.7.2.7) naturally catalyze the following reaction

Butyrate + ATP ⇄ butyryl phosphate + ADP

It has been described, e.g. by Hartmanis (J. Biol. Chem. 262 (1987), 617-621) that butyrate kinase can use a number of substrates in addition to butyrate, e.g. valerate, isobutyrate, isovalerate and vinyl acetate. The enzyme has been described in a variety of organisms, in particular bacteria. In one preferred embodiment the enzyme is from a bacterium, preferably from a bacterium of the genus Clostridium, Butyrivibrio, Thermotoga, Enterococcus, Lactobacillus or Geobacillus. Preferred is Clostridium, Lactobacillus or Geobacillus. More preferably the enzyme is from a bacterium of the species Clostridium acetobutylicum, Clostridium proteoclasticum, Clostridium tyrobutyricum, Clostridium butyricum, Clostridium pasteurianum, Clostridium tetanomorphum, Butyrivibrio firbrosolvens, Butyrivibrio hungatei, Thermotoga maritime, Enterococcus durans, Enterococcus feacalis, Lactobacillus casei (Uniprot Accession number K0N529) or Geobacillus sp. (Uniprot Accession number L8AOE1). Preferred is Clostridium acetobutylicum, Lactobacillus casei W56 or Geobacillus sp. GHH01. For Clostridium acetobutylicum two butyrate kinases have been described: butyrate kinase 1 (Uniprot Accession number: Q45829) and butyrate kinase II (Uniprot Accession number: Q97II19). Preferably, the enzyme is an enzyme encoded by the buk gene from Clostridium acetobutylicum (Hartmanis (J. Biol. Chem. 262 (1987), 617-621)) or the homologue of this enzyme which has been found in Enterococcus feacalis (Ward et al. (J. Bacteriol. 181 (1999), 5433-5442)).

As mentioned, in a preferred embodiment, the enzyme is a butyrate kinase (EC 2.7.2.7) from Lactobacillus casei W56 (Uniprot Accession number K0N529). In a particularly preferred embodiment, the butyrate kinase (EC 2.7.2.7) employed in the method of the invention has an amino acid sequence as shown in SEQ ID NO: 10 or shows an amino acid sequence which is at least x% homologous to SEQ ID NO: 10 and has the activity of a butyrate kinase with x being an integer between 30 and 100, preferably 35, 40, 45, 50, 55, 60, 65, 70, 75, 80, 85, 90, 91, 92, 93, 94, 95, 96, 97, 98 or 99 wherein such an enzyme is capable of converting 3-methylcrotonyl-phosphate into 3-methylcrotonic acid as set forth herein above.

In another preferred embodiment, the enzyme is a butyrate kinase (EC 2.7.2.7) from Geobacillus sp. GHH01 (Uniprot Accession number L8A0E1). In a particularly preferred embodiment, the butyrate kinase (EC 2.7.2.7) employed in the method of the invention has an amino acid sequence as shown in SEQ ID NO: 11 or shows an amino acid sequence which is at least x% homologous to SEQ ID NO: 11 and has the activity of a butyrate kinase with x being an integer between 30 and 100, preferably 35, 40, 45, 50, 55, 60, 65, 70, 75, 80, 85, 90, 91, 92, 93, 94, 95, 96, 97, 98 or 99 wherein such an enzyme is capable of converting 3-methylcrotonyl-phosphate into 3-methylcrotonic acid as set forth herein above.

Branched-chain-fatty-acid kinases (EC 2.7.2.14) naturally catalyze the following reaction

2-methylpropanoate + ATP ⇄ 2-methylpropanoyl phosphate + ADP

This enzyme has been described to occur in a number of bacteria. Thus, in one preferred embodiment the enzyme is an enzyme from a bacterium, preferably of the genus Spirochaeta or Thermotoga, more preferably Thermotoga maritime.

Propionate kinases (EC 2.7.2.15) naturally catalyze the following reactions

Propanoate + ATP ⇄ propanoyl phosphate + ADP

Acetate + ATP ⇄ acetyl phosphate + ADP

This enzyme has been described to occur in a number of bacteria, in particular Enterobacteriacea. Thus, in one preferred embodiment the enzyme is an enzyme from a bacterium, preferably of the genus Salmonella or Escherichia, more preferably of the species Salmonella enterica or Escherichia coli.

Acetate kinases (EC 2.7.2.1) naturally catalyze the following reaction

Acetate + ATP ⇄ acetyl phosphate + ADP

This enzyme has been described to occur in a number of organisms, in particular bacteria and eukaryotes. In one preferred embodiment the enzyme is from a bacterium, preferably from a bacterium of the genus Methanosarcina, Cryptococcus, Ethanoligenens, Propionibacterium, Roseovarius, Streptococcus, Salmonella, Acholeplasma, Acinetobacter, Ajellomyces, Bacillus, Borrelia, Chaetomium, Clostridium, Coccidioides, Coprinopsis, Cryptococcus, Cupriavidus, Desulfovibrio, Enterococcus, Escherichia, Ethanoligenes, Geobacillus, Helicobacter, Lactobacillus, Lactococcus, Listeria, Mesoplasma, Moorella, Mycoplasma, Oceanobacillus, Propionibacterium, Rhodospeudomonas, Roseovarius, Salmonella, Staphylococcus, Thermotoga or Veillonella, more preferably from a bacterium of the species Methanosarcina thermophila, Cryptococcus neoformans, Ethanoligenens harbinense, Propionibacterium acidipropionici, Streptococcus pneumoniae, Streptococcus enterica, Streptococcus pyogenes, Acholeplasma laidlawii, Acinetobacter calcoaceticus, Ajellomyces capsulatus, Bacillus subtilis, Borrelia burgdorferi, Chaetomium globosum, Clostridium acetobutylicum, Clostridium thermocellum, Coccidioides immitis, Coprinopsis cinerea, Cryptococcus neoformans, Cupriavidus necator, Desulfovibrio vulgaris, Enterococcus faecalis, Escherichia coli, Ethanoligenes harbinense, Geobacillus stearothermophilus, Helicobacter pylori, Lactobacillus delbrueckii, Lactobacillus acidophilus, Lactobacillus sanfranciscensis, Lactococcus lactis, Listeria monocytogenes, Mesoplasma florum, Methanosarcina acetivorans, Methanosarcina mazei, Moorella thermoacetica, Mycoplasma pneumoniae, Oceanobacillus iheyensis, Propionibacterium freudenreichii, Propionibacterium acidipropionici, Rhodospeudomonas palustris, Salmonella enteric, Staphylococcus aureus, Thermotoga maritime or Veillonella parvula.

In another preferred embodiment the enzyme is an enzyme from a fungus, preferably from a fungus of the genus Aspergillus, Gibberella, Hypocrea, Magnaporthe, Phaeosphaeria, Phanerochaete, Phytophthora, Sclerotinia, Uncinocarpus, Ustilago or Neurospora even more preferably from a fungus of the species Aspergillus fumigates, Aspergillus nidulans, Gibberella zeae, Hypocrea jecorina, Magnaporthe grisea, Phaeosphaeria nodorum, Phanerochaete chrysosporium, Phytophthora ramorum, Phytophthora sojae, Sclerotinia sclerotiorum, Uncinocarpus reesii, Ustilago maydis or Neurospora crassa.

In a further preferred embodiment the enzyme is an enzyme from a plant or an algae, preferably from the genus Chlamydomonas, even more preferably from the species Chlamydomonas reinhardtii.

In another embodiment the enzyme is from an organism of the genus Entamoeba, more preferably of the species Entamoeba histolytica.

The above mentioned enzyme families suitable for the conversion of 3-methylbutyryl phosphate into 3-methylbutyric acid have been shown to be evolutionary related and contain common sequence signatures. Theses signatures are referenced and described in Prosite database:
http://prosite.expasy.org/cgi-bin/prosite/nicedoc.pl?PS01075
Gao et al. (FEMS Microbiol. Lett. 213 (2002), 59-65) already described genetically modified E. coli cells which have been transformed, inter alia, with the ptb gene and the buk gene from Clostridium acetobutylicum encoding a phosphate butyryltransferase (EC 2.3.1.19) and a butyrate kinase (EC 2.7.2.7), respectively. These E. coli cells have been shown to be able to D-(-)-3-hydroxybutyric acid (3HB).

As described above the conversion of 3-methylcrotonyl-CoA into 3-methylbutyric acid can also be achieved by first converting 3-methylcrotonyl-CoA into 3-methylcrotonic acid and then further converting 3-methylcrotonic acid into 3-methylbutyric acid, i.e. by a method wherein the enzymatic conversion of 3-methylcrotonyl-CoA into 3-methylbutyric acid according to step (a) is achieved by a method comprising the following steps:
(al) enzymatically converting 3-methylcrotonyl-CoA into 3-methylcrotonic acid; and
(aII) further enzymatically converting the thus produced 3-methylcrotonic acid into 3-methylbutyric acid.

The conversion of 3-methylcrotonyl-CoA into 3-methylcrotonic acid according to step (al) can be achieved by different enzymatic conversions. One possibility is the direct conversion via a hydrolysis reaction. Another possibility is the direct conversion via a reaction catalyzed by a CoA-transferase and a third possibility is a two-step conversion via 3-methylcrotonyl phosphate. These three options will be further discussed in the following.

In one embodiment of the invention, as defined in the claims, the conversion of 3-methylcrotonyl-CoA into 3-methylcrotonic acid according to step (al) can be achieved by a direct conversion, i.e. by a method comprising the step of enzymatically converting 3-methylcrotonyl-CoA into 3-methylcrotonic acid in a hydrolysis reaction by making use of a thioesterase (EC 3.1.2). The conversion of 3-methylcrotonyl-CoA into 3-methylcrotonic acid catalyzed by a thioesterase is schematically shown in Figure 7.

Thioesterases have been described herein above and the same as has been set forth above also applies in connection with this embodiment of a method according to the invention. In this context it may be noteworthy that the acyl-CoA hydrolases (EC 3.1.2.20) from E. coli, Haemophilus influenza and Homo sapiens have been reported to be able to accept 3-methylcrotonyl-CoA (aka beta-methylcrotonyl-CoA) as a substrate.

Particularly preferred are the enzymes acyl-CoA thioesterase 2 from E. coli (Uniprot P0AGG2; SEQ ID NO: 12) and acyl-CoA thioesterase II from Pseudomonas putida (Uniprot Q88DR1; SEQ ID NO: 13).

In another embodiment the conversion of 3-methylcrotonyl-CoA into 3-methylcrotonic acid according to step (al) can be achieved by a direct conversion, i.e. by a method comprising the step of enzymatically converting 3-methylcrotonyl-CoA into 3-methylcrotonic acid in a transferase reaction by making use of a CoA-transferase (EC 2.8.3).

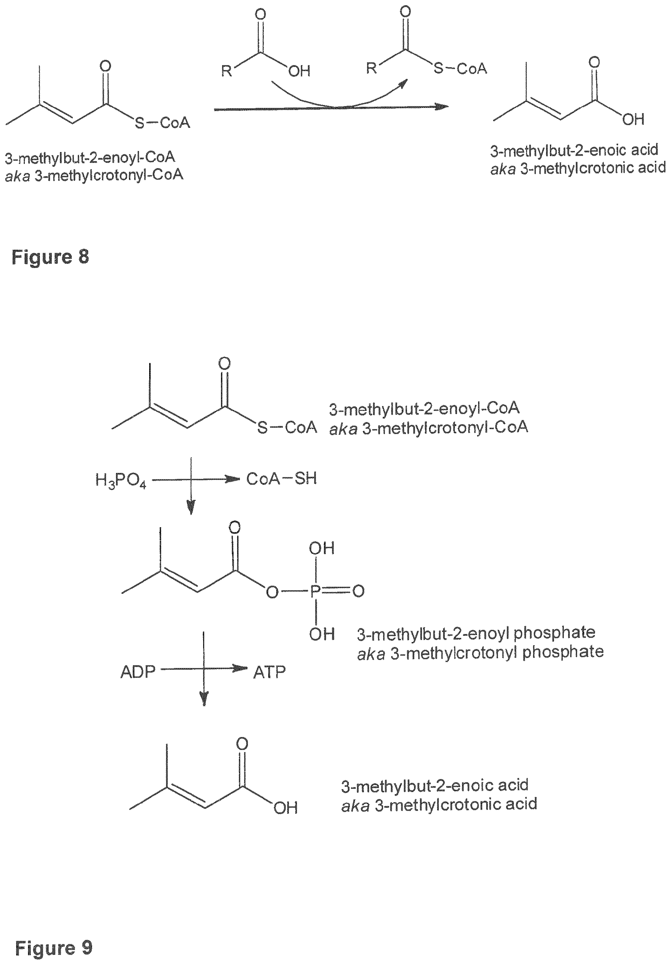

The conversion of 3-methylcrotonyl-CoA into 3-methylcrotonic acid catalyzed by a CoA-transferase is schematically shown in Figure 8.

CoA-transferases have been described herein above and the same as has been set forth above also applies in connection with this embodiment of a method according to the invention.

As mentioned above, the conversion of 3-methylcrotonyl-CoA into 3-methylcrotonic acid can in the alternative also be achieved by a conversion which first encompasses the conversion of 3-methylcrotonyl-CoA into 3-methylcrotonyl phosphate and the subsequent conversion of 3-methylcrotonyl phosphate into 3-methylcrotonic acid. The corresponding reaction is schematically shown in Figure 9.

The conversion of 3-methylcrotonyl-CoA into 3-methylcrotonyl phosphate can, e.g., be achieved by the use of a phosphate butyryltransferase (EC 2.3.1.19) or a phosphate acetyltransferase (EC 2.3.1.8). These enzymes have been described above in connection with the conversion of 3-methylbutyryl-CoA into 3-methylbutyryl phosphate and the same which has been set forth above also applies in connection with the present embodiment.

The conversion of 3-methylcrotonyl phosphate into 3-methylcrotonic acid can, e.g., be achieved by the use of enzymes which are classified in EC 2.7.2, i.e. enzymes which are classified as phosphotransferases with a carboxy group as acceptor. In a preferred embodiment the conversion of 3-methylcrotonyl phosphate into 3-methylcrotonic acid can, e.g., be achieved by the use of a butyrate kinase (EC 2.7.2.7), a branched-chain-fatty-acid kinase (EC 2.7.2.14), a propionate kinase (EC 2.7.2.15) or an acetate kinase (EC 2.7.2.1). These enzymes have been described above in connection with the conversion of 3-methylbutyryl phosphate into 3-methylbutyric acid and the same which has been set forth above also applies in connection with the present embodiment.

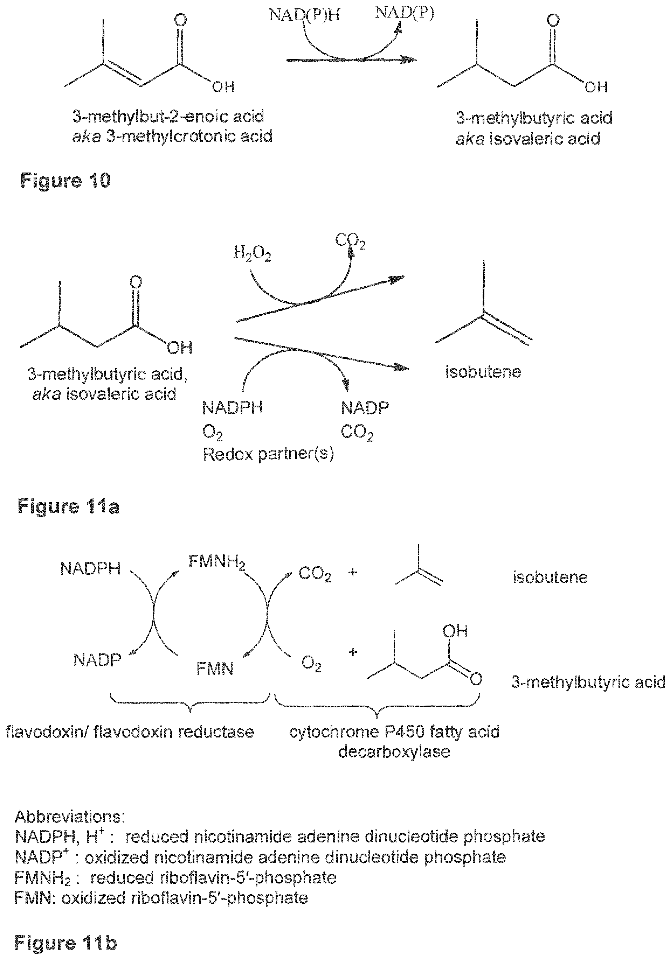

The 3-methylcrotonic acid which is obtained in step (al) of the above described method is enzymatically further converted into 3-methylbutyric acid according to step (aII) of the above described method. This conversion can, e.g., be achieved by making use of an enzyme which is classified as a 2-enoate reductase (EC 1.3.1.31). The reaction is schematically shown in Figure 10. 2-enoate reductases are enzymes which naturally catalyze the following reaction:

Butanoate + NAD⁺ ⇄ but-2-enoate + NADH + H⁺

This enzyme occurs in a variety of organism, including eukaryotic and prokaryotic organisms, such as animals, fungi and bacteria. The enzyme has, e.g., been described in Cichorium intybus, Marchantia polymorpha, Solanum lycopersicum, Absidia glauca, Kluyveromyces lactis, Penicillium citrinum; Rhodosporidium, Saccharomyces cerevisiae, Clostridium kluyveri, Clostridium bifermentans, Clostridium botulinum, Clostridium difficile, Clostridium ghonii, Clostridium mangenotii, Clostridium oceanicum, Clostridium sordellii, Clostridium sporogenes, Clostridium sticklandii, Clostridium tyrobutyricum, Achromobacter sp., Burkholderia sp., Gluconobacter oxydans, Lactobacillus casei, Pseudomonas putida, Shewanella sp., Yersinia bercovieri, Bacillus subtilis, Moorella thermoacetica and Peptostreptococcus anaerobius. The enoate reductase of Clostridiae has been described, e.g., in Tischler et al. (Eur. J. Bioche. 97 (1979), 103-112).

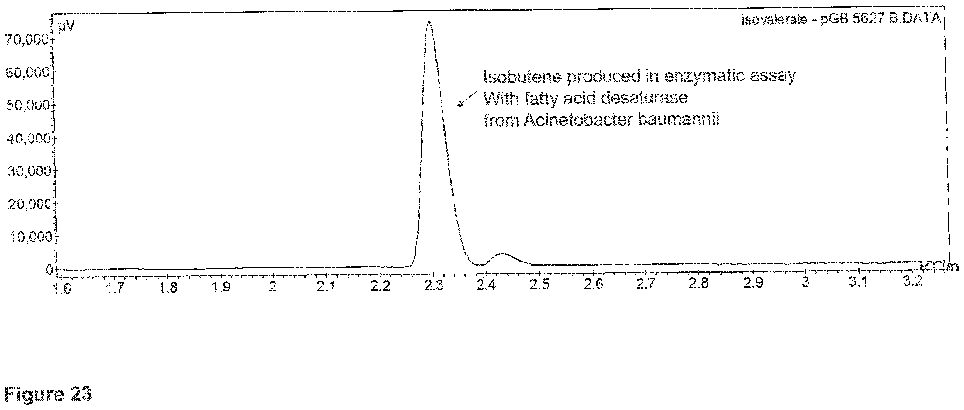

The 3-methylbutyric acid produced according to any of the described methods is then converted into isobutene by a UndB fatty acid desaturase (trans-membrane non-heme iron oxygenase) as e.g. shown in Figure 23.

As regards the determination of sequence identity, the following should apply: When the sequences which are compared do not have the same length, the degree of identity either refers to the percentage of amino acid residues in the shorter sequence which are identical to amino acid residues in the longer sequence or to the percentage of amino acid residues in the longer sequence which are identical to amino acid residues in the shorter sequence. Preferably, it refers to the percentage of amino acid residues in the shorter sequence which are identical to amino acid residues in the longer sequence. The degree of sequence identity can be determined according to methods well known in the art using preferably suitable computer algorithms such as CLUSTAL.

When using the Clustal analysis method to determine whether a particular sequence is, for instance, at least 60% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.

For instance, ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix; BLOSUM 62; gap open: 10; gap extension: 0.2; gap distance: 5; no end gap.

Preferably, the degree of identity is calculated over the complete length of the sequence.

Amino acid residues located at a position corresponding to a position as indicated herein-below in the amino acid sequence shown in SEQ ID NO:1 can be identified by the skilled person by methods known in the art. For example, such amino acid residues can be identified by aligning the sequence in question with the sequence shown in SEQ ID NO:1 and by identifying the positions which correspond to the above indicated positions of SEQ ID NO:1. The alignment can be done with means and methods known to the skilled person, e.g. by using a known computer algorithm such as the Lipman-Pearson method (Science 227 (1985), 1435) or the CLUSTAL algorithm. It is preferred that in such an alignment maximum homology is assigned to conserved amino acid residues present in the amino acid sequences.

In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.

As mentioned above in (b), the enzyme, as defined in the claims, shows the activity of converting 3-methylbutyric acid via oxidative decarboxylation into isobutene. This activity can be assayed as described in the appended Examples.

According to the present invention, the enzymatic conversion of 3-methylbutyric acid into isobutene is achieved by making use an enzyme referred to as a UndB fatty acid desaturase (membrane-bound non-heme iron oxygenase). A part from UndA enzymes, it has been found that another type of enzyme is expressed, e.g., in Pseudomonas which is involved in the synthesis of 1-alkenes, i.e. an enzyme referred to as "UndB" (see Rui et al., ACS Catal. 5 (2015), 7091-7094). Such enzymes or homologues thereof could be found so far in Pseudomonas species, such as P. fluorescens, P. mendocina and P. brassicacearum and in some related species, e.g., in Acinetobacter species, such as Acinetobacter baumannii or Acinetobacter oleivorans, or in Burkholderia species, such as Burkholderia cepacia, Burkholderia cenocepacia, Burkholderia pseudomallei and Burkholderia thailandensis. Moreover, proteins with related amino acid sequences could be found in the following organisms: Norcardia brasiliensis, Nocardia ATCC 700358, Glaciecola psychrophila, Leptospira borgpetersenii sv. Hardjo-bovis and Turneriella parva H. DSM.

The UndB fatty acid desaturase is a fatty acid desaturase which belongs to the UndB family of membrane bound desaturase-like enzymes. Preferably an UndB protein contains transmembrane domains and three conserved His-boxes. More moreferably these three His-boxes H₈₉DLIH₉₃, H₁₂₈(L/F)(N/H)H₁₃₁H₁₃₂, and H₂₉₇(G/S/A)IH₃₀₀H₃₀₁. The three His-boxes presumably include the ligands for a di-iron cluster at the catalytic site of the enzymes. UndB proteins are assumed to catalyze the oxidative decarboxylation of fatty acids through a mechanism similar to the fatty acid desaturation. Accordingly, this type of enzyme could also be referred to as "trans-membrane non-heme iron oxygenase".

In a preferred embodiment, the conversion of 3-methylbutyric acid into isobutene is achieved by making use of a UndB fatty acid desaturase from Pseudomonas sp., preferably from Pseudomonas mendocina, more preferably Pseudomonas mendocina strain ymp. Particularly preferred is the use of an enzyme comprising the amino acid sequence as shown in Uniprot accession number A4YOK1 or the amino acid sequence as shown in SEQ ID NO: 14.

In another preferred embodiment, the conversion of 3-methylbutyric acid into isobutene is achieved by making use of a UndB fatty acid desaturase from Acinetobacter, preferably Acinetobacter baumannii. Particularly preferred is the use of an enzyme comprising the amino acid sequence as shown in Uniprot accession number A0A0D5YLX9 or the amino acid sequence as shown in SEQ ID NO: 15.

It is, of course, not only possible to use an enzyme exactly showing an amino acid sequence of SEQ ID NOs: 14 or 15. It is also possible to use an enzyme which comprises a sequence which is at least 60% identical to any one of the amino acid sequences shown in SEQ ID NO: 14 or 15. Preferably, the sequence identity is at least 70%, more preferably at least 80%, 85% or 90%, even more preferably 91%, 92%, 93,%, 94%, 95%, 96%, 97%, 98% and particularly preferred at least 99% to an amino acid sequence as shown in SEQ ID NO: 14 or 15 and the enzyme has the enzymatic activity of converting 3-methylbutyric acid into isobutene. As regards the determination of the sequence identity, the same applies as has been set forth above.

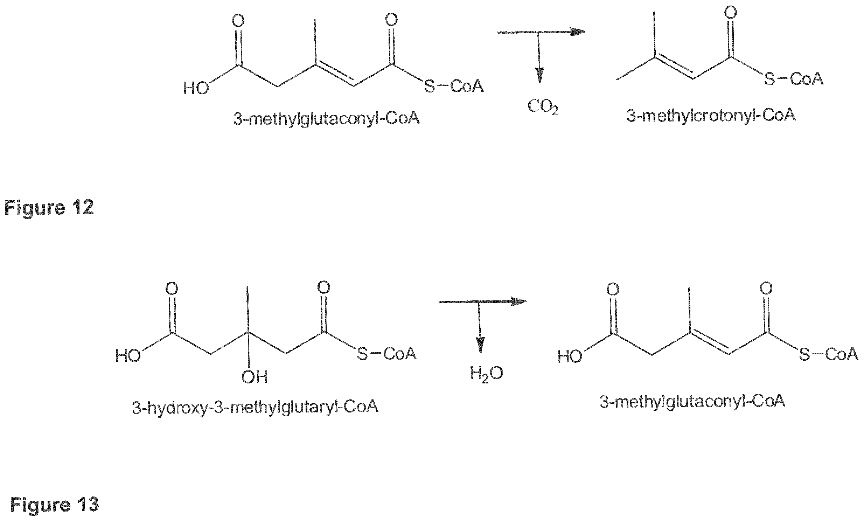

The 3-methylcrotonyl-CoA which is converted into 3-methylbutyric acid according to any of the above described methods may itself be provided by an enzymatic reaction, e.g. by the conversion of 3-methylglutaconyl-CoA via decarboxylation into 3-methylcrotonyl-CoA. This reaction is schematically shown in Figure 12. It may be catalyzed by different enzymes. In one preferred embodiment of the method as defined in the claims, the conversion of 3-methylglutaconyl-CoA via decarboxylation into 3-methylcrotonyl-CoA is catalyzed by a methylcrotonyl-CoA carboxylase (EC 6.4.1.4). Methylcrotonyl-CoA carboxylases have been described to catalyze the following reaction:

ATP + 3-methylcrotonyl-CoA + HCO₃⁻ + H⁺ ⇄ ADP + phosphate + 3-methylglutaconyl-CoA

i.e. the carboxylation, but they can be used to catalyze the reaction of decarboxylation. Methylcrotonyl-CoA carboxylases occur in a variety of organism, including eukaryotic and prokaryotic organisms, such as plants, animals, fungi and bacteria. The enzyme has, e.g., been described in Daucus carota, Glycine max, Hordeum vulgare, Pisum sativum, Solanum lycopersicum, Solanum tuberosum, Zea mays, Arabidopsis sp., Lens culinaris, Homo sapiens, Bos taurus, Rattus norvegicus, Mus musculus, Pagrus major, Emericella nidulans, Pseudomonas aeruginosa, Pseudomonas citronellolis, Acidaminococcus fermentans, Escherichia coli, Mycobacterium sp. and Achromobacter sp.

In another preferred embodiment the conversion of 3-methylglutaconyl-CoA via decarboxylation into 3-methylcrotonyl-CoA is catalyzed by a geranoyl-CoA carboxylase (EC 6.4.1.5). Geranoyl-CoA carboxylases naturally catalyze the following reaction:

ATP + geranoyl-CoA + HCO₃⁻ + H⁺ ⇄ ADP + phosphate + 3-(4-methylpent-3-en-1-yl) pent-2-enedioyl-CoA

The enzymes occurs in eukaryotes and prokaryotes, such as plants and bacteria. The enzyme has, e.g., been described in Daucus carota, Glycine max, Zea mays, Pseudomonas sp., Pseudomonas aeruginosa, Pseudomonas citronellolis and Pseudomonas mendocina.

In another preferred embodiment the conversion of 3-methylglutaconyl-CoA via decarboxylation into 3-methylcrotonyl-CoA is catalyzed by a 3-methylglutaconyl-CoA decarboxylase, e.g. a 3-methylglutaconyl-CoA decarboxylase of Myxococcus xanthus encoded by the liuB gene. This gene codes for an enzyme having the two subunits AibA and AibB (Li et al., Angew. Chem. Int. Ed. 52 (2013), 1304-1308).

Thus, the present invention also relates to a method for producing isobutene from 3-methylglutaconyl-CoA in which 3-methylglutaconyl-CoA is first converted by a decarboxylation reaction into 3-methylcrotonyl-CoA which is then further enzymatically converted into 3-methylbutyric acid as described herein above which is then further converted into isobutene by an UndB fatty acid desaturase, as described above.

The 3-methylglutaconyl-CoA to be converted into 3-methylcrotonyl-CoA can itself be provided by an enzymatic reaction which occurs naturally, which involves the conversion of 3-hydroxy-3-methylglutaryl-CoA into 3-methylglutaconyl-CoA and which is catalyzed, e.g., by enzymes classified as 3-methylglutaconyl-coenzyme A hydratase (EC 4.2.1.18). This reaction is schematically shown in Figure 13. 3-methylglutaconyl-coenzyme A hydratase are enzymes which catalyze the following reaction:

(S)-3-hydroxy-3-methylglutaryl-CoA ⇄ trans-3-methylglutaconyl-CoA + H₂O

This enzyme occurs in a variety of organism, including eukaryotic and prokaryotic organisms, such as plants, animals and bacteria. The enzyme has, e.g., been described in Catharantus roseus, Homo sapiens, Bos taurus, Ovis aries, Acinetobacter sp. and Pseudomonas putida.

The conversion of 3-hydroxy-3-methylglutaryl-CoA into 3-methylglutaconyl-CoA can also be achieved by making use of a 3-hydroxy-3-methylglutaryl-coenzyme A dehydratase activity which has been identified, e.g., in Myxococcus xanthus and which is encoded by the liuC gene (Li et al., Angew. Chem. Int. Ed. 52 (2013), 1304-1308).

Thus, the present invention also relates to a method for producing isobutene from 3-hydroxy-3-methylglutaryl-CoA in which 3-hydroxy-3-methylglutaryl-CoA is first converted into 3-methylglutaconyl-CoA which is then converted by a decarboxylation reaction into 3-methylcrotonyl-CoA, which is then further enzymatically converted into 3-methylbutyric acid and isobutene as described herein above.

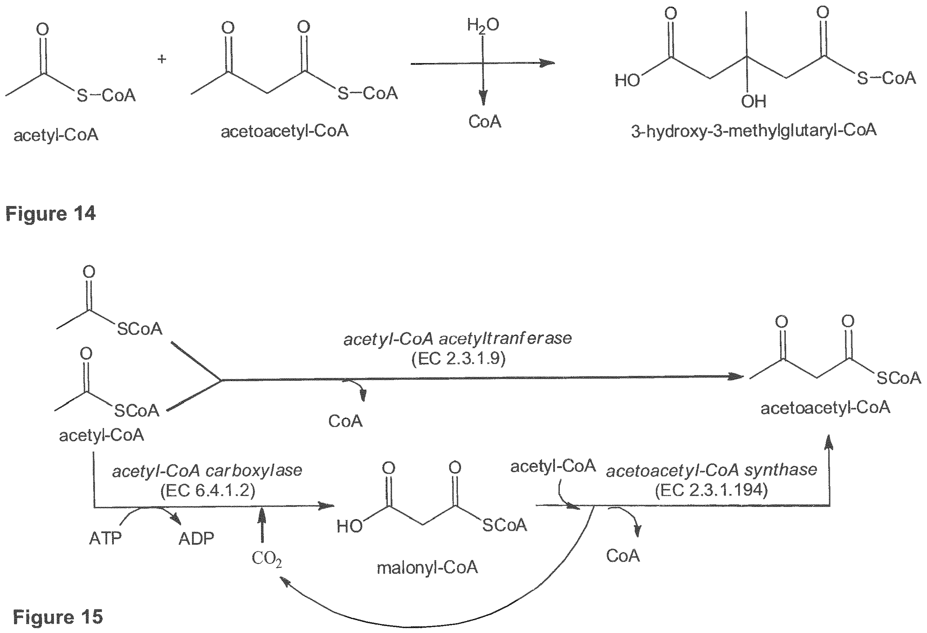

The 3-hydroxy-3-methylglutaryl-CoA which is converted into 3-methylglutaconyl-CoA can itself be provided enzymatically, e.g. by the condensation of acetyl-CoA and acetoacetyl-CoA, a reaction which is naturally catalzyed by the enzyme 3-hydroxy-3-methylglutaryl-CoA synthase (also referred to as HMG-CoA synthase). HMG-CoA synthases are classified in EC 2.3.3.10 (formerly, HMG-CoA synthase has been classified as EC 4.1.3.5 but has been transferred to EC 2.3.3.10). The term "HMG-CoA synthase" refers to any enzyme which is able to catalyze the reaction where acetyl-CoA condenses with acetoacetyl-CoA to form 3-hydroxy-3-methylglutaryl-CoA (HMG-CoA) (see Figure 14). HMG-CoA synthase is part of the mevalonate pathway. Two pathways have been identified for the synthesis of isopentenyl pyrophosphate (IPP), i.e. the mevalonate pathway and the glyceraldehyde 3-phosphate-pyruvate pathway. HMG-CoA synthase catalyzes the biological Claisen condensation of acetyl-CoA with acetoacetyl-CoA and is a member of a superfamily of acyl-condensing enzymes that includes beta-ketothiolases, fatty acid synthases (beta-ketoacyl carrier protein synthase) and polyketide synthases.

HMG-CoA synthase has been described for various organisms. Also amino acid and nucleic acid sequences encoding HMG-CoA synthases from numerous sources are available. Generally, the sequences only share a low degree of overall sequence identity. For example, the enzymes from Staphylococcus or Streptococcus show only about 20% identity to those of human and avian HMG-CoA synthase. In some sources it is reported that the bacterial HMG-CoA synthases and their animal counterparts exhibit only about 10% overall sequence identity (Sutherlin et al., J. Bacteriol. 184 (2002), 4065-4070). However, the amino acid residues involved in the acetylation and condensation reactions are conserved among bacterial and eukaryotic HMG-CoA synthases (Campobasso et al., J. Biol. Chem. 279 (2004), 44883-44888). The three-dimensional structure of three HMG-CoA synthase enzymes has been determined and the amino acids crucial for the enzymatic reaction are in principle well characterized (Campobasso et al., loc. cit.; Chun et al., J. Biol.Chem. 275 (2000), 17946-17953; Nagegowda et al., Biochem. J. 383 (2004), 517-527; Hegardt, Biochem. J. 338 (1999), 569-582). In eukaryotes there exist two forms of the HMG-CoA synthase, i.e. a cytosolic and a mitochondrial form. The cytosolic form plays a key role in the production of cholesterol and other isoprenoids and the mitochondrial form is involved in the production of ketone bodies.

In principle any HMG-CoA synthase enzyme can be used in the context of the present invention, in particular from prokaryotic or eukaryotic organisms.

Prokaryotic HMG-CoA synthases are described, e.g., from Staphylococcus aureus (Campobasso et al., loc. cit.; Uniprot accession number Q9FD87), Staphylococcus epidermidis (Uniprot accession number Q9FD76), Staphylococcus haemolyticus (Uniprot accession number Q9FD82), Enterococcus faecalis (Sutherlin et al., loc. cit.; Unirprot accession number Q9FD7), Enterococcus faecium (Uniprot accession number Q9FD66), Streptococcus pneumonia (Uniprot accession number Q9FD56), Streptococcus pyogenes (Uniprot accession number Q9FD61) and Methanobacterium thermoautotrophicum (accession number AE000857), Borrelia burgdorferi (NCBI accession number BB0683). Further HMG-CoA synthases are, e.g., described in WO 2011/032934.

Thus, the present invention also relates to a method for producing isobutene from acetyi-CoA and acetoacetyl-CoA in which acetyl-CoA and acetoacetyl-CoA are first condensed so as to form 3-hydroxy-3-methylglutaryl-CoA, in which 3-hydroxy-3-methylglutaryl-CoA is then converted into 3-methylglutaconyl-CoA, which is then converted by a decarboxylation reaction into 3-methylcrotonyl-CoA, which is then further enzymatically converted into 3-methylbutyric acid, which is then further converted into isobutene as described herein above.

The acetoacetyl-CoA which is used in the production of 3-hydroxy-3-methylglutaryl-CoA can itself be provided by enzymatic reactions. For example, acetoacetyl-CoA can be produced from acetyl-CoA as, e.g., described in WO 2013/057194. Thus, according to the present invention, as defined in the claims, acetyl-CoA can, for example, be converted into acetoacetyl-CoA by the following reaction:

2 acetyl-CoA ⇄ acetoacetyl-CoA + CoA

This reaction is catalyzed by enzymes called acetyl-CoA C-acetyltransferases which are classified as EC 2.3.1.9. Enzymes belonging to this class and catalyzing the above shown conversion of two molecules of acetyl-CoA into acetoacetyl-CoA and CoA occur in organisms of all kingdoms, i.e. plants, animals, fungi, bacteria etc. and have extensively been described in the literature. Nucleotide and/or amino acid sequences for such enzymes have been determined for a variety of organisms, like Homo sapiens, Arabidopsis thaliana, E. coli, Bacillus subtilis and Candida, to name just some examples. In principle, any acetyl-CoA C-acetyltransferase (EC 2.3.1.9) can be used in the context of the present invention.

Alternatively, the provision of acetoacetyl-CoA may also be achieved by the enzymatic conversion of acetyl-CoA and malonyl-CoA into acetoacetyl-CoA according to the following reaction.

acetyl-CoA + malonyl-CoA → acetoacetyl-CoA + CoA +CO₂

This reaction is catalyzed by an enzyme called acetoacetyl-CoA synthase (EC 2.3.1.194). The gene encoding this enzyme was identified in the mevalonate pathway gene cluster for terpenoid production in a soil-isolated Gram-positive Streptomyces sp. Strain CL190 (Okamura et al., PNAS USA 107 (2010), 11265-11270, 2010).

Moreover a biosynthetic pathway using this enzyme for acetoacetyl-CoA production was recently developed in E. coli (Matsumoto K et al., Biosci. Biotechnol. Biochem, 75 (2011), 364-366).

Accordingly, in a preferred embodiment, the enzymatic conversion of acetyl-CoA into said acetoacetyl-CoA consists of a single enzymatic reaction in which acetyl-CoA is directly converted into acetoacetyl-CoA. Preferably, the enzymatic conversion of acetyl-CoA into acetoacetyl-CoA is achieved by making use of an acetyl-CoA acetyltransferase (EC 2.3.1.9) as described above.

Alternatively, the acetoacetyl-CoA can also be provided by an enzymatic conversion which comprises two steps, i.e.;
(i) the enzymatic conversion of acetyl-CoA into malonyl-CoA; and
(ii) the enzymatic conversion of malonyl-CoA and acetyl-CoA into acetoacetyl-CoA.

Preferably, the enzymatic conversion of acetyl-CoA into malonyl-CoA is achieved by the use of an acetyl-CoA carboxylase (EC 6.4.1.2). This enzyme catalyzes the following reaction:

Acetyl-CoA + ATP + CO₂ → Malonyl-CoA +ADP

Preferably, the enzymatic conversion of malonyl-CoA and acetyl-CoA into acetoacetyl-CoA is achieved by the use of an acetoacetyl-CoA synthase (EC 2.3.1.194). In principle, any acetyl-CoA acetyltransferase (EC 2.3.1.9), acetyl-CoA carboxylase (EC 6.4.1.2) and/or acetoacetyl-CoA synthase (EC 2.3.1.194) can be applied in the method according to the invention.

Figure 15 shows schematically possible ways of producing acetoacetyl-CoA from acetyl-CoA.

Thus, the present invention also relates to a method for producing isobutene from acetyl-CoA, in which acetoacetyl-CoA is produced from acetyl-CoA as described above, in which the thus produced acetoacetyl-CoA is then condensed with acetyl-CoA so as to form 3-hydroxy-3-methylglutaryl-CoA, in which 3-hydroxy-3-methylglutaryl-CoA is then converted into 3-methylglutaconyl-CoA, which is then converted by a decarboxylation reaction into 3-methylcrotonyl-CoA, which is then further enzymatically converted into 3-methylbutyric acid and isobutene as described herein above.

A method according to the present invention may be carried out in vitro or in vivo. An in vitro reaction is understood to be a reaction in which no cells are employed, i.e. an acellular reaction. Thus, in vitro preferably means in a cell-free system. The term "in vitro" in one embodiment means in the presence of isolated enzymes (or enzyme systems optionally comprising possibly required cofactors). In one embodiment, the enzymes employed in the method are used in purified form.

For carrying out the method in vitro the substrates for the reaction and the enzymes are incubated under conditions (buffer, temperature, cosubstrates, cofactors etc.) allowing the enzymes to be active and the enzymatic conversion to occur. The reaction is allowed to proceed for a time sufficient to produce the respective product. The production of the respective products can be measured by methods known in the art, such as gas chromatography possibly linked to mass spectrometry detection. The enzymes may be in any suitable form allowing the enzymatic reaction to take place. They may be purified or partially purified or in the form of crude cellular extracts or partially purified extracts. It is also possible that the enzymes are immobilized on a suitable carrier.

In another embodiment the method according to the invention is carried out in culture, in the presence of an organism, preferably a microorganism, producing the enzymes described above for the conversions of the method according to the present invention as described herein above. A method which employs a microorganism for carrying out a method according to the invention is referred to as an "in vivo" method. It is possible to use a microorganism which naturally produces the enzymes described above for the conversions of the method according to the present invention or a microorganism which had been genetically modified so that it expresses (including overexpresses) one or more of such enzymes. Thus, the microorganism can be an engineered microorganism which expresses enzymes described above for the conversions of the method according to the present invention, i.e. which has in its genome a nucleotide sequence encoding such enzymes and which has been modified to overexpress them. The expression may occur constitutively or in an induced or regulated manner.

In another embodiment the microorganism can be a microorganism which has been genetically modified by the introduction of one or more nucleic acid molecules containing nucleotide sequences encoding one or more enzymes described above for the conversions of the methods according to the present invention. The nucleic acid molecule can be stably integrated into the genome of the microorganism or may be present in an extrachromosomal manner, e.g. on a plasmid.

Such a genetically modified microorganism can, e.g., be a microorganism that does not naturally express enzymes described above for the conversions of the method according to the present invention and which has been genetically modified to express such enzymes or a microorganism which naturally expresses such enzymes and which has been genetically modified, e.g. transformed with a nucleic acid, e.g. a vector, encoding the respective enzyme(s), and/or insertion of a promoter in front of the endogenous nucleotide sequence encoding the enzyme in order to increase the respective activity in said microorganism.

However, the invention preferably excludes naturally occurring microorganisms as found in nature expressing an enzyme as described above at levels as they exist in nature. Instead, the microorganism of the present invention and employed in a method of the present invention is preferably a non-naturally occurring microorganism, whether it has been genetically modified to express (including overexpression) an exogenous enzyme of the invention not normally existing in its genome or whether it has been engineered to overexpress an exogenous enzyme. Thus, the enzymes and (micro)organisms employed in connection with the present invention are preferably non-naturally occurring enzymes or (microorganisms), i.e. they are enzymes or (micro)organisms which differ significantly from naturally occurring enzymes or microorganism and which do not occur in nature. As regards the enzymes, they are preferably variants of naturally occurring enzymes which do not as such occur in nature. Such variants include, for example, mutants, in particular prepared by molecular biological methods, which show improved properties, such as a higher enzyme activity, higher substrate specificity, higher temperature resistance and the like. As regards the (micro)organisms, they are preferably genetically modified organisms as described herein above which differ from naturally occurring organisms due to a genetic modification. Genetically modified organisms are organisms which do not naturally occur, i.e., which cannot be found in nature, and which differ substantially from naturally occurring organisms due to the introduction of a foreign nucleic acid molecule.

By overexpressing an exogenous or endogenous enzyme as described herein above, the concentration of the enzyme is substantially higher than what is found in nature, which can then unexpectedly force the reaction of the present invention which uses a non-natural for the respective enzyme. Preferably, the concentration of the overexpressed enzyme is at least 5%, 10%, 20%, 30% or 40% of the toatal host cell protein.

A "non-natural" substrate is understood to be a molecule that is not acted upon by the respective enzyme in nature, even though it may actually coexist in the microorganism along with the endogenous enzyme. This "non-natural" substrate is not converted by the microorganism in nature as other substrates are preferred (e.g. the "natural substrate"). Thus, the present invention contemplates utilizing a non-natural substrate with the enzymes described above in an environment not found in nature.

Thus, it is also possible in the context of the present invention that the microorganism is a microorganism which naturally does not have the respective enzyme activity but which is genetically modified so as to comprise a nucleotide sequence allowing the expression of a corresponding enzyme. Similarly, the microorganism may also be a microorganism which naturally has the respective enzyme activity but which is genetically modified so as to enhance such an activity, e.g. by the introduction of an exogenous nucleotide sequence encoding a corresponding enzyme or by the introduction of a promoter for the endogenous gene encoding the enzyme to increase endogenous production to overexpressed (non-natural) levels.

If a microorganism is used which naturally expresses a corresponding enzyme, it is possible to modify such a microorganism so that the respective activity is overexpressed in the mircroorganism. This can, e.g., be achieved by effecting mutations in the promoter region of the corresponding gene or introduction of a high expressing promoter so as to lead to a promoter which ensures a higher expression of the gene. Alternatively, it is also possible to mutate the gene as such so as to lead to an enzyme showing a higher activity.

By using microorganisms which express enzymes described above for the conversions of the methods according to the present invention, it is possible to carry out the methods according to the invention directly in the culture medium, without the need to separate or purify the enzymes.

In one embodiment the organism employed in a method according to the invention is a microorganism which has been genetically modified to contain a foreign nucleic acid molecule encoding at least one enzyme described above for the conversions of the methods according to the present invention. The term "foreign" or "exogenous" in this context means that the nucleic acid molecule does not naturally occur in said microorganism. This means that it does not occur in the same structure or at the same location in the microorganism. In one preferred embodiment, the foreign nucleic acid molecule is a recombinant molecule comprising a promoter and a coding sequence encoding the respective enzyme in which the promoter driving expression of the coding sequence is heterologous with respect to the coding sequence. "Heterologous" in this context means that the promoter is not the promoter naturally driving the expression of said coding sequence but is a promoter naturally driving expression of a different coding sequence, i.e., it is derived from another gene, or is a synthetic promoter or a chimeric promoter. Preferably, the promoter is a promoter heterologous to the microorganism, i.e. a promoter which does naturally not occur in the respective microorganism. Even more preferably, the promoter is an inducible promoter. Promoters for driving expression in different types of organisms, in particular in microorganisms, are well known to the person skilled in the art.

In a further embodiment the nucleic acid molecule is foreign to the microorganism in that the encoded enzyme is not endogenous to the microorganism, i.e. is naturally not expressed by the microorganism when it is not genetically modified. In other words, the encoded enzyme is heterologous with respect to the microorganism. The foreign nucleic acid molecule may be present in the microorganism in extrachromosomal form, e.g. as a plasmid, or stably integrated in the chromosome. A stable integration is preferred. Thus, the genetic modification can consist, e.g. in integrating the corresponding gene(s) encoding the enzyme(s) into the chromosome, or in expressing the enzyme(s) from a plasmid containing a promoter upstream of the enzyme-coding sequence, the promoter and coding sequence preferably originating from different organisms, or any other method known to one of skill in the art.

The term "microorganism" in the context of the present invention refers to bacteria, as well as to fungi, such as yeasts, and also to algae and archaea. In one preferred embodiment, the microorganism is a bacterium. In principle any bacterium can be used. Preferred bacteria to be employed in the process according to the invention are bacteria of the genus Bacillus, Clostridium, Corynebacterium, Pseudomonas, Zymomonas or Escherichia. In a particularly preferred embodiment the bacterium belongs to the genus Escherichia and even more preferred to the species Escherichia coli. In another preferred embodiment the bacterium belongs to the species Pseudomonas putida or to the species Zymomonas mobilis or to the species Corynebacterium glutamicum or to the species Bacillus subtilis.

It is also possible to employ an extremophilic bacterium such as Thermus thermophilus, or anaerobic bacteria from the family Clostridiae.

In another preferred embodiment the microorganism is a fungus, more preferably a fungus of the genus Saccharomyces, Schizosaccharomyces, Aspergillus, Trichoderma, Kluyveromyces or Pichia and even more preferably of the species Saccharomyces cerevisiae, Schizosaccharomyces pombe, Aspergillus niger, Trichoderma reesei, Kluyveromyces marxianus, Kluyveromyces lactis, Pichia pastoris, Pichia torula or Pichia utilis.

In another embodiment, the method according to the invention makes use of a photosynthetic microorganism expressing at least one enzyme for the conversion according to the invention as described above. Preferably, the microorganism is a photosynthetic bacterium, or a microalgae. In a further embodiment the microorganism is an algae, more preferably an algae belonging to the diatomeae.

It is also conceivable to use in the method according to the invention a combination of microorganisms wherein different microorganisms express different enzymes as described above. The genetic modification of microorganisms to express an enzyme of interest will also be further described in detail below.

In another embodiment, the method of the invention comprises the step of providing the organism, preferably the microorganism carrying the respective enzyme activity or activities in the form of a (cell) culture, preferably in the form of a liquid cell culture, a subsequent step of cultivating the organism, preferably the microorganism in a fermenter (often also referred to a bioreactor) under suitable conditions allowing the expression of the respective enzyme and further comprising the step of effecting an enzymatic conversion of a method of the invention as described herein above. Suitable fermenter or bioreactor devices and fermentation conditions are known to the person skilled in the art. A bioreactor or a fermenter refers to any manufactured or engineered device or system known in the art that supports a biologically active environment. Thus, a bioreactor or a fermenter may be a vessel in which a chemical/biochemical like the method of the present invention is carried out which involves organisms, preferably microorganisms and/or biochemically active substances, i.e., the enzyme(s) described above derived from such organisms or organisms harboring the above described enzyme(s). In a bioreactor or a fermenter, this process can either be aerobic or anaerobic. These bioreactors are commonly cylindrical, and may range in size from litres to cubic metres, and are often made of stainless steel. In this respect, without being bound by theory, the fermenter or bioreactor may be designed in a way that it is suitable to cultivate the organisms, preferably microorganisms, in, e.g., a batch-culture, feed-batch-culture, perfusion culture or chemostate-culture, all of which are generally known in the art.

The culture medium can be any culture medium suitable for cultivating the respective organism or microorganism.

In a preferred embodiment the method according to the present invention also comprises the step of recovering the isobutene produced by the method. For example, if the method according to the present invention is carried out in vivo by fermenting a corresponding microorganism expressing the necessary enzymes, the isobutene can be recovered from the fermentation off-gas by methods known to the person skilled in the art.

The enzymes used in the method according to the invention can be naturally occurring enzymes or enzymes which are derived from a naturally occurring enzymes, e.g. by the introduction of mutations or other alterations which, e.g., alter or improve the enzymatic activity, the stability, etc.

Methods for modifying and/or improving the desired enzymatic activities of proteins are well-known to the person skilled in the art and include, e.g., random mutagenesis or site-directed mutagenesis and subsequent selection of enzymes having the desired properties or approaches of the so-called "directed evolution".

For example, for genetic modification in prokaryotic cells, a nucleic acid molecule encoding a corresponding enzyme can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be ligated by using adapters and linkers complementary to the fragments. Moreover, engineering measures which provide suitable restriction sites or remove surplus DNA or restriction sites can be used. In those cases, in which insertions, deletions or substitutions are possible, *in vitro* mutagenesis, "primer repair", restriction or ligation can be used. In general, a sequence analysis, restriction analysis and other methods of biochemistry and molecular biology are carried out as analysis methods. The resulting enzyme variants are then tested for the desired activity, e.g., enzymatic activity, with an assay as described above and in particular for their increased enzyme activity.

As described above, the microorganism employed in a method of the invention may be a microorganism which has been genetically modified by the introduction of a nucleic acid molecule encoding a corresponding enzyme. Thus, in a preferred embodiment, the microorganism is a recombinant microorganism which has been genetically modified to have an increased activity of at least one enzyme described above for the conversions of the method according to the present invention. This can be achieved e.g. by transforming the microorganism with a nucleic acid encoding a corresponding enzyme. A detailed description of genetic modification of microorganisms will be given further below. Preferably, the nucleic acid molecule introduced into the microorganism is a nucleic acid molecule which is heterologous with respect to the microorganism, i.e. it does not naturally occur in said microorganism.

In the context of the present invention, an "increased activity" means that the expression and/or the activity of an enzyme in the genetically modified microorganism is at least 10%, preferably at least 20%, more preferably at least 30% or 50%, even more preferably at least 70% or 80% and particularly preferred at least 90% or 100% higher than in the corresponding non-modified microorganism. In even more preferred embodiments the increase in expression and/or activity may be at least 150%, at least 200% or at least 500%. In particularly preferred embodiments the expression is at least 10-fold, more preferably at least 100-fold and even more preferred at least 1000-fold higher than in the corresponding non-modified microorganism.

The term "increased" expression/activity also covers the situation in which the corresponding non-modified microorganism does not express a corresponding enzyme so that the corresponding expression/activity in the non-modified microorganism is zero. Preferably, the concentration of the overexpressed enzyme is at least 5%, 10%, 20%, 30%, or 40% of the total host cell protein.

Methods for measuring the level of expression of a given protein in a cell are well known to the person skilled in the art. In one embodiment, the measurement of the level of expression is done by measuring the amount of the corresponding protein. Corresponding methods are well known to the person skilled in the art and include Western Blot, ELISA etc. In another embodiment the measurement of the level of expression is done by measuring the amount of the corresponding RNA. Corresponding methods are well known to the person skilled in the art and include, e.g., Northern Blot.

In the context of the present invention the term "recombinant" means that the microorganism is genetically modified so as to contain a nucleic acid molecule encoding an enzyme as defined above as compared to a wild-type or non-modified microorganism. A nucleic acid molecule encoding an enzyme as defined above can be used alone or as part of a vector.

The nucleic acid molecules can further comprise expression control sequences operably linked to the polynucleotide comprised in the nucleic acid molecule. The term "operatively linked" or "operably linked", as used throughout the present description, refers to a linkage between one or more expression control sequences and the coding region in the polynucleotide to be expressed in such a way that expression is achieved under conditions compatible with the expression control sequence.

Expression comprises transcription of the heterologous DNA sequence, preferably into a translatable mRNA. Regulatory elements ensuring expression in fungi as well as in bacteria, are well known to those skilled in the art. They encompass promoters, enhancers, termination signals, targeting signals and the like. Examples are given further below in connection with explanations concerning vectors.

Promoters for use in connection with the nucleic acid molecule may be homologous or heterologous with regard to its origin and/or with regard to the gene to be expressed. Suitable promoters are for instance promoters which lend themselves to constitutive expression. However, promoters which are only activated at a point in time determined by external influences can also be used. Artificial and/or chemically inducible promoters may be used in this context.

The vectors can further comprise expression control sequences operably linked to said polynucleotides contained in the vectors. These expression control sequences may be suited to ensure transcription and synthesis of a translatable RNA in bacteria or fungi.

In addition, it is possible to insert different mutations into the polynucleotides by methods usual in molecular biology (see for instance Sambrook and Russell (2001), Molecular Cloning: A Laboratory Manual, CSH Press, Cold Spring Harbor, NY, USA), leading to the synthesis of polypeptides possibly having modified biological properties. The introduction of point mutations is conceivable at positions at which a modification of the amino acid sequence for instance influences the biological activity or the regulation of the polypeptide.

Moreover, mutants possessing a modified substrate or product specificity can be prepared. Preferably, such mutants show an increased activity. Alternatively, mutants can be prepared the catalytic activity of which is abolished without losing substrate binding activity.

Furthermore, the introduction of mutations into the polynucleotides encoding an enzyme as defined above allows the gene expression rate and/or the activity of the enzymes encoded by said polynucleotides to be reduced or increased.

For genetically modifying bacteria or fungi, the polynucleotides encoding an enzyme as defined above or parts of these molecules can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, *in vitro* mutagenesis, "primer repair", restriction or ligation can be used. In general, a sequence analysis, restriction analysis and other methods of biochemistry and molecular biology are carried out as analysis methods.

Thus, a recombinant microorganism can be produced by genetically modifying fungi or bacteria comprising introducing the above-described polynucleotides, nucleic acid molecules or vectors into a fungus or bacterium.

The polynucleotide encoding the respective enzyme is expressed so as to lead to the production of a polypeptide having any of the activities described above. An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). An overview of yeast expression systems is for instance given by Hensing et al. (Antonie van Leuwenhoek 67 (1995), 261-279), Bussineau et al. (Developments in Biological Standardization 83 (1994), 13-19), Gellissen et al. (Antonie van Leuwenhoek 62 (1992), 79-93, Fleer (Current Opinion in Biotechnology 3 (1992), 486-496), Vedvick (Current Opinion in Biotechnology 2 (1991), 742-745) and Buckholz (Bio/Technology 9 (1991), 1067-1072).

Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E*. *coli, S. cerevisiae*) are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.

The transformation of the host cell with a polynucleotide or vector as described above can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc.

Further disclosed is the use of
- a phosphate butyryltransferase (EC 2.3.1.19) or an organism expressing a phosphate butyryltransferase (EC 2.3.1.19); or
- a phosphate acetyltransferase (EC 2.3.1.8) or an organism expressing a phosphate acetyltransferase (EC 2.3.1.8)
for the enzymatic conversion of 3-methylcrotonyl-CoA into 3-methylcrotonyl phosphate.

Further disclosed is the use of a phosphotransferase (EC 2.7.2), e.g. of a butyrate kinase (EC 2.7.2.7), a branched-chain-fatty-acid kinase (EC 2.7.2.14), a propionate kinase (EC 2.7.2.15) and/or an acetate kinase (EC 2.7.2.1), or of a microorganisms expressing such enzymes for the enzymatic conversion of 3-methylcrotonyl-CoA into 3-methylcrotonyl phosphate.

Further disclosed is the use of a combination comprising
- a phosphate butyryltransferase (EC 2.3.1.19) and/or a phosphate acetyltransferase (EC 2.3.1.8); and
- a phosphotransferase (EC 2.7.2), e.g. a a butyrate kinase (EC 2.7.2.7), a branched-chain-fatty-acid kinase (EC 2.7.2.14), a propionate kinase (EC 2.7.2.15) and/or an acetate kinase (EC 2.7.2.1),
or of a microorganism expressing such a combination of enzymes for the conversion of 3-methylcrotonyl-CoA into 3-methylcrotonic acid.

Further disclosed the use of a combination comprising
- a phosphate butyryltransferase (EC 2.3.1.19) and/or a phosphate acetyltransferase (EC 2.3.1.8); and
- a phosphotransferase (EC 2.7.2), e.g. a butyrate kinase (EC 2.7.2.7), a branched-chain-fatty-acid kinase (EC 2.7.2.14), a propionate kinase (EC 2.7.2.15) and/or an acetate kinase (EC 2.7.2.1); and
- a 2-enoate reductase (EC 1.3.1.31)
or of a microorganism expressing such a combination of enzymes for the conversion of 3-methylcrotonyl-CoA into 3-methylbutyric acid.

Further disclosed is the use of a combination comprising
- a phosphate butyryltransferase (EC 2.3.1.19) and/or a phosphate acetyltransferase (EC 2.3.1.8); and
- a phosphotransferase (EC 2.7.2), e.g. a butyrate kinase (EC 2.7.2.7), a branched-chain-fatty-acid kinase (EC 2.7.2.14), a propionate kinase (EC 2.7.2.15) and/or an acetate kinase (EC 2.7.2.1); and
- a 2-enoate reductase (EC 1.3.1.31); and
- a cytochrome P450 or a non-heme iron oxygenase or a fatty acid desaturase or of a microorganism expressing such a combination of enzymes for the conversion of 3-methylcrotonyl-CoA into isobutene.

Further disclosed is the use of a thioesterase (EC 3.1.2) and/or a CoA-transferase (EC 2.8.3) or of a microorganism expressing such an enzyme for the conversion of 3-methylcrotonyl-CoA into 3-methylcrotonic acid.

Further disclosed is the use of a combination comprising
- a thioesterase (EC 3.1.2) and/or a CoA-transferase (EC 2.8.3); and
- a 2-enoate reductase (EC 1.3.1.31)
or of a microorganism expressing such a combination of enzymes for the conversion of 3-methylcrotonyl-CoA into 3-methylbutyric acid.

Further disclosed is the use of a combination comprising
- a thioesterase (EC 3.1.2) and/or a CoA-transferase (EC 2.8.3); and
- a 2-enoate reductase (EC 1.3.1.31); and
- a cytochrome P450 or a non-heme iron oxygenase or a fatty acid desaturase or of a microorganism expressing such a combination of enzymes for the conversion of 3-methylcrotonyl-CoA into isobutene.

Further disclosed is the use of a combination comprising
(i) an enzyme classified as EC 1.3._._; and
(ii1) a phosphate butyryltransferase (EC 2.3.1.19) and/or a phosphate acetyltransferase (EC 2.3.1.8); and
   a phosphotransferase (EC 2.7.2), e.g. a butyrate kinase (EC 2.7.2.7), a branched-chain-fatty-acid kinase (EC 2.7.2.14), a propionate kinase (EC 2.7.2.15) and/or an acetate kinase (EC 2.7.2.1); or
(ii2) a thioesterase (EC 3.1.2); or
(ii3) a CoA-transferase (EC 2.8.3)
or of a microorganism expressing such a combination of enzymes for the conversion of 3-methylcrotonyl-CoA into 3-methylbutyric acid.

Further disclosed is the use of a combination comprising
(i) an enzyme classified as EC 1.3._._; and
(ii1) a phosphate butyryltransferase (EC 2.3.1.19) and/or a phosphate acetyltransferase (EC 2.3.1.8); and a phosphotransferase (EC 2.7.2), e.g. a butyrate kinase (EC 2.7.2.7), a branched-chain-fatty-acid kinase (EC 2.7.2.14), a propionate kinase (EC 2.7.2.15) and/or an acetate kinase (EC 2.7.2.1); or
(ii2) a thioesterase (EC 3.1.2); or
(ii3) a CoA-transferase (EC 2.8.3)
   and
(iii) a cytochrome P450 or a non-heme iron oxygenase or a fatty acid desaturase or of a microorganism expressing such a combination of enzymes for the conversion of 3-methylcrotonyl-CoA into isobutene.

- **Figure 1**: shows schematically the reaction of the conversion of 3-methylcrotonyl-CoA into 3-methylbutyryl-CoA by making use of an enzyme classified as EC 1.3.1._ which uses NADPH as a co-factor.
- **Figure 2**: shows schematically the reaction of the conversion of 3-methylcrotonyl-CoA into 3-methylbutyryl-CoA by making use of an enzyme classified as EC 1.3.1.- which uses NADH as a co-factor.
- **Figure 3**: shows schematically the reaction of the conversion of 3-methylcrotonyl-CoA into 3-methylbutyryl-CoA by making use of an enzyme classified as EC 1.3.8._ which uses FADH as a co-factor.
- **Figure 4**: shows schematically the conversion of 3-methylbutyryl-CoA into 3-methylbutyric acid catalyzed by a thioesterase (EC 3.1.2).
- **Figure 5**: shows schematically the conversion of 3-methylbutyryl-CoA into 3-methylbutyric acid catalyzed by a CoA-transferase (EC 2.8.3).
- **Figure 6**: shows schematically the conversion of 3-methylbutyryl-CoA into 3-methylbutyryl phosphate and the subsequent conversion of 3-methylbutyryl phosphate into 3-methylbutyric acid.
- **Figure 7**: shows schematically the conversion of 3-methylcrotonyl-CoA into 3-methylcrotonic acid catalyzed by a thioesterase (EC 3.1.2).
- **Figure 8**: shows schematically the conversion of 3-methylcrotonyl-CoA into 3-methylcrotonic acid catalyzed by a CoA-transferase (EC 2.8.3).
- **Figure 9**: shows schematically the conversion of 3-methylcrotonyl-CoA into 3-methylcrotonyl phosphate and the subsequent conversion of 3-methylcrotonyl phosphate into 3-methylcrotonic acid.
- **Figure 10**: shows schematically the conversion of 3-methylcrotonic acid into 3-methylbutyric acid by making use of an enzyme which is classified as a 2-enoate reductase (EC 1.3.1.31).
- **Figure 11a**: shows schematically the conversion 3-methylbutyric acid into isobutene.
- **Figure 11b**: shows schematically the conversion 3-methylbutyric acid into isobutene making use of NADPH as reducing agent and a flavodoxin/flavodoxin reductase as redox mediator protein.
- **Figure 12**: shows the conversion of 3-methylglutaconyl-CoA into 3-methylcrotonyl-CoA via a decarboxylation reaction.
- **Figure 13**: shows conversion of 3-hydroxy-3-methylglutaryl-CoA into 3-methylglutaconyl-CoA.
- **Figure 14**: shows the condensation of acetyl-CoA and acetoacetyl-CoA into 3-hydroxy-3-methylglutaryl-CoA.
- **Figure 15**: shows possible pathways for producing acetoacetyl-CoA from acetyl-CoA.
- **Figure 16**: shows a GC chromatogram obtained for the enzyme-catalyzed oxidative decarboxylation of isovalerate with the cytochrome P450 from Jeotgalicoccus sp. (Uniprot Accession number: E9NSU2) as outlined in Example 2.
- **Figure 17**: shows chromatograms of isobutene produced by an E.coli strain expressing cytochrome P450 from Jeotgalicoccus sp. and an E. coli control strain as outlined in Example 3.
- **Figure 18**: shows a GC chromatogram obtained for the enzyme-catalyzed oxidative decarboxylation of isovalerate with the cytochrome P450 from Macrococcus caseolyticus (Uniprot Accession number: B9EBA0) as outlined in Example 4.
- **Figure 19**: shows an example of a typical HPLC-chromatogram obtained for the enzymatic assay with acyl-CoA thioesterase II from Pseudomonas putida.
- **Figure 20a**: shows an overlay of typical HPLC-chromatograms (analysis of 3-methylcrotonyl-CoA, 3-methylcrotonic acid and CoA-SH) obtained for
a) enzymatic assay (assay A, Example 7)
b) enzyme-free assay (assay H, Example 7).
   The consumption of 3-methylcrotonyl-CoA with simultaneous production of CoA-SH and 3-methylcrotonic acid was observed in the enzymatic assay combining phosphate butyryltransferase with butyrate kinase.
- **Figure 20b**: shows an overlay of typical HPLC-chromatograms obtained for (analysis of ADP and ATP) obtained for
a) enzymatic assay (assay A, Example 7)
b) enzyme-free assay (assay H, Example 7).
   The consumption of ADP with simultaneous production of ATP was observed in the enzymatic assay combining phosphate butyryltransferase with butyrate kinase.
- **Figure 21**: shows the results of the production of 3-methylcrotonic acid and ATP in enzymatic assays comprising phosphate butyryltransferase from Bacilllus subtilis combined with different butyrate kinases, as well as in different control assays.
- **Figure 22**: shows the results of the production of 3-methylcrotonic acid and ATP in enzymatic assays comprising phosphate butyryltransferase from Enterococcus faecalis combined with different butyrate kinases, as well as in different control assays.
- **Figure 23,**: an embodiment of the invention shows a GC/FID chromatogram for the conversion of isovalerate into isobutene catalyzed by fatty acid desaturase from *Acinetobacterbaumannii.*

### EXAMPLES

### GENERAL METHODS AND MATERIALS

All reagents and materials used in the experiences were obtained from Sigma-Aldrich Company (St. Luis, MO) unless otherwise specified. Materials and methods suitable for growth of bacterial cultures and protein expression are well known in the art. Vector pCAN contained gene coding for flavodoxin reductase from E.coli (Uniprot P0A6L0) was purchased from NAIST (Nara Institute of Science and Technology, Japan, ASKA collection). The provided vector contained a stretch of 6 histidine codons after the methionine initiation codon. Flavodoxin reductase thus cloned was overexpressed in E.coli BL21(DE3) strain and purified on PROTINO-2000 Ni-TED column (Macherey-Nagel) allowing adsorption of 6-His tagged proteins. Fractions contained the enzyme of interest were pooled and concentrated on Amicon Ultra-4 10 kDa filter unit (Millipore). Flavodoxin reductase was then resuspended in 100 mM phosphate buffer pH 7.0, containing 100 mM NaCl to be used in subsequent enzymatic assays. Protein concentration was determined by direct UV 280 nm measurement on the NanoDrop 1000 spectrophotometer (Thermo Scientific).

### Example 1: Cloning and expression of recombinant cytochrome P450 fatty acid decarboxylases

### Gene synthesis, cloning and expression of recombinant proteins

The sequences of the studied enzymes inferred from the genome of prokaryotic organisms were generated by oligonucleotide concatenation to fit the codon usage of E. coli (genes were commercially synthesized by GeneArt®). A stretch of 6 histidine codons was inserted after the methionine initiation codon to provide an affinity tag for purification. The genes thus synthesized were cloned in a pET-25b(+) expression vector (vectors were constructed by GeneArt®).

BL21(DE3) competent cells (Novagen) were transformed with these vectors according to standard heat shock procedure and plated out onto LB agar plates supplemented with the appropriate antibiotic. Single transformants were used to inoculate 200 ml of ZYM-5052 auto-induction medium (Studier FW, Prot. Exp. Pur. 41, (2005), 207-234), supplemented with 0.5 mM aminolevulinic acid for cytochrome P450 expression. The cultures were incubated for 6h at 30°C in a shaker incubator and protein expression was continued at 18°C overnight (approximately 16 h).

The cells were collected by centrifugation at 4°C, 10,000 rpm for 20 min and the pellets were stored at -80°C.

### Example 2: In vitro oxidative decarboxylation of isovaleric acid into isobutene catalyzed by cytochrome P450 fatty acid decarboxylase from Jeotgalicoccus sp.

The pellet from 200 ml of cultured cells was resuspended in 50 ml of lysis buffer (100 mM potassium phosphate pH 7, 100 mM KCI) supplemented with 20 µl of lysonase (Merck-Novagen). Cell suspensions were then incubated for 10 minutes at room temperature followed by 20 minutes on ice. The amount of cytochrome P450 fatty acid decarboxylase in the total cell lysate was estimated on SDS-PAGE using gel densitometry.

0.5 M stock solution of 3-methylbutyrate (isovalerate) was prepared in water and adjusted to pH 7.0 with 10 M solution of NaOH.

Enzymatic assays were set up in 2 ml glass vials (Interchim) in the following conditions:
100 mM potassium phosphate buffer pH 7.0
100 mM NaCl
1 mM NADPH
50 mM isovalerate
0.2 mg/ml purified flavodoxin reductase from E.coli

Assays were started by adding the 30 µl of cell lysate containing the recombinant P450 fatty acid decarboxylase from Jeotgalicoccus sp. (Uniprot Accession number: E9NSU2; SEQ ID NO: 1) (total volume 300 µl).

A series of control assays were performed in parallel (Table 1).

The vials were sealed and incubated for 30 minutes at 30°C. The assays were stopped by incubating for 1 minute at 80°C and the isobutene present in the reaction headspace was analysed by Gas Chromatography (GC) equipped with Flame Ionization Detector (FID).

For the GC headspace analysis, one ml of the headspace gas was separated in a Bruker GC-450 system equipped with a GS-alumina column (30 m x 0.53 mm) (Agilent) using isothermal mode at 130°C. Nitrogen was used as carrier gas with a flow rate of 6 ml/min.

The enzymatic reaction product was identified by comparison with an isobutene standard. Under these GC conditions, the retention time of isobutene was 2.23 min.

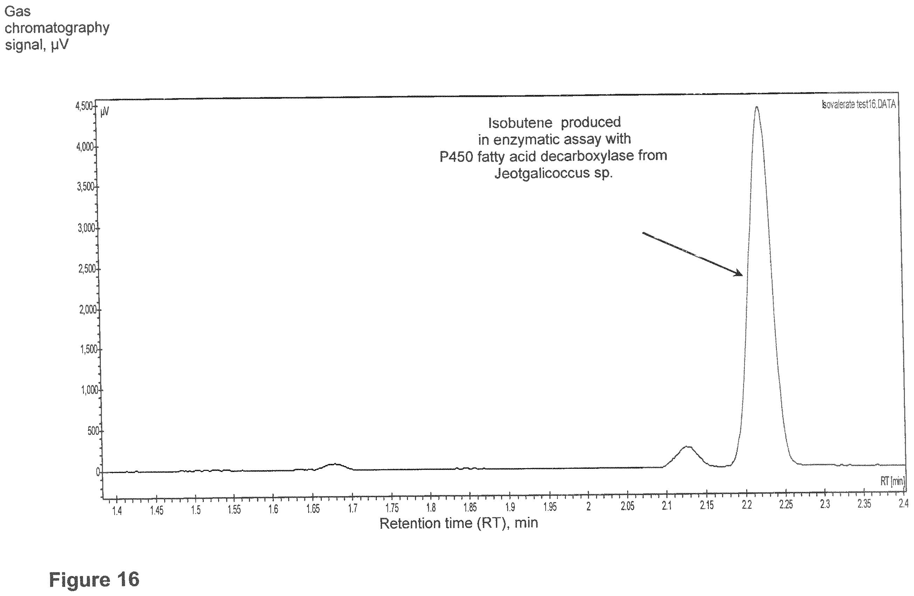

A significant production of isobutene from isovalerate was observed in the assay, contained cytochrome P450 fatty acid decarboxylase and redox partners (Table 1, Figure 16).

**Table 1**

| Assay | Isobutene peak area, arbitrary units |
|---|---|
| Enzymatic assay | 147.9 |
| Control assay without cytochrome P450 fatty acid decarboxylase | 14.5 |
| Control assay without flavodoxine reductase | 16.8 |
| Control assay without cytochrome P450 fatty acid decarboxylase and without flavodoxine reductase | 12.5 |
| Control assay without NADPH | 8 |

### Example 3: In vivo oxidative decarboxylation of isovaleric acid into isobutene catalyzed by cytochrome P450 fatty acid decarboxylase from Jeotgalicoccus sp.

BL21(DE3) competent cells were transformed with pET-25b(+) expression vector, coding for cytochrome P450 fatty acid decarboxylase from Jeotgalicoccus sp. ATCC 8456 and plated out onto LB agar plates supplemented with ampicillin (100 µg/ml). BL21(DE3) strain transformed with empty pET-25b(+) vector was used as a negative control in the subsequent assays (control strain). Plates were incubated overnight at 30°C. Single transformants were used to inoculate LB medium, supplemented with ampicillin, followed by incubation at 30°C overnight. 1 ml of this overnight culture was used to inoculate 300 ml of ZYM-5052 auto-inducing media (Studier FW (2005), local citation), supplemented with 0.5 mM aminolevulinic acid. The cultures were grown for 20 hours at 30°C and 160 rpm shaking.

A volume of cultures corresponding to OD₆₀₀ of 30 was removed and centrifuged. The pellet was resuspended in 30 ml of MS medium (Richaud C., Mengin-Leucreulx D., Pochet S., Johnson EJ., Cohen GN. and Marlière P, The Journal of Biological Chemistry, 268, (1993), 26827-26835), containing glucose (45 g/L) and MgSO₄ (1mM) and supplemented with 50 mM isovalerate. These cultures were then incubated in 160 ml bottles, sealed with a screw cap, at 30°C with shaking for 22 h. The pH value of the cultures was adjusted to 8.5 after 8 hours of incubation by using 30 % NH₄OH.

After an incubation period, the isobutene produced in the headspace was analysed by Gas Chromatography (GC) equipped Flame Ionization Detector (FID). One ml of the headspace gas phase was separated and analysed according to the method described in Example 2.

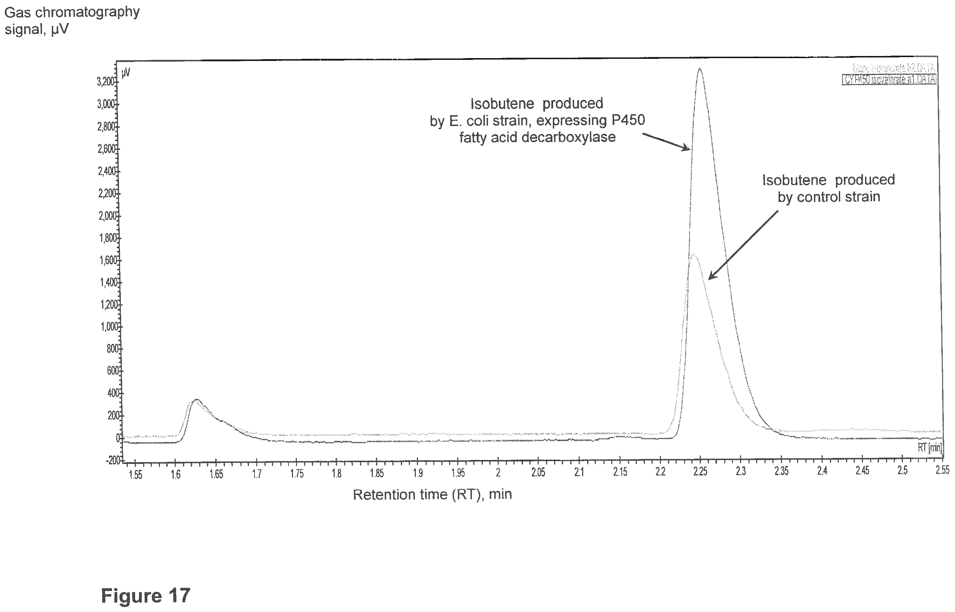

Figure 17 shows that a certain quantity of isobutene was produced with a control strain probably due to the spontaneous decomposition of isovalerate. The ratio of isobutene produced by E.coli strain, expressing P450 fatty acid decarboxylase, versus isobutene produced with a control strain was about 2.2 fold judging from isobutene peak areas (Figure 17). These results clearly indicate that a production of isobutene from isovalerate can be achieved in vivo by E.coli strain expressing cytochrome P450 fatty acid decarboxylase from Jeotgalicoccus sp. ATCC 8456.

### Example 4: In vitro oxidative decarboxylation of isovaleric acid into isobutene catalyzed by cytochrome P450 fatty acid decarboxylase from Macrococcus caseolyticus

The pellet from 200 ml of cultured cells was resuspended in 50 ml of lysis buffer (100 mM potassium phosphate pH 7, 100 mM KCI) supplemented with 20 µl of lysonase (Merck-Novagen). Cell suspensions were then incubated for 10 minutes at room temperature followed by 20 minutes on ice. The amount of cytochrome P450 fatty acid decarboxylase in the total cell lysate was estimated on SDS-PAGE using gel densitometry.

0.5 M stock solution of 3-methylbutyrate (isovalerate) was prepared in water and adjusted to pH 7.0 with 10 M solution of NaOH.

Enzymatic assays were set up in 2 ml glass vials (Interchim) in the following conditions:
100 mM potassium phosphate buffer pH 7.0
100 mM NaCl
2 mM NADPH
50 mM isovalerate
0.2 mg/ml purified flavodoxin reductase from E.coli

Assays were started by adding 100µl of cell lysate containing the recombinant P450 fatty acid decarboxylase from Macrococcus caseolyticus (total volume 300 µl).

A series of control assays were performed in parallel (Table 2).

The vials were sealed and incubated for 60 minutes at 30°C. The assays were stopped by incubating for 1 minute at 80°C and the isobutene present in the reaction headspace was analysed by Gas Chromatography (GC) equipped with Flame Ionization Detector (FID).

For the GC headspace analysis, one ml of the headspace gas was separated in a Bruker GC-450 system equipped with a GS-alumina column (30 m x 0.53 mm) (Agilent) using isothermal mode at 130°C. Nitrogen was used as carrier gas with a flow rate of 6 ml/min.

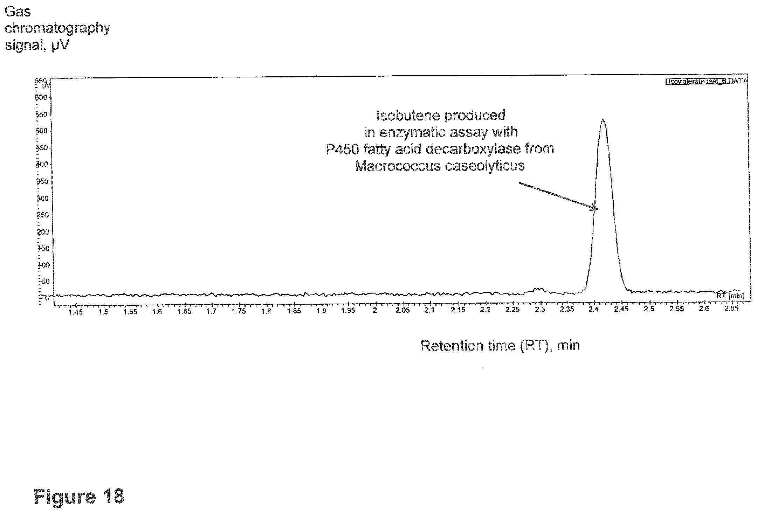

The enzymatic reaction product was identified by comparison with an isobutene standard. Under these GC conditions, the retention time of isobutene was 2.45 min. A significant production of isobutene from isovalerate was observed in the assay, contained cytochrome P450 fatty acid decarboxylase and redox partners (Table 2, Figure 18).

**Table 2**

| Assay | Isobutene peak area, arbitrary units |
|---|---|
| Enzymatic assay | 18 |
| Control assay without cytochrome P450 fatty acid decarboxylase | 2.5 |
| Control assay without flavodoxine reductase | 2.7 |
| Control assay without cytochrome P450 fatty acid decarboxylase and without flavodoxine reductase | 2.5 |
| Control assay without NADPH | 1.5 |

### Example 5: Cloning and overexpression of recombinant phosphate butyryltransferases and butyrate kinases

### Gene synthesis, cloning and expression of recombinant proteins

The sequences of phosphate butyryltransferase genes from Bacillus subtilis (strain 168) and Enterococcus faecalis MTUP9 (Uniprot Accession number: P54530 and A0A038BNC2, respectively) and butyrate kinase from Lactobacillus casei W56 and Geobacillus sp. GHH01 (Uniprot Accession number: K0N529 and L8A0E1, respectively) were generated by oligonucleotide concatenation to fit the codon usage of E. coli (genes were commercially synthesized by GeneArt®). The expression of corresponding proteins was conducted following the method described in Example 1. The cells were collected by centrifugation at 4°C, 10.000 rpm for 20 min and the pellets were stored at -80°C.

### Protein purification and concentration

The pellets from 200 ml of cultured cells were thawed on ice and resuspended in 5 ml of 50 mM potassium phosphate buffer pH 7.5 containing 100 mM NaCl, 10 mM MgCl₂, 10 mM imidazole and 1 mM DTT. Twenty microliters of lysonase (Novagen) were added. Cells were incubated 10 minutes at room temperature and then returned to ice for 20 minutes. Cell lysis was completed by sonication for 2 x 30 seconds. The bacterial extracts were then clarified by centrifugation at 4°C, 4000 rpm for 40 min. The clarified bacterial lysates were loaded onto a PROTINO-2000 Ni-TED column (Macherey-Nagel) allowing adsorption of 6-His tagged proteins. Columns were washed and the enzymes of interest were eluted with 6 ml of 50 mM potassium phosphate buffer pH 7.5 containing 100 mM NaCl and 250 mM imidazole. Eluates were then concentrated, desalted on an Amicon Ultra-4 10 kDa filter unit (Millipore) and enzymes were resuspended in 50 mM potassium phosphate buffer pH 7.5, containing 100 mM NaCl. The purity of proteins thus purified varied from 70% to 90% as estimated by SDS-PAGE analysis. Protein concentrations were determined by direct UV 280 nm measurement on the NanoDrop 1000 spectrophotometer (Thermo Scientific) or by Bradford assay (BioRad).

### Example 6: Enzyme-catalyzed hydrolysis of 3-methylcrotonyl-CoA into 3-methylcrotonic acid and free coenzyme-A

The gene coding for acyl-CoA thioesterase II from Pseudomonas putida was synthesized according to the procedure as described in Example 1.

The vector pCA24N which contained the gene encoding acyl-CoA thioesterase 2 (TesB) from Escherichia coli was purchased from NAIST (Nara Institute of Science and Technology, Japan, ASKA collection). This vector rovided contained a stretch of 6 histidine codons after the methionine initiation codon.

The corresponding enzymes were produced according to the procedure described in Example 1.

The enzymatic assays were conducted in a total reaction volume of 0.2 ml.

The standard reaction mixture contained:
50 mM HEPES pH 7.0
10 mM 3-methylcrotonyl-CoA (Sigma-Aldrich)
20 mM MgCl₂
20 mM NaCl
1 mg/ml of purified recombinant thioesterase.

Control assays were performed in which either no enzyme was added or no substrate was added.

The assays were incubated for 30 min with shaking at 30°C, the reactions were stopped by the addition of 0.1 ml of acetonitrile and the samples were then analyzed by HPLC-based procedure.

### HPLC based analysis of the consumption of 3-methylcrotonyl-CoA and the formation of 3-methylcrotonic acid and free coenzyme A (CoA-SH)

HPLC analysis was performed using an 1260 Inifinity LC System (Agilent), equipped with a column heating module and a UV detector (210 nm). 5 µl of samples were separated on Zorbax SB-Aq column (250 x 4.6 mm, 5 µm particle size, column temp.30°C) with a mobile phase flow rate of 1.5 ml/min. The separation was performed using mixed A (H₂O containing 8.4 mM sulfuric acid) and B (acetonitrile) solutions in a linear gradient (0% B at initial time 0 min→70% B at 8 min). Commercial 3-methylcrotonyl-CoA, 3-methylcrotonic acid (Sigma-Aldrich) and CoA-SH (Sigma-Aldrich) were used as references. In these conditions, the retention time of free coenzyme A (CoA-SH), 3-methylcrotonyl-CoA and 3-methylcrotonic acid were 4.05, 5.38 and 5.83 min, respectively.

No 3-methylcrotonic acid signal was observed in control assays.

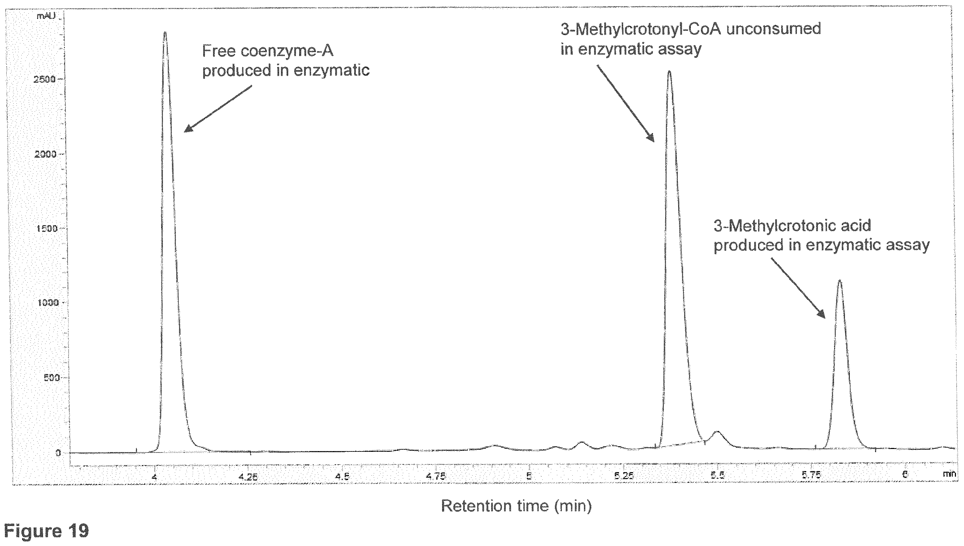

Both studied thioesterases catalyzed the hydrolysis of 3-methylcrotonyl-CoA with the formation of 3-methylcrotonic acid. An example of a chromatogram obtained with acyl-CoA thioesterase II from Pseudomonas putida is shown in Figure 19.

The degree of production of 3-methylcrotonic acid as observed in the enzymatic assays is shown in Table 3.

**Table 3**

| **GENE NAMES** | **ORGANISM** | **UNIPROT ACCESSION NUMBER** | **3-METHYLCROTONIC ACID PRODUCED, MM** |
|---|---|---|---|
| TESB | ESCHERICHIA COLI | P0AGG2 | 0.6 |
| TESB | PSEUDOMONAS PUTIDA | Q88DR1 | 3.1 |

### Example 7: Conversion of 3-methylcrotonyl-CoA and ADP into 3-methylcrotonic acid and ATP catalysed by the combined action of phosphate butyryltransferase from Bacillus subtilis and butyrate kinase from Lactobacillus casei or Geobacillus sp.

In the assays described in the following, the following enzymes were used:

**Table 4**

| **GENE NAMES** | **ORGANISM** | **UNIPROT ACCESSION NUMBER** |
|---|---|---|
| PTB (YQIS) | BACILLUS SUBTILIS | P54530 |
| BUK | LACTOBACILLUS CASEI | K0N529 |
| BUK | GEOBACILLUS SP. | L8A0E1 |

The enzymatic assays were conducted in a total reaction volume of 0.2 ml.

The standard reaction mixture contained:
50 mM potassium phosphate buffer pH 7.5
4 mM 3-methylcrotonyl-CoA
4 mM ADP
10 mM MgCl₂
10 mM NaCl
0.2 mg/ml of purified phosphate butyryltransferase from Bacillus subtilis
0.2 mg/ml of purified butyrate kinase from Lactobacillus casei or Geobacillus sp.

A series of controls was performed in parallel (Assays C-H as shown in Table 5).

**Table 5**

| | **Assay composition** | | | | | | | |
|---|---|---|---|---|---|---|---|---|
| | **A** | **B** | **C** | **D** | **E** | **F** | **G** | **H** |
| 3-methylcrotonyl-CoA | + | + | + | + | + | + | + | + |
| ADP | + | + | | | + | + | + | + |
| phosphate butyryltransferase from Bacillus subtilis | + | + | + | + | + | | | |
| butyrate kinase from Lactobacillus casei | + | | + | | | + | | |
| butyrate kinase from Geobacillus sp | | + | | + | | | + | |

Assays were then incubated for 20 min with shaking at 30° C.

After an incubation period, the reactions were stopped by heating the reaction medium 4 min at 90°C. The samples were centrifuged, filtered through a 0.22 µm filter and the clarified supernatants were transferred into a clean vial for further analysis. The consumption of ADP and 3-methylcrotonyl-CoA, and the formation of ATP, 3-methylcrotonic acid and free coenzyme A (CoA-SH) were followed by using HPLC-based methods.

### HPLC-based analysis of ADP and ATP

HPLC analysis was performed using the 1260 Inifinity LC System (Agilent) equipped with a column heating module and an RI detector. 2 µl of samples were separated on a Polaris C18-A column (150 x 4.6 mm, 5 µm particle size, column temp.30°C) with a mobile phase flow rate of 1.5 ml/min. The consumption of ADP and the formation of ATP were followed by HPLC.

### HPLC-based analysis of ADP and ATP

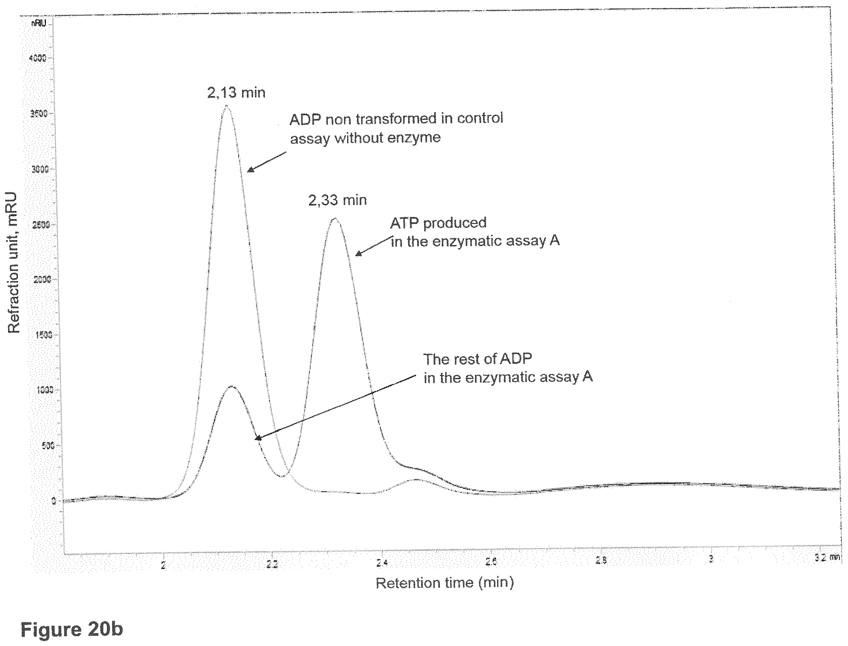

HPLC analysis was performed using 1260 Inifinity LC System (Agilent), equipped with column heating module and RI detector. 2 µl of samples were separated on Polaris C18-A column (150 x 4.6 mm, 5 µm particle size, column temp.30°C) with a mobile phase flow rate of 1.5 ml/min. The separation was performed using 8.4 mM sulfuric acid in H₂O/MeOH mixed solution (99/1) (V/V). In these conditions, the retention time of ADP and ATP were 2.13 min, 2.33 min, respectively (see Figure 20b).

### HPLC based analysis of 3-methylcrotonyl-CoA, 3-methylcrotonic acid and free coenzyme A (CoA-SH)

HPLC analysis was performed using the 1260 Inifinity LC System (Agilent) equipped with a column heating module and a UV detector (260 nm). 1 µl of samples were separated on a Zorbax SB-Aq column (250 x 4.6 mm, 5 µm particle size, column temp.30°C), with a mobile phase flow rate of 1.5 ml/min.

The consumption of 3-methylcrotonyl-CoA and the formation of 3-methylcrotonic acid and free coenzyme A (CoA-SH) were followed according to the procedure described in Example 6. Under these conditions, the retention time of 3-methylcrotonyl-CoA, 3-methylcrotonic acid and free coenzyme A (CoA-SH) was 5.38 min, 5.73 min and 4.07 min, respectively.

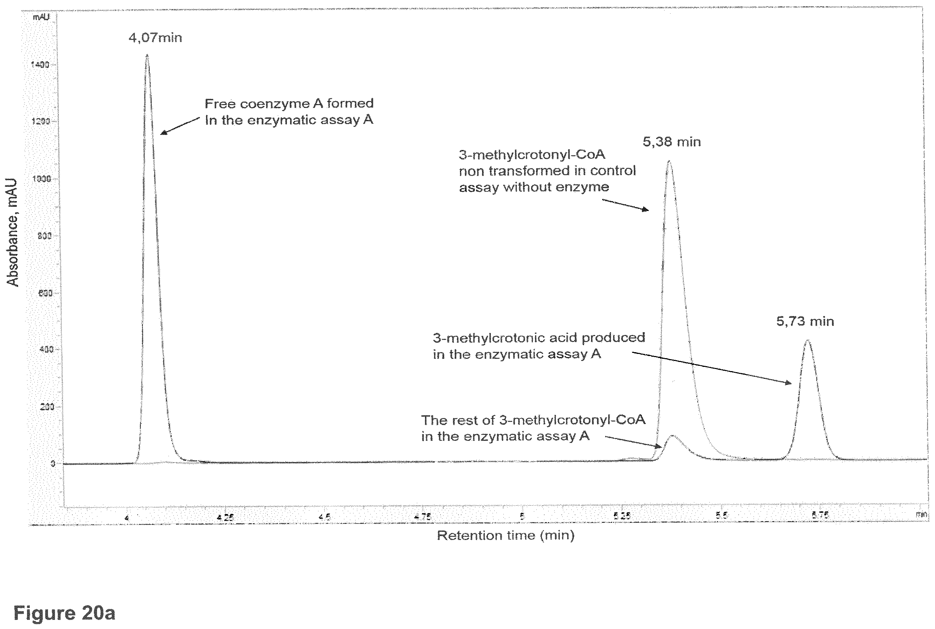

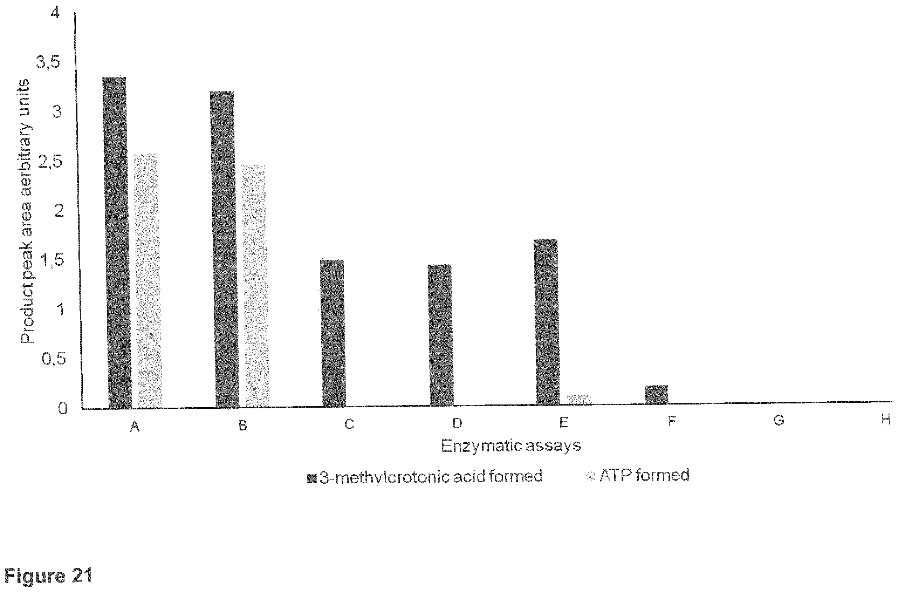

Typical chromatograms obtained for the enzymatic assay A and enzyme-free assay H are shown in Figures 20a and 20b. The results of the HPLC analysis are summarized in Figure 21.

The obtained data indicate that 3-methylcrotonyl-CoA was converted into 3-methylcrotonic acid with the concomitant generation of ATP from ADP in a two-step reaction, catalyzed by two enzymes, respectively (assays A and B). Thus, the conversion occurred through the formation of the intermediate 3-methylcrotonyl phosphate followed by transfer of a phosphate group from this intermediate to ADP, thereby releasing ATP.

A significant production of 3-methylcrotonic acid without a simultaneous generation of ATP was observed when phosphate butyryltransferase was used alone (assay E). This production is due to a spontaneous hydrolysis of 3-methylcrotonyl phosphate generated by the action of phosphate butyryltransferase.

The production of 3-methylcrotonic acid was observed in the same manner for the control assays without ADP (assays C and D). This production was also due to a hydrolysis of 3-methylcrotonyl phosphate generated by the action of phosphate butyryltransferase.

### Example 8: Conversion of 3-methylcrotonyl-CoA and ADP into 3-methylcrotonic acid and ATP catalysed by the combined action of the phosphate butyryltransferase from Enterococcus faecalis and butyrate kinase from Lactobacillus casel or Geobacillus sp.

In the assays described in the following, the following enzymes were used:

**Table 6**

| **GENE NAMES** | **ORGANISM** | **UNIPROT ACCESSION NUMBER** |
|---|---|---|
| PTB | ENTEROCOCCUS FAECALIS | A0A038BNC2 |
| BUK | LACTOBACILLUS CASEI | K0N529 |
| BUK | GEOBACILLUS SP. | L8A0E1 |

The enzymatic assays were conducted in a total reaction volume of 0.2 ml. The standard reaction mixture contained:
50 mM potassium phosphate buffer pH 7.5
4 mM 3-methylcrotonyl-CoA
4 mM ADP
10 mM MgCl₂
10 mM NaCl
0.2 mg/ml of purified phosphate butyryltransferase from Enterococcus faecalis
0.2 mg/ml of purified butyrate kinase from Lactobacillus casei or Geobacillus sp.

A series of controls was performed in parallel (Assays C-H Table 7).

**Table 7**

| | **Assay composition** | | | | | | | |
|---|---|---|---|---|---|---|---|---|
| | **A** | **B** | **C** | **D** | **E** | **F** | **G** | **H** |
| 3-methylcrotonyl-CoA | + | + | + | + | + | + | + | + |
| ADP | + | + | | | + | + | + | + |
| phosphate butyryltransferase from Enterococcus faecalis | + | + | + | + | + | | | |
| butyrate kinase from Lactobacillus casei | + | | + | | | + | | |
| butyrate kinase from Geobacillus sp | | + | | + | | | + | |

Assays were then incubated for 20 min with shaking at 30° C.

After an incubation period, the reactions were stopped by heating the reaction medium 4 min at 90°C. The samples were centrifuged, filtered through a 0.22 µm filter and the clarified supernatants were transferred into a clean vial for further analysis. The consumption of ADP and 3-methylcrotonyl-CoA and the formation of ATP and 3-methylcrotonic acid and free coenzyme A (CoA-SH) were followed by HPLC analysis according to the methods described in Example 7 and Example 6.

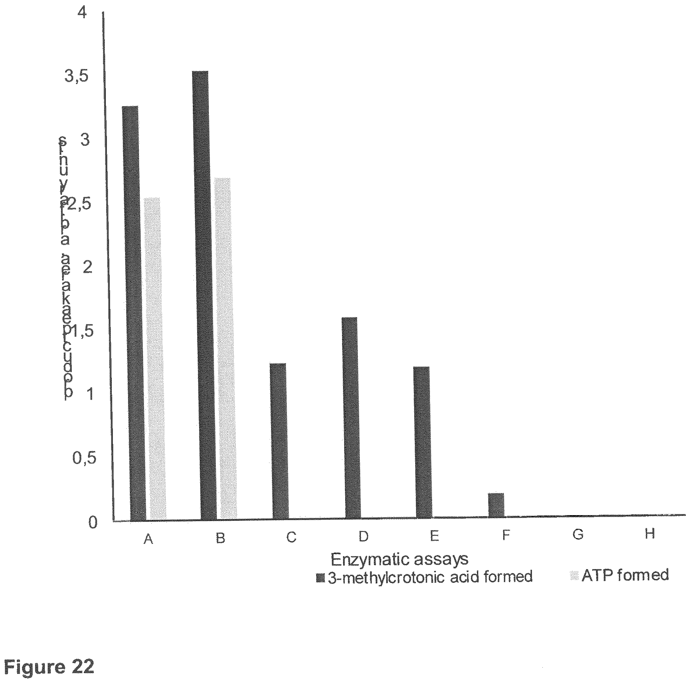

The results of the HPLC analysis are summarized in Figure 22.

The obtained data indicate that the 3-metylcrotonyl-CoA was converted into 3-methylcrotonic acid with the concomitant generation of ATP from ADP in a two-step reaction, catalyzed by two enzymes, respectively (assays A and B). Thus, the conversion occurred through the formation of the intermediate 3-methylcrotonyl phosphate followed by the transfer of a phosphate group from this intermediate on ADP, thereby releasing ATP.

A significant production of 3-methylcrotonic acid without simultaneous generation of ATP was observed when phosphate butyryltransferase was used alone (assay E). This production was due to a hydrolysis of 3-methylcrotonyl phosphate generated by the action of phosphate butyryltransferase.

The production of 3-methylcrotonic acid was observed in a similar manner for the control assays without ADP (assays C and D). This production was also due to a hydrolysis of 3-methylcrotonyl phosphate generated by the action of phosphate butyryltransferase.

### Example 9 (an embodiment of the invention) In vivo bioconversion of isovalerate into isobutene catalyzed by fatty acid desaturases (UndB)

All reagents and materials used in the experiences were obtained from Sigma-Aldrich Company (St. Luis, MO) unless otherwise specified. Materials and methods suitable for growth of bacterial cultures and protein expression are well known in the art.

### Gene synthesis, cloning and expression of recombinant proteins

The sequences of the studied enzymes inferred from the genome of prokaryotic organisms were generated by oligonucleotide concatenation to fit the codon usage of E. coli (genes were commercially synthesized by GeneArt®) (Table 8).The genes thus synthesized were cloned in a pET-25b(+) expression vector (vectors were constructed by GeneArt®).

BL21(DE3) competent cells (Novagen) were transformed with these vectors according to standard heat shock procedure and plated out onto LB agar plates supplemented with the appropriate antibiotic. BL21(DE3) strain transformed with empty pET-25b(+) vector was used as a negative control in the subsequent assays (control strain). Single transformants were used to inoculate LB medium, supplemented with ampicillin, followed by incubation at 30°C overnight. 1 ml of this overnight culture was used to inoculate 300 ml of ZYM-5052 auto-inducing media (Studier FW, Prot. Exp. Pur. 41, (2005), 207-234.The cultures were grown for 20 hours at 30°C and 160 rpm shaking.

A volume of cultures corresponding to OD₆₀₀ of 30 was removed and centrifuged. The pellet was resuspended in 30 ml of MS medium (Richaud C., Mengin-Leucreulx D., Pochet S., Johnson EJ., Cohen GN. and Marlière P, The Journal of Biological Chemistry, 268, (1993), 26827-26835), containing glucose (45 g/L) and MgSO₄ (1mM) and supplemented with 40 mM isovalerate (3-methylbutyrate). These cultures were then incubated in 160 ml bottles, sealed with a screw cap, at 30°C with shaking for 24 h. The pH value of the cultures was adjusted to 8.5 after 8 hours of incubation by using 30 % NH₄OH.

After an incubation period, the isobutene produced in the headspace was analysed by Gas Chromatography (GC) equipped Flame Ionization Detector (FID).

For the GC headspace analysis, one ml of the headspace gas was separated in a Bruker GC-450 system equipped with a GS-alumina column (30 m x 0.53 mm) (Agilent) using isothermal mode at 130°C. Nitrogen was used as carrier gas with a flow rate of 6 ml/min.

The enzymatic reaction product was identified by comparison with an isobutene standard. Under these GC conditions, the retention time of isobutene was 2.30 min.

A significant production of isobutene from isovalerate was, e.g., observed with strains expressing fatty acid desaturases from Pseudomonas mendocina and Acinetobacter baumanii (Table 8). A typical chromatogram of isobutene production catalyzed by fatty acid decarboxylase from Acinetobacter baumanii is shown on Figure 23.

**Table 8**

| Uniprot accession number | Organism | isobutene peak area µV.min-1 |
|---|---|---|
| A4Y0K1 | Pseudomonas mendocina | 1016 |
| A0AOD5YLX9 | Acinetobacter baumannii | 3637 |
| negative control (empty vector) | | 0 |

## Claims

1. A method for the production of isobutene from 3-methylcrotonyl-CoA comprising the steps of:
(a) enzymatically converting 3-methylcrotonyl-CoA into 3-methylbutyric acid; and
(b) further enzymatically converting the thus produced 3-methylbutyric acid into isobutene,
wherein the conversion of 3-methylbutyric acid into isobutene according to step (b) is achieved by a UndB fatty acid desaturase (trans-membrane non-heme iron oxygenase).

2. The method of claim 1, wherein the enzymatic conversion of 3-methylcrotonyl-CoA into 3-methylbutyric acid according to step (a) is achieved by a method comprising the following steps:
(a1) enzymatically converting 3-methylcrotonyl-CoA into 3-methylbutyryl-CoA; and
(a2) further enzymatically converting the thus produced 3-methylbutyryl-CoA into 3-methylbutyric acid.

3. The method of claim 2, wherein the conversion of 3-methylcrotonyl-CoA into 3-methylbutyryl-CoA according to step (a1) is achieved by the use of an enzyme classified in EC 1.3.

4. The method of claim 3, wherein the enzyme is selected from the group consisting of
(i) an acyl-CoA dehydrogenase (NADP+) (EC 1.3.1.8);
(ii) an enoyl-[acyl-carrier-protein] reductase (NADPH, Si-specific) (EC 1.3.1.10);
(iii) a cis-2-enoyl-CoA reductase (NADPH) (EC 1.3.1.37);
(iv) a trans-2-enoyl-CoA reductase (NADPH) (EC 1.3.1.38);
(v) an enoyl-[acyl-carrier-protein] reductase (NADPH, Re-specific) (EC 1.3.1.39);
(vi) crotonyl-CoA reductase (EC 1.3.1.86) ;
(vii) an enoyl-[acyl-carrier-protein] reductase (NADH) (EC 1.3.1.9) ;
(viii) a trans-2-enoyl-CoA reductase (NAD⁺) (EC 1.3.1.44) ; and
(ix) an isovaleryl-CoA dehydrogenase (EC 1.3.8.4).

5. The method of any one of claims 2 to 4, wherein the conversion of 3-methylbutyryl-CoA into 3-methylbutyric acid according to step (a2) is achieved by a method comprising the step of enzymatically converting 3-methylbutyryl-CoA into 3-methylbutyric acid in a hydrolysis reaction by making use of a thioester hydrolase (EC 3.1.2).

6. The method of any one of claims 2 to 4, wherein the conversion of 3-methylbutyryl-CoA into 3-methylbutyric acid according to step (a2) is achieved by a method comprising the step of enzymatically converting 3-methylbutyryl-CoA into 3-methylbutyric acid in a transferase reaction by making use of a CoA-transferase (EC 2.8.3).

7. The method of any one of claims 2 to 4, wherein the conversion of 3-methylbutyryl-CoA into 3-methylbutyric acid according to step (a2) is achieved by a method comprising the following steps:
(i) enzymatically converting 3-methylbutyryl-CoA into 3-methylbutyryl phosphate; and
(ii) further enzymatically converting the thus produced 3-methylbutyryl phosphate into 3-methylbutyric acid.

8. The method of claim 7, wherein the conversion of 3-methylbutyryl-CoA into 3-methylbutyryl phosphate according to step (i) is achieved by making use of a phosphate butyryltransferase (EC 2.3.1.19) or a phosphate acetyltransferase (EC 2.3.1.8).

9. The method of claim 7 or 8, wherein the conversion of 3-methylbutyryl phosphate into 3-methylbutyric acid according to step (ii) is achieved by making use of a phosphotransferase (EC 2.7.2).

10. The method of claim 9, wherein the phosphotransferase (EC 2.7.2) is selected from the group consisting of a butyrate kinase (EC 2.7.2.7), a branched-chain-fatty-acid kinase (EC 2.7.2.14), a propionate kinase (EC 2.7.2.15) or an acetate kinase (EC 2.7.2.1).

11. The method of claim 1, wherein the enzymatic conversion of 3-methylcrotonyl-CoA into 3-methylbutyric acid according to step (a) is achieved by a method comprising the following steps:
(aI) enzymatically converting 3-methylcrotonyl-CoA into 3-methylcrotonic acid; and
(aII) further enzymatically converting the thus produced 3-methylcrotonic acid into 3-methylbutyric acid.

12. The method of claim 11, wherein the conversion of 3-methylcrotonyl-CoA into 3-methylcrotonic acid according to step (aI) is achieved by a method comprising the step of enzymatically converting 3-methylcrotonyl-CoA into 3-methylcrotonic acid in a hydrolysis reaction by making use of a thioester hydrolase (EC 3.1.2).

13. The method of claim 11, wherein the conversion of 3-methylcrotonyl-CoA into 3-methylcrotonic acid according to step (aI) is achieved by a method comprising the step of enzymatically converting 3-methylcrotonyl-CoA into 3-methylcrotonic acid in a transferase reaction by making use of a CoA-transferase (EC 2.8.3).

14. The method of claim 11, wherein the conversion of 3-methylcrotonyl-CoA into 3-methylcrotonic acid according to step (aI) is achieved by a method comprising the following steps:
(i) enzymatically converting 3-methylcrotonyl-CoA into 3-methylcrotonyl phosphate; and
(ii) further enzymatically converting the thus produced 3-methylcrotonyl phosphate into 3-methylcrotonic acid.

15. The method of claim 14, wherein the conversion of 3-methylcrotonyl-CoA into 3-methylcrotonyl phosphate according to step (i) is achieved by making use of a phosphate butyryltransferase (EC 2.3.1.19) or a phosphate acetyltransferase (EC 2.3.1.8).

16. The method of claim 14 or 15, wherein the conversion of 3-methylcrotonyl phosphate into 3-methylcrotonic acid according to step (ii) is achieved by making use of a phosphotransferase (EC 2.7.2).

17. The method of claim 16, wherein the phosphotransferase (EC 2.7.2) is selected from the group consisting of a butyrate kinase (EC 2.7.2.7), a branched-chain-fatty-acid kinase (EC 2.7.2.14), a propionate kinase (EC 2.7.2.15) or an acetate kinase (EC 2.7.2.1).

18. The method of any one of claims 11 to 17, wherein the conversion of 3-methylcrotonic acid into 3-methylbutyric acid is achieved by making use of a 2-enoate reductase (EC 1.3.1.31).

## Patentansprüche

1. Verfahren zur Herstellung von Isobuten aus 3-Methylcrotonyl-CoA, umfassend die Schritte:
(a) enzymatisches Umsetzen von 3-Methylcrotonyl-CoA in 3-Methylbuttersäure; und
(b) des Weiteren enzymatisches Umsetzen der dadurch hergestellten 3-Methylbuttersäure in Isobuten,
wobei die Umsetzung von 3-Methylbuttersäure in Isobuten nach Schritt (b) durch eine UndB-Fettsäure-Desaturase (Transmembran-nicht-Hämeisen-Oxygenase; trans-membrane non-heme iron oxygenase) erreicht wird.

2. Verfahren nach Anspruch 1, wobei die enzymatische Umsetzung von 3-Methylcrotonyl-CoA in 3-Methylbuttersäure nach Schritt (a) durch ein Verfahren erreicht wird, das die folgenden Schritte umfasst:
(a1) enzymatisches Umsetzen von 3-Methylcrotonyl-CoA in 3-Methylbutyryl-CoA; und
(a2) des Weiteren enzymatisches Umsetzen des dadurch hergestellten 3-Methylbutyryl-CoA in 3-Methylbuttersäure.

3. Verfahren nach Anspruch 2, wobei die Umsetzung von 3-Methylcrotonyl-CoA in 3-Methylbutyryl-CoA nach Schritt (a1) durch Verwendung eines Enzyms erreicht wird, das unter EC 1.3. klassifiziert ist.

4. Verfahren nach Anspruch 3, wobei das Enzym ausgewählt ist aus der Gruppe bestehend aus:
(i) einer Acyl-CoA-Dehydrogenase (NADP+) (EC 1.3.1.8);
(ii) einer Enoyl-[Acyl-Trägerprotein]-Reductase (NADPH, Si-spezifisch) (EC 1.3.1.10);
(iii) einer Cis-2-Enoyl-CoA-Reductase (NADPH) (EC 1.3.1.37);
(iv) einer Trans-2-Enoyl-CoA-Reductase (NADPH) (EC 1.3.1.38);
(v) einer Enoyl-[Acyl-Trägerprotein]-Reductase (NADPH, Re-spezifisch) (EC 1.3.1.39);
(vi) Crotonyl-CoA-Reductäse (EC 1.3.1.86);
(vii) einer Enoyl-[Acyl-Trägerprotein]-Reductase (NADH) (EC 1.3.1.9);
(viii) einer Trans-2-Enoyl-CoA-Reductase (NAD⁺) (EC 1.3.1.44); und
(ix) einer Isovaleryl-CoA-Dehydrogenase (EC 1.3.8.4).

5. Verfahren nach einem der Ansprüche 2 bis 4, wobei die Umsetzung von 3-Methylbutyryl-CoA in 3-Methylbuttersäure nach Schritt (a2) durch ein Verfahren erreicht wird, das den Schritt des enzymatischen Umsetzens von 3-Methylbutyryl-CoA in 3-Methylbuttersäure in einer Hydrolysereaktion durch Verwendung einer Thioester-Hydrolase (EC 3.1.2) umfasst.

6. Verfahren nach einem der Ansprüche 2 bis 4, wobei die Umsetzung von 3-Methylbutyryl-CoA in 3-Methylbuttersäure nach Schritt (a2) durch ein Verfahren erreicht wird, das den Schritt des enzymatischen Umsetzens von 3-Methylbutyryl-CoA in 3-Methylbuttersäure in einer Transferasereaktion durch Verwendung einer CoA-Transferase (EC 2.8.3) umfasst.

7. Verfahren nach einem der Ansprüche 2 bis 4, wobei die Umsetzung von 3-Methylbutyryl-CoA in 3-Methylbuttersäure nach Schritt (a2) durch ein Verfahren erreicht wird, das die folgenden Schritte umfasst:
(i) enzymatisches Umsetzen von 3-Methylbutyryl-CoA in 3-Methylbutyrylphosphat; und
(ii) des Weiteren enzymatisches Umsetzen des dadurch hergestellten 3-Methylbutyrylphosphats in 3-Methylbuttersäure.

8. Verfahren nach Anspruch 7, wobei die Umsetzung von 3-Methylbutyryl-CoA in 3-Methylbutyrylphosphat nach Schritt (i) durch Verwendung einer Phosphat-Butyryltransferase (EC 2.3.1.19) oder einer Phosphat-Acetyltransferase (EC 2.3.1.8) erreicht wird.

9. Verfahren nach Anspruch 7 oder 8, wobei die Umsetzung von 3-Methylbutyrylphosphat in 3-Methylbuttersäure nach Schritt (ii) durch Verwendung einer Phosphotransferase (EC 2.7.2) erreicht wird.

10. Verfahren nach Anspruch 9, wobei die Phosphotransferase (EC 2.7.2) ausgewählt ist aus der Gruppe bestehend aus einer Butyratkinase (EC 2.7.2.7), einer verzweigtkettige-Fettsäurekinase (branched-chain-fatty-acid kinase; EC 2.7.2.14), einer Propionatkinase (EC 2.7.2.15) oder einer Acetatkinase (EC 2.7.2.1).

11. Verfahren nach Anspruch 1, wobei die enzymatische Umsetzung von 3-Methylcrotonyl-CoA in 3-Methylbuttersäure nach Schritt (a) durch ein Verfahren erreicht wird, das die folgenden Schritte umfasst:
(aI) enzymatisches Umsetzen von 3-Methylcrotonyl-CoA in 3-Methylcrotonsäure; und
(aII) des Weiteren enzymatisches Umsetzen der dadurch hergestellten 3-Methylcrotonsäure in 3-Methylbuttersäure.

12. Verfahren nach Anspruch 11, wobei die Umsetzung von 3-Methylcrotonyl-CoA in 3-Methylcrotonsäure nach Schritt (aI) durch ein Verfahren erreicht wird, das den Schritt des enzymatischen Umsetzens von 3-Methylcrotonyl-CoA in 3-Methylcrotonsäure in einer Hydrolysereaktion durch Verwendung einer Thioester-Hydrolase (EC 3.1.2) umfasst.

13. Verfahren nach Anspruch 11, wobei die Umsetzung von 3-Methylcrotonyl-CoA in 3-Methylcrotonsäure nach Schritt (aI) durch ein Verfahren erreicht wird, das den Schritt des enzymatischen Umsetzens von 3-Methylcrotonyl-CoA in 3-Methylcrotonsäure in einer Transferasereaktion durch Verwendung einer CoA-Transferase (EC 2.8.3) umfasst.

14. Verfahren nach Anspruch 11, wobei die Umsetzung von 3-Methylcrotonyl-CoA in 3-Methylcrotonsäure nach Schritt (aI) durch ein Verfahren erreicht wird, das die folgenden Schritte umfasst:
(i) enzymatisches Umsetzen von 3-Methylcrotonyl-CoA in 3-Methylcrotonylphosphat; und
(ii) des Weiteren enzymatisches Umsetzen des dadurch hergestellten 3-Methylcrotonylphosphats in 3-Methylcrotonsäure.

15. Verfahren nach Anspruch 14, wobei die Umsetzung von 3-Methylcrotonyl-CoA in 3-Methylcrotonylphosphat nach Schritt (i) durch Verwendung einer Phosphat-Butyryltransferase (EC 2.3.1.19) oder einer Phosphat-Acetyltransferase (EC 2.3.1.8) erreicht wird.

16. Verfahren nach Anspruch 14 oder 15, wobei die Umsetzung von 3-Methylcrotonylphosphat in 3-Methylcrotonsäure nach Schritt (ii) durch Verwendung einer Phosphotransferase (EC 2.7.2) erreicht wird.

17. Verfahren nach Anspruch 16, wobei die Phosphotransferase (EC 2.7.2) ausgewählt ist aus der Gruppe bestehend aus einer Butyratkinase (EC 2.7.2.7), einer verzweigtkettige-Fettsäurekinase (EC 2.7.2.14), einer Propionatkinase (EC 2.7.2.15) oder einer Acetatkinase (EC 2.7.2.1).

18. Verfahren nach einem der Ansprüche 11 bis 17, wobei die Umsetzung von 3-Methylcrotonsäure in 3-Methylbuttersäure durch Verwendung einer 2-Enoatreductase (EC 1.3.1.31) erreicht wird.

## Revendications

1. Procédé pour la production d'isobutène à partir de 3-méthylcrotonyl-CoA, comprenant les étapes suivantes :
(a) conversion enzymatique de 3-méthylcrotonyl-CoA en acide 3-méthylbutyrique ; et
(b) en outre conversion enzymatique de l'acide 3-méthylbutyrique ainsi produit en isobutène,
dans lequel la conversion d'acide 3-méthylbutyrique en isobutène conformément à l'étape (b) est réalisée au moyen d'une UndB acide gras désaturase (oxygénase de fer non-hème transmembranaire).

2. Procédé selon la revendication 1, dans lequel la conversion enzymatique de la 3-méthylcrotonyl-CoA en acide 3-méthylbutyrique conformément à l'étape (a) est réalisée par un procédé comprenant les étapes suivantes :
(a1) conversion enzymatique de 3-méthylcrotonyl-CoA en 3-méthylbutyryl-CoA ; et
(a2) en outre conversion enzymatique de la 3-méthylbutyryl-CoA ainsi produite en acide 3-méthylbutyrique.

3. Procédé selon la revendication 2, dans lequel la conversion de 3-méthylcrotonyl-CoA en 3-méthylbutyryl-CoA conformément à l'étape (a1) est réalisée par l'utilisation d'une enzyme classée parmi EC 1.3.

4. Procédé selon la revendication 3, dans lequel l'enzyme est choisie dans le groupe constitué par
(i) une acyl-CoA déshydrogénase (NADP+) (EC 1.3.1.8) ;
(ii) une énoyl-[acyl-support-protéine] réductase (NADPH, spécifique de Si) (EC 1.3.1.10) ;
(iii) une cis-2-énoyl-CoA réductase (NADPH) (EC 1.3.1.37) ;
(iv) une trans-2-énoyl-CoA réductase (NADPH) (EC 1.3.1.38) ;
(v) une énoyl-[acyl-support-protéine] réductase (NADPH, spécifique de Re) (EC 1.3.1.39) ;
(vi) la crotonyl-CoA réductase (EC 1.3.1.86) ;
(vii) une énoyl-[acyl-support-protéine] réductase (NADH) (EC 1.3.1.9) ;
(viii) une trans-2-énoyl-CoA réductase (NAD⁺) (EC 1.3.1.44) ; et
(ix) une isovaléryl-CoA déshydrogénase (EC 1.3.8.4).

5. Procédé selon l'une quelconque des revendications 2 à 4, dans lequel la conversion de la 3-méthylbutyryl-CoA en acide 3-méthylbutyrique conformément à l'étape (a2) est réalisée par un procédé comprenant l'étape de conversion enzymatique de 3-méthylbutyryl-CoA en acide 3-méthylbutyrique dans une réaction d'hydrolyse par utilisation d'une thioester hydrolase (EC 3.1.2).

6. Procédé selon l'une quelconque des revendications 2 à 4, dans lequel la conversion de la 3-méthylbutyryl-CoA en acide 3-méthylbutyrique conformément à l'étape (a2) est réalisée par un procédé comprenant l'étape de conversion enzymatique de 3-méthylbutyryl-CoA en acide 3-méthylbutyrique dans une réaction avec une transférase par utilisation d'une CoA-transférase (EC 2.8.3).

7. Procédé selon l'une quelconque des revendications 2 à 4, dans lequel la conversion de la 3-méthylbutyryl-CoA en acide 3-méthylbutyrique conformément à l'étape (a2) est réalisée par un procédé comprenant les étapes suivantes :
(i) conversion enzymatique de 3-méthylbutyryl-CoA en phosphate de 3-méthylbutyryle ; et
(ii) en outre conversion enzymatique du phosphate de 3-méthylbutyryle ainsi produit en acide 3-méthylbutyrique.

8. Procédé selon la revendication 7, dans lequel la conversion de 3-méthylbutyryl-CoA en phosphate de 3-méthylbutyryle conformément à l'étape (i) est réalisée par utilisation d'une phosphate butyryltransférase (EC 2.3.1.19) ou d'une phosphate acétyltransférase (EC 2.3.1.8).

9. Procédé selon la revendication 7 ou 8, dans lequel la conversion de phosphate de 3-méthylbutyryle en acide 3-méthylbutyrique conformément à l'étape (ii) est réalisée par utilisation d'une phosphotransférase (EC 2.7.2).

10. Procédé selon la revendication 9, dans lequel la phosphotransférase (EC 2.7.2) est choisie dans le groupe constitué par une butyrate kinase (EC 2.7.2.7), une acide gras à chaîne ramifiée kinase (EC 2.7.2.14), une propionate kinase (EC 2.7.2.15) et une acétate kinase (EC 2.7.2.1).

11. Procédé selon la revendication 1, dans lequel la conversion enzymatique de 3-méthylcrotonyl-CoA en acide 3-méthylbutyrique conformément à l'étape (a) est réalisée par un procédé comprenant les étapes suivantes :
(aI) conversion enzymatique de 3-méthylcrotonyl-CoA en acide 3-méthylcrotonique ; et
(aII) en outre conversion enzymatique de l'acide 3-méthylcrotonique ainsi produit en acide 3-méthylbutyrique.

12. Procédé selon la revendication 11, dans lequel la conversion de 3-méthylcrotonyl-CoA en acide 3-méthylcrotonique conformément à l'étape (aI) est réalisée par un procédé comprenant l'étape de conversion enzymatique de 3-méthylcrotonyl-CoA en acide 3-méthylcrotonique dans une réaction d'hydrolyse par utilisation d'une thioester hydrolase (EC 3.1.2).

13. Procédé selon la revendication 11, dans lequel la conversion de 3-méthylcrotonyl-CoA en acide 3-méthylcrotonique conformément à l'étape (aI) est réalisée par un procédé comprenant l'étape de conversion enzymatique de 3-méthylcrotonyl-CoA en acide 3-méthylcrotonique dans une réaction avec une transférase par utilisation d'une CoA-transférase (EC 2.8.3).

14. Procédé selon la revendication 11, dans lequel la conversion de 3-méthylcrotonyl-CoA en acide 3-méthylcrotonique conformément à l'étape (aI) est réalisée par un procédé comprenant les étapes suivantes :
(i) conversion enzymatique de 3-méthylcrotonyl-CoA en phosphate de 3-méthylcrotonyle ; et
(ii) en outre conversion enzymatique du phosphate de 3-méthylcrotonyle ainsi produit en acide 3-méthylcrotonique.

15. Procédé selon la revendication 14, dans lequel la conversion de 3-méthylcrotonyl-CoA en phosphate de 3-méthylcrotonyle conformément à l'étape (i) est réalisée par utilisation d'une phosphate butyryltransférase (EC 2.3.1.19) ou d'une phosphate acétyltransférase (EC 2.3.1.8).

16. Procédé selon la revendication 14 ou 15, dans lequel la conversion de phosphate de 3-méthylcrotonyle en acide 3-méthylcrotonique conformément à l'étape (ii) est réalisée par utilisation d'une phosphotransférase (EC 2.7.2).

17. Procédé selon la revendication 16, dans lequel la phosphotransférase (EC 2.7.2) est choisie dans le groupe constitué par une butyrate kinase (EC 2.7.2.7), un acide gras à chaîne ramifiée kinase (EC 2.7.2.14), une propionate kinase (EC 2.7.2.15) et une acétate kinase (EC 2.7.2.1).

18. Procédé selon l'une quelconque des revendications 11 à 17, dans lequel la conversion d'acide 3-méthylcrotonique en acide 3-méthylbutyrique est réalisée par utilisation d'une 2-énoate réductase (EC 1.3.1.31).
